# EUROPÄISCHE PATENTSCHRIFT

(11) **EP 0 567 828 B1**
(45) Veröffentlichungstag und Bekanntmachung des Hinweises auf die Patenterteilung: **24.01.1996**
(21) Anmeldenummer: 93105924.0
(22) Anmeldetag: 13.04.1993
(51) Int. Cl.: C07C 255/37, C07C 69/734, C07C 251/48, A01N 37/36, A01N 37/50

(54) **Benzylenolether und deren Verwendung als Pflanzenschutzmittel**
Benzylenol ethers and their use as plant-protective agents
Benzylénoléthers et leur application comme produits phytosanitaires

(30) Priorität: 30.04.1992 DE 4214189
(43) Veröffentlichungstag der Anmeldung: 03.11.1993
(73) Patentinhaber: BASF Aktiengesellschaft, D-67063 Ludwigshafen (DE)
(72) Erfinder: Wingert, Horst, Dr., W-6800 Mannheim 1 (DE); Sauter, Hubert, Dr., W-6800 Mannheim 1 (DE); Benoit, Remy, Dr., W-6700 Ludwigshafen (DE); Roehl, Franz, Dr., W-6707 Schifferstadt (DE); Ammermann, Eberhard, Dr., W-6148 Heppenheim (DE); Lorenz, Gisela, Dr., W-6730 Neustadt (DE)

(56) Entgegenhaltungen:
- EP-A- 0 178 826
- EP-A- 0 253 213
- EP-A- 0 463 488
- EP-A- 0 513 580

## Beschreibung

Die vorliegende Erfindung betrifft neue Benzyl-Enolether, diese enthaltende Schädlingsbekämpfungsmittel, insbesondere Fungizide, und ein Verfahren zur Bekämpfung von Schädlingen, insbesondere von Pilzen, Insekten, Nematoden und Spinnmilben mit diesen Verbindungen.

Es ist bekannt, daß β-Methoxacrylate, z.B. der 2-[2-(Phenyloxymethylen)-phenyl]-3-methoxy-acrylsäuremethylester (EP 178 826) wie auch Oximether, z.B. der 2-[2-(Phenyloxymethylen)-phenyl]-glyoxylsäuremethylester-O-methyloximether (vgl. EP 253 213) fungizide bzw. insektizide Aktivität besitzen. Ihre Wirkung ist jedoch unbefriedigend.

Es wurde überraschend gefunden, daß Benzyl-Enolether der allgemeinen Formel I 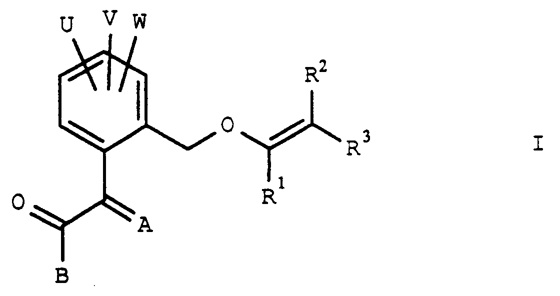
in der
B
OH, C₁-C₄-Alkoxy, C₁-C₄-Alkylthio und C₁-C₄-Alkylamino bedeutet,
U, V, W
gleich oder verschieden sind und Wasserstoff, Halogen, C₁-C₄-Alkyl und C₁-C₄-Alkoxy bedeuten, und
a) für den Fall, daß
   A
   CH₂, CHCl, CH-O-C₁-C₄-Alkyl, CH-S-C₁-C₄-Alkyl, N-O-C₁-C₄-Alkyl bedeutet, und
   R¹, R², R³
   gleich oder verschieden sind und Wasserstoff, Cyano, Halogen, NR⁴R⁵, CO₂R⁴, CONR⁴R⁵, COR⁴, S(O)ₙR⁴ mit n = 0, 1 und 2, PO(OR⁴)₂, ggf. verzweigtes C₁-C₁₀-Alkyl, C₁-C₄-Halogenalkyl, C₃-C₆-Cycloalkyl, C₃-C₆-Halogencycloalkyl, C₃-C₆-Cycloalkyl-C₁-C₄-Alkyl, C₁-C₄-Alkoxy-C₁-C₄-Alkyl,C₁-C₄-Alkylthio-C₁-C₄-Alkyl, Arylthio-C₁-C₄-Alkyl, C₂-C₆-Alkenyl, C₂-C₅-Halogenalkenyl, C₃-C₆-Cycloalkenyl, C₃-C₆-Halogencycloalkenyl, C₂-C₆-Alkinyl, C₁-C₆-Alkoxy, C₁-C₆-Halogenalkoxy, C₁-C₄-Alkylthio, Benzylthio, C₁-C₄-Alkylcarbonyl, ggf. subst. Phenylcarbonyl, ggf.
   subst. Benzylcarbonyl, C₁-C₄Alkoxycarbonyl, ggf. subst. Phenoxycarbonyl, ggf. subst. Benzyloxycarbonyl, ggf. subst. Aryl, ggf. subst. Aryloxy, ggf. subst. Arylthio, ggf. subst. Aryl-C₁-C₄-alkyl, ggf. subst. Aryl-C₂-C₄-alkenyl, ggf. subst. Aryloxy-C₁-C₄-alkyl, ggf. subst. Arylthio-C₁-C₄-alkyl, ggf. subst. Hetaryl, ggf. subst. Hetaryloxy, ggf. subst. Hetarylthio, ggf. subst. Heteroaryl-C₁-C₄-alkyl, ggf. subst. Hetaryl-C₂-C₄-alkenyl, ggf. subst. Hetaryloxy-C₁-C₄-alkyl, ggf. subst. Heterocyclyl, ggf. subst. Heterocyclyloxy bedeuten,
   wobei "ggf. subst." neben Wasserstoff die Reste Halogen, Cyano, Nitro, C₁-C₄-Alkyl, C₁-C₄-Alkoxy, C₁-C₄-Halogenalkyl, C₁-C₄-Halogenalkoxy, C₁-C₁₀-Alkoximino-C₁-C₂-alkyl, Aryl, Aryloxy, Benzyloxy, Hetaryl, Hetaryloxy, C₃-C₆-Cycloalkyl, Heterocyclyl, Heterocyclyloxy bedeuten,
   und die Reste
   R⁴, R⁵
   gleich oder verschieden sind und Wasserstoff und C₁-C₄-Alkyl bedeuten,
   R¹ und R³ zusammen, sowie auch R² und R³ zusammen jeweils einen carbocyclischen oder einen heterocyclischen Ring bilden können, der seinerseits benzanneliert sein kann und durch die unter "ggf. subst." genannten Rest substituiert sein kann,
   und für den Fall, daß
b) A CH-C₁-C₄-Alkyl bedeutet,
   R¹, R², R³
   gleich oder verschieden sind und Wasserstoff, Cyano, Halogen, NR⁴R⁵, CO₂R⁴, CONR⁴R⁵, COR⁴, S(O)ₙR⁴ mit n = 0, 1 und 2, PO(OR⁴)₂, ggf. verzweigtes C₁-C₁₀-Alkyl, C₁-C₄-Halogenalkyl, C₃-C₆-Cycloalkyl, C₃-C₆-Halogencycloalkyl, C₃-C₆-Cycloalkyl-C₁-C₄-Alkyl, C₁-C₄-Alkoxy-C₁-C₄-Alkyl, C₁-C₄-Alkylthio-C₁-C₄-Alkyl, Arylthio-C₁-C₄-Alkyl, C₂-C₆-Alkenyl, C₂-C₅-Halogenalkenyl, C₃-C₆-Cycloalkenyl, C₃-C₆-Halogencycloalkenyl, C₂-C₆-Alkinyl, C₁-C₆-Alkoxy, C₁-C₆-Halogenalkoxy, C₁-C₄-Alkylthio, Benzylthio, C₁-C₄-Alkylcarbonyl, ggf. subst. Phenylcarbonyl, ggf.
   subst. Benzylcarbonyl, C₁-C₄Alkoxycarbonyl, ggf. subst. Phenoxycarbonyl, ggf. subst. Benzyloxycarbonyl, ggf. subst. Aryl, ggf. subst. Aryloxy, ggf. subst. Arylthio, ggf. subst. Aryl-C₁-C₄-alkyl, ggf. subst. Aryl-C₂-C₄-alkenyl, ggf. subst. Aryloxy-C₁-C₄-alkyl, ggf. subst. Arylthio-C₁-C₄-alkyl, ggf. subst. Hetaryl, ggf. subst. Hetaryloxy, ggf. subst. Hetarylthio, ggf. subst. Heteroaryl-C₁-C₄-alkyl, ggf. subst. Hetaryl-C₂-C₄-alkenyl, ggf. subst. Hetaryloxy-C₁-C₄-alkyl, ggf. subst. Heterocyclyl, ggf. subst. Heterocyclyloxy bedeuten,
   wobei "ggf. subst." neben Wasserstoff die Reste Halogen, Cyano, Nitro, C₁-C₄-Alkyl, C₁-C₄-Alkoxy, C₁-C₄-Halogenalkyl, C₁-C₄-Halogenalkoxy, C₁-C₁₀-Alkoximino-C₁-C₂-alkyl, Aryl, Aryloxy, Benzyloxy, Hetaryl, Hetaryloxy, C₃-C₆-Cycloalkyl, Heterocyclyl, Heterocyclyloxy bedeuten,
   und die Reste
   R⁴, R⁵
   gleich oder verschieden sind und Wasserstoff und C₁-C₄-Alkyl bedeuten,
   R¹ und R³ zusammen, sowie auch R² und R³ zusammen jeweils einen der folgenden Ringsysteme A - F bilden können
   für die Gruppierung 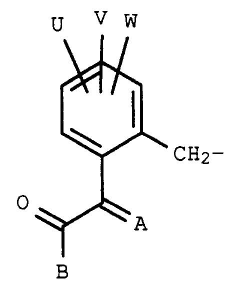 steht im folgenden das Symbol 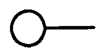 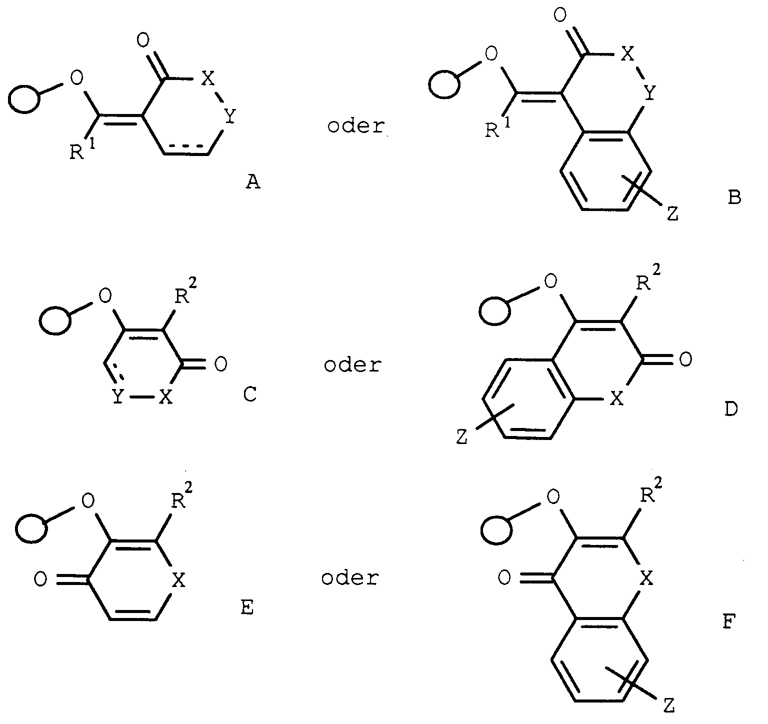 wobei
   X
   CH₂, O, NR⁴ bedeutet,
   Y
   CH, (CH₂)ₙ, mit n = 0, 1 und 2 bedeutet,
   Z
   Wasserstoff, Halogen, C₁-C₄-Alkyl und C₁-C₄-Alkoxy und
   R⁴
   Wasserstoff und C₁-C₄-Alkyl und .... eine Einfachbindung oder eine Doppelbindung bedeutet,
   eine ausgezeichnete fungizide, insektizide, nematizide und akarizide Wirkung haben, die besser ist, als die der bekannten β-Methoxyacrylate bzw. Oximether. Die fungizide Wirkung wird bevorzugt.

Die in der allgemeinen Formel I aufgeführten Reste können beispielsweise folgende Bedeutung haben:
A
kann z.B. C₁-C₄-Alkyliden (z.B. Methyliden, Ethyliden, n- oder iso-Propyliden, n-, iso-, sec.- oder tert.-Butyliden), C₁-C₄-Alkoxymethyliden (z.B. Methoxy-, Ethoxy-, n- oder iso-Propoxy, n-, iso-, sec.- oder tert.-Butoxymethyliden), C₁-C₄-Alkylthiomethyliden (z.B. Methyl-, Ethyl-, n- oder iso-Propyl, n-, iso-, sec.- oder tert.-Butylthiomethyliden), C₁-C₄-Alkoxyimino (z.B. Methoxy-, Ethoxy-, n- oder iso-Propoxy, n-, iso-, sec.- oder tert.-Butoxyimino), Chlormethyliden,
B
kann OH, C₁-C₄-Alkoxy (z.B. Methoxy, Ethoxy, n- oder iso-Propoxy, n-, iso-, sec.- oder tert.-Butoxy), C₁-C₄-Alkylthio (z.B. Methylthio, Ethylthio, n- oder iso-Propylthio, n-, iso-, sec.-oder tert.-Butylthio) und C₁-C₄-Alkylamin (z.B. Methylamin, Ethylamin, n- oder iso-Propylamin, n-, iso-, sec.- oder tert.-Butylamino),
U, V, W
können z.B. H, Halogen (z.B. Fluor, Chlor, Brom, Jod), Methyl, Methoxy,
R¹, R², R³
können gleich oder verschieden sein und Wasserstoff, Cyano, Halogen (z.B. Fluor, Chlor, Brom, Jod), NR⁴R⁵, CO₂R⁴, CONR⁴R⁵, COR⁴, S(O)ₙR⁴ mit n = 0, 1 und 2, PO(OR⁴)₂,
wobei die Reste
R⁴, R⁵
gleich oder verschieden sind und Wasserstoff und C₁-C₄-Alkyl (z.B. Methyl, Ethyl, n-, iso-Propyl, n-, iso-, sec.- und tert-Butyl) bedeuten,
R¹, R², R³
können ferner ggf. verzweigtes C₁-C₁₀-Alkyl (z.B. Methyl, Ethyl, n- oder iso-Propyl, n-, iso-, sec.- oder tert.-Butyl, n-, iso-, sec.-, tert.- oder neo-Pentyl, n-Hexyl, n-Decyl),
C₁-C₄-Halogenalkyl (z.B. Trifluormethyl, 2-Fluorethyl, 2,2,2-Trifluorethyl, Pentafluorethyl, Fluordichlormethyl, Difluorchlormethyl, Chlormethyl, Dichlormethyl, Trichlormethyl, 2-Chlorethyl, 2,2,2-Trichlorethyl, Pentachlorethyl),
C₃-C₆-Cycloalkyl (z.B. Cyclopropyl, Cyclobutyl, Cyclopentyl, Cyclohexyl),
C₃-C₆-Halogencycloalkyl (z.B. 2,2-Difluorcyclopropyl, 2,2-Dichlorcyclopropyl, 2,2-Dibromcyclopropyl, 2,2-Dichlor-3-Methylcyclopropyl, Tetrafluorcyclobutyl), C₃-C₆-Cycloalkyl-C₁-C₄-alkyl (z.B. 1-Methylcyclopropyl, 2,2-Dimethylcyclopropyl, 1-Methylcyclohexyl),
C₁-C₄-Alkoxy-C₁-C₄-alkyl (z. B Methoxymethyl, Ethoxymethyl, n- oder iso-Propoxylmethyl, n-, iso-, sec.- oder tert.-Butoxymethyl, 2-Methoxy-prop-2-yl, 2-Ethoxyprop-2-yl, 2-n- oder iso-Propoxyprop-2-yl, 2-n-, iso-, sec.- oder tert.-Butoxy-prop-2-yl),
C₁-C₄-Alkylthio-C₁-C₄-alkyl (z.B. Methylthiomethyl, Ethylthiomethyl, n-, oder iso-Propylthiomethyl, n-, iso-, sec.- oder tert.-Butylthiomethyl, 2-Methylthioprop-2-yl, 2-Ethylthioprop-2-yl, 2-n- oder iso-Propylthio-prop-2-yl, 2-n-, iso-, sec.- odert tert.-Butylthio-prop-2-yl),
Aryl(Phenyl)thio-C₁-C₄-alkyl (z.B. Phenylthiomethyl, 2-Chlorphenyl-thiomethyl),
C₂-C₆-Alkenyl (z.B. Vinyl, 1-Propenyl, 2-Propenyl, 2-Butenyl, 3-Butenyl, 1-Methyl-2-propenyl, 3-Methyl-2-butenyl,
2-Methyl-2-penten-5-yl),
C₂-C₅-Halogenalkenyl (z.B. 2,2-Difluorvinyl, 2,2-Dichlorvinyl, 3,3,3-Trifluorpropenyl, 3,3,3-Trichlorpropenyl, 3-Chlor-2-propenyl),
C₃-C₆-Cycloalkenyl (z.B. Cyclopent-1-enyl, Cyclopentadienyl, Cyclohex-1-enyl),
C₃-C₆-Halogencycloalkenyl (z.B. Pentafluorcyclopentadienyl, Pentachlorcyclopentadienyl),
C₂-C₄-Alkinyl (z.B. Ethinyl, 1-Propinyl, 1-Propargyl),
C₁-C₄-Alkoxy (z.B. Methoxy, Ethoxy, n- oder iso-Propoxy, n-, iso-, sec.- oder tert.-Butoxy),
C₁-C₄-Alkylthio (z.B. Methylthio, Ethylthio, n- oder iso-Propylthio, n-, iso-, sec.- oder tert.-Butylthio), Benzylthio,
C₁-C₄-Halogenalkoxy (z.B. Trifluormethoxy, Pentafluorethoxy, 1,1,2,2-Tetrafluorethoxy),
C₁-C₄-Alkylcarbonyl (z.B. Acetyl, Propionyl, Butyryl, iso-Butyryl, Pivaloyl),
ggf. subst. Phenylcarbonyl (z.B. Benzoyl, 4-Chlorbenzoyl),
ggf. subst. Benzylcarbonyl (z.B. Benzylcarbonyl),
C₁-C₄-Alkoxycarbonyl (z.B. Methoxycarbonyl, Ethoxycarbonyl, n-oder iso-Propoxycarbonyl, n-, iso-, sec.- oder tert.-Butoxycarbonyl),
ggf. subst. Phenoxycarbonyl (z.B. Phenoxycarbonyl, 4-Chlorphenoxycarbonyl),
ggf. subst. Benzyloxycarbonyl (z.B. Benzyloxycarbonyl),
ggf. subst. Aryl (z.B. Phenyl, Naphthyl, Anthryl),
ggf. subst. Aryloxy (z.B. Phenoxy, Naphthoxy, Anthroxy),
ggf. subst. Arylthio (z.B. Phenylthio),
ggf. subst. Aryl-C₂-C₄-alkyl (z.B. Benzyl, 1-Phenethyl, 2-Phenethyl, 1-Phenylpropyl, 2-Phenylpropyl, 3-Phenylpropyl, 2-Methyl-3-phenylpropyl, 2-Methyl-2-phenylpropyl, 4-Phenylbutyl),
ggf. subst. Aryl-C₁-C₄-alkenyl (z.B. Phenyl-1-ethenyl, 2-Phenyl-1-propenyl, 2,2-Diphenylethenyl, 1-Phenyl-1-propen-2-y, 1-Phenyl-1-ethenyl),
ggf. subst. Aryloxy-C₁-C₄-alkyl (z.B. Phenoxymethyl),
ggf. subst. Arylthio-C₁-C₄-alkyl (z.B. Phenylthiomethyl),
ggf. subst. Heteroaryl (z.B. Pyridyl, 2-Pyridyl, 3-Pyridyl, 4-Pyridyl, Pyrimidinyl, 4-Pyrimidinyl, 2-Pyrimidinyl, Thienyl, 2-Thienyl, 3-Thienyl, Furyl, 2-Furyl, 3-Furyl, 1-Pyrrolyl, 1-Imidazolyl, 1,2,4-Triazolyl, 1,3,4-Triazolyl, 4-Thiazolyl, 2-Benzothiazolyl),
ggf. subst. Heteroaryloxy (z.B. 2-Pyridyloxy, 2-Pyrimidinyloxy),
ggf. subst. Heteroarylthio (z.B. 2-Pyridylthio, 2-Pyrimidinylthio, 2-Benzothiazolylthio),
ggf. subst. Heteroaryl-C₁-C₄-alkyl (z.B. 2-Pyridylmethyl, 3-Pyridylmethyl),
ggf. subst. Heteroaryloxy-C₁-C₄-alkyl (z.B. Furfurylmethoxy, Thienylmethoxy, 3-Isoxazolylmethoxy, 2-Oxazolylmethoxy, 2-Pyridylmethoxy),
ggf. subst. Heteroaryl-C₂-C₄-alkenyl(z.B. 2'-Furyl-2-ethenyl, 2'-Thienyl-2-ethenyl, 3'-Pyridyl-2-ethenyl),
ggf. subst. Heterocyclyl (z.B. Oxiranyl, 1-Aziridinyl, 1-Azetidinyl, 1-Pyrrolidinyl, 2-Tetrahydrofuryl, 2-Tetrahydropyranyl, 3-Tetrahydropyranyl, 1-Piperidinyl, 1-Morpholinyl, 1-Piperazinyl, 1,3-Dioxanyl, 3-Tetrahydrothiopyranyl),
ggf. subst. Heterocycloxy (z.B. 2-Dihydropyranyloxy, 2-Tetrahydropyranyloxy) bedeuten.

Die mit "ggf. substituiert" bezeichneten Reste im Vorstehenden sind neben Wasserstoff z.B. Fluor, Chlor, Brom, Jod, Cyano, Nitro, Methyl, Ethyl, iso-Propyl, tert.-Butyl, Methoxy, Ethoxy, iso-Propoxy, tert.-Butoxy, Trifluormethyl, Chlormethyl, Dichlormethyl, Trichlormethyl, Trifluormethoxy, 1,1,2,2-Tetrafluorethoxy, Methoxyiminomethyl, Ethoxyiminomethyl, n-Propoxyiminomethyl, n-Butoxyiminomethyl, n-Pentoxyiminomethyl, n-Hexoxyiminomethyl, Allyloxyiminomethyl, Benzyloxyiminomethyl, iso-Propoxyiminomethyl, iso-Butoxyiminomethyl, tert.-Butoxyiminomethyl, Methylimino-1-ethyl, Ethoxyimino-1-ethyl, n-Propoxyimino-1-ethyl, n-Butoxyimino-1-ethyl, n-Pentoxyiminol-1-ethyl, n-Hexoxyimino-1-ethyl, Allyloximini-1-ethyl, Benzyloxyimino-1-ethyl, Phenyl, Phenoxy, Benzyloxy, Imidazol-1-yl, Piperazin-1-yl, 4-Morpholinyl, Piperidin-1-yl, Pyridyl-2-oxy, Cyclopropyl, Cyclohexyl, Oxiranyl, 1,3-Dioxan-2-yl, 1,3-Dioxolan-2-yl, Tetrahydropyran-2-yloxy bedeuten.

R¹ und R³ zusammen, sowie auch R² und R³ zusammen können jeweils einen carbocyclischen oder einen heterocyclischen Ring bilden, der seinerseits benzanneliert sein kann und durch die unter "ggf. subst." genannten Rest substituiert sein kann, wobei u.a. die folgenden Ringsysteme A - F möglich sind:
für die Gruppierung 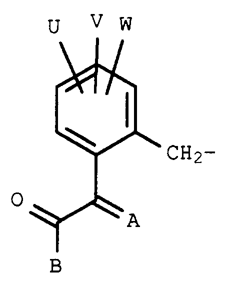
steht im folgenden das Symbol 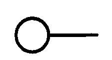 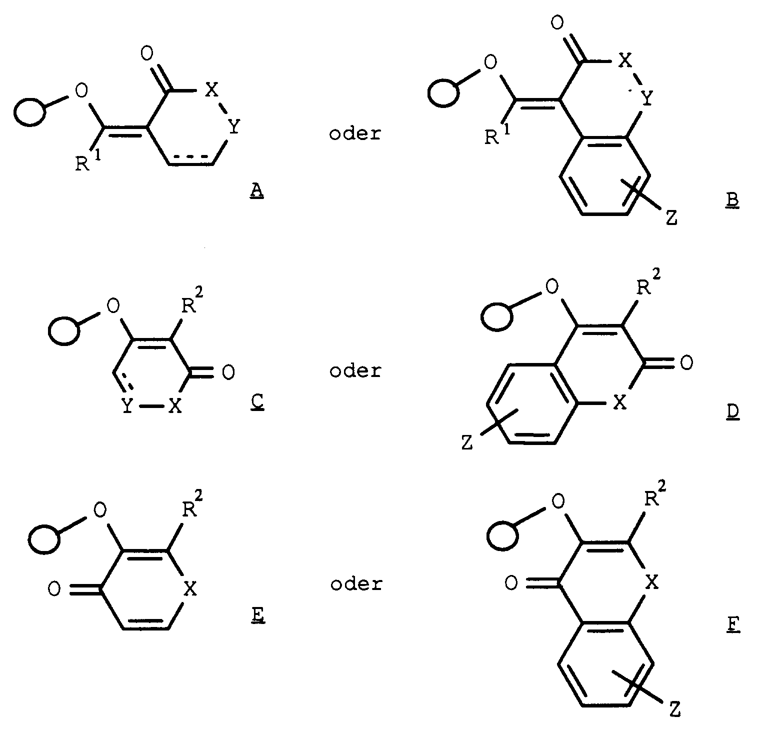
wobei
X
CH₂, O, NR⁴ bedeutet,
Y
CH, (CH₂)ₙ, und n = 0, 1 und 2 bedeutet,
Z
H, Halogen (z.B. Fluor, Chlor, Brom, Jod), C₁-C₄-Alkyl (z.B. Methyl, Ethyl) und C₁-C₄-Alkoxy (z.B. Methoxy, Ethoxy) bedeutet,
R⁴
Wasserstoff und C₁-C₄-Alkyl (z.B. Methyl, Ethyl, n-Propyl und n-Butyl) bedeutet.

Bevorzugt werden Verbindungen der allgemeinen Formel I, in der
A
CHCH₃, CH-CH₂CH₃, CH-OCH₃, NOCH₃
B
OCH₃, NHCH₃
U, V, W
Wasserstoff
R¹, R², R³, R⁴, R⁵
gleich oder verschieden sind und die unter Anspruch 1 gegebene Bedeutung haben.

Die neuen Verbindungen der allgemeinen Formel I können bei der Herstellung aufgrund der C=C- bzw. C=N-Doppelbindungen als E/Z-Isomerengemische anfallen. Diese können in der üblichen Weise, z.B. durch Kristallisation oder Chromatographoe in die einzelnen Komponenten aufgetrennt werden. Sowohl die einzelnen isomeren Verbindungen als auch ihre Gemische werden von der Erfindung umfaßt und sind als Schädlingsbekämpfungsmittel brauchbar.

Die Herstellung der Verbindungen der allgemeinen Formel I gemäß Anspruch 1 erfolgt beispielsweise wie in Schema 1 beschrieben. 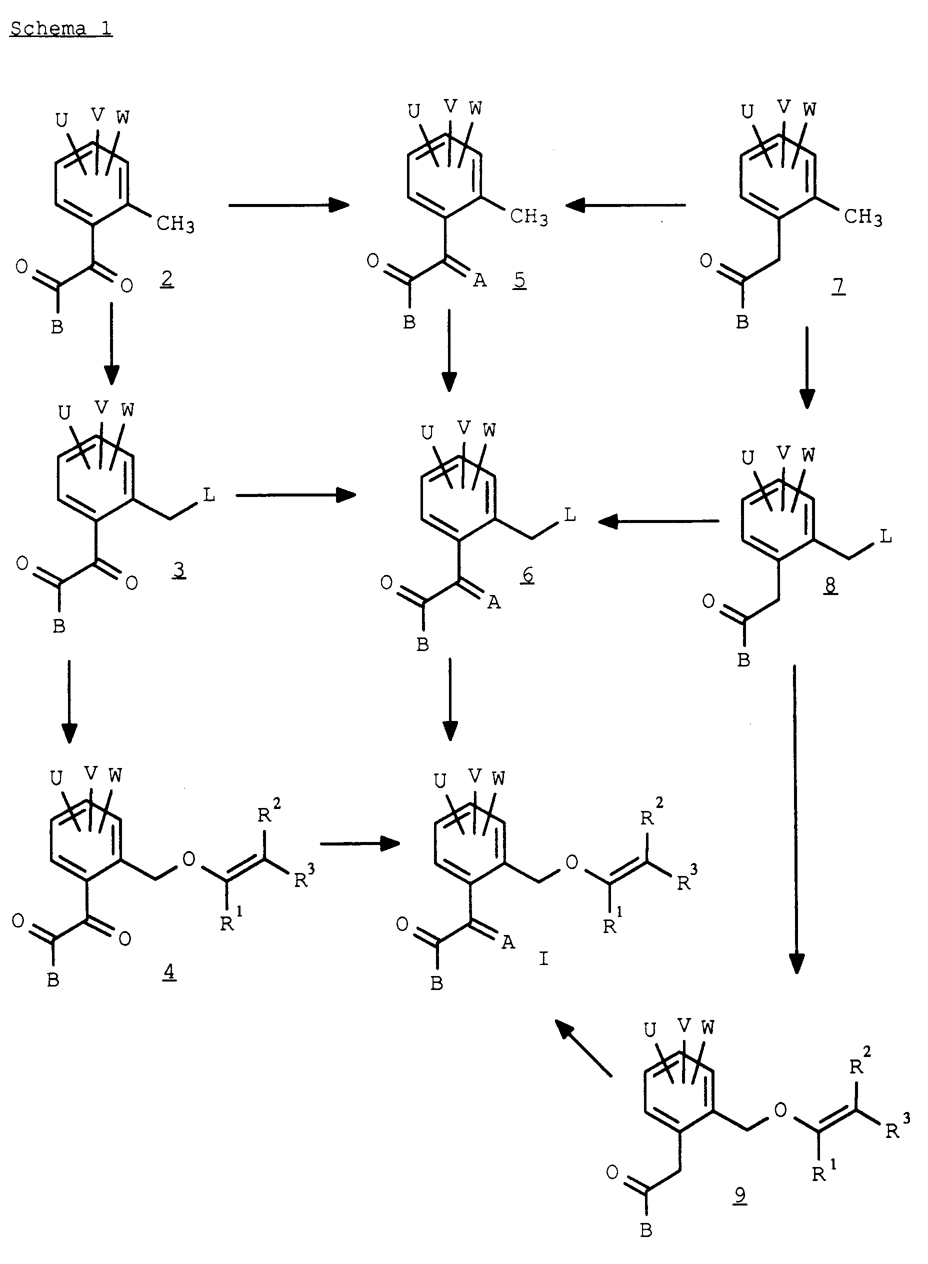
Die Verbindungen der allgemeinen Formel I, in denen A = CH₂, CH-Alkyl, CH-Alkoxy ist, lassen sich beispielsweise aus den Ketoestern 4 durch Wittig- oder Wittig-Horner-Reaktion herstellen (vgl. EP 348 766, DE 3 705 389, EP 178 826). Ebenso erhält man die analogen Verbindungen 5 aus den Ketoestern 2.

Alternativ kann auch so vorgegangen werden, daß man Verbindungen der Formel 7 bzw. 9 mit geeigneten Reagentien kondensiert, z.B: für A = CH₂ mit Formaldehyd (s. DE 3 317 356), für A = CH-Alkyl a) mit Aldehyden (vgl. D.M. Brown, J. Chem. Soc. 1948, 2147) oder b) zuerst mit N,N-Dimethylformamiddimethylacetal, gefolgt von der Reaktion mit einem Grignardreagenz (analog zu C. Jutz Chem. Ber. 91, 1867 (1958)), für A = CH-O-Alkyl mit Ameisensäureester gefolgt von einer Alkylierung (s. EP 178 826). Weitere Herstellvorschriften für die Verbindungen der Formel 5 und I mit A = CH-O-Alkyl sind beschrieben in EP 178 826.

Eine weitere Möglichkeit zur Herstellung der Verbindungen der Formel I mit A = CH-Alkyl und B = O-Alkyl ist die Umsetzung von Ketenacetalen mit Phenylchlorcarbonaten (s. N. Slougui, G. Rousseau, Synth. Commun. 12 (5) 401-7 (1982)).

Für Verbindungen der allgemeinen Formel I in der A = CH-S-Alkyl und A = CHCl ist, kann die Herstellung nach den Methoden aus EP 244 077 oder 310 954 erfolgen.

Die Zwischenprodukte der Formel 3, 6 und 8 lassen sich aus den Verbindungen 2, 5 und 7 herstellen, indem man diese nach bekannten Methoden halogeniert, z.B. mit Chlor, Brom, N-Bromsuccinimid in einem inerten Lösungsmittel (z.B. CCl₄, Cyclohexan) unter Belichtung mit z.B. einer Hg-Dampflampe oder Radikalstartern, wie z.B. Dibenzoylperoxid, oder indem man über geeignete Zwischenverbindungen (L = Halogen, OH) die Reste L wie z.B. Mesylat, Tosylat, Acetat oder Triflat einführt.

Die Oximether der Formel I mit A = N-O-Alkyl lassen sich aus 4 a) durch Umsetzung mit O-Alkylhydroxylaminhydrochlorid oder b) mit Hydroxylaminhydrochlorid und nachfolgender Alkylierung mit einem Alkylierungsmittel (wie z.B. Alkyliodid, Dialkylsulfat etc.) herstellen (vgl. DE 3 623 921).

Ebenso kann analog zur Methode im EP 254 426 ein Phenylessigester der Formel 9 mit einer Base (wie z.B. NaOMe, NaH, K-tert.-Butylat, etc.) in einem Lösungsmittel (wie z.B. Diethylether, Toluol, tert.-Butanol etc.) in sein Anion überführt und mit einem geeigneten Nitrosierungsmittel (wie z.B. Methylnitrit, Amylnitrit, tert.-Butylnitrit etc.) oximiert werden. Das resultierende Oximat wird mit einem Alkylierungsmittel (wie z.B. Alkyliodid, Dialkylsulfat) alkyliert.

Dieselben Verfahren sind entsprechend auch auf die Verbindungen der Formel 2 und 7 übertragbar, wobei die resultierenden Oximether 5 wie bekannt (EP 254 426) über die Intermediate 6 (L = z.B. Halogen) in die Zielverbindungen I überführt werden können.

Üblicherweise ist bei den vorbeschriebenen Herstellungsverfahren der Rest B = Alkoxy und U, V, W = H.

Die Verbindungen mit B = OH (11) lassen sich nach literaturbekannten Methoden (Organikum 16. Auflage, S. 415, 622) aus den Verbindungen der allgemeinen Formel I, in der B = O-Alkyl (10) ist, herstellen (s. Schema 2): 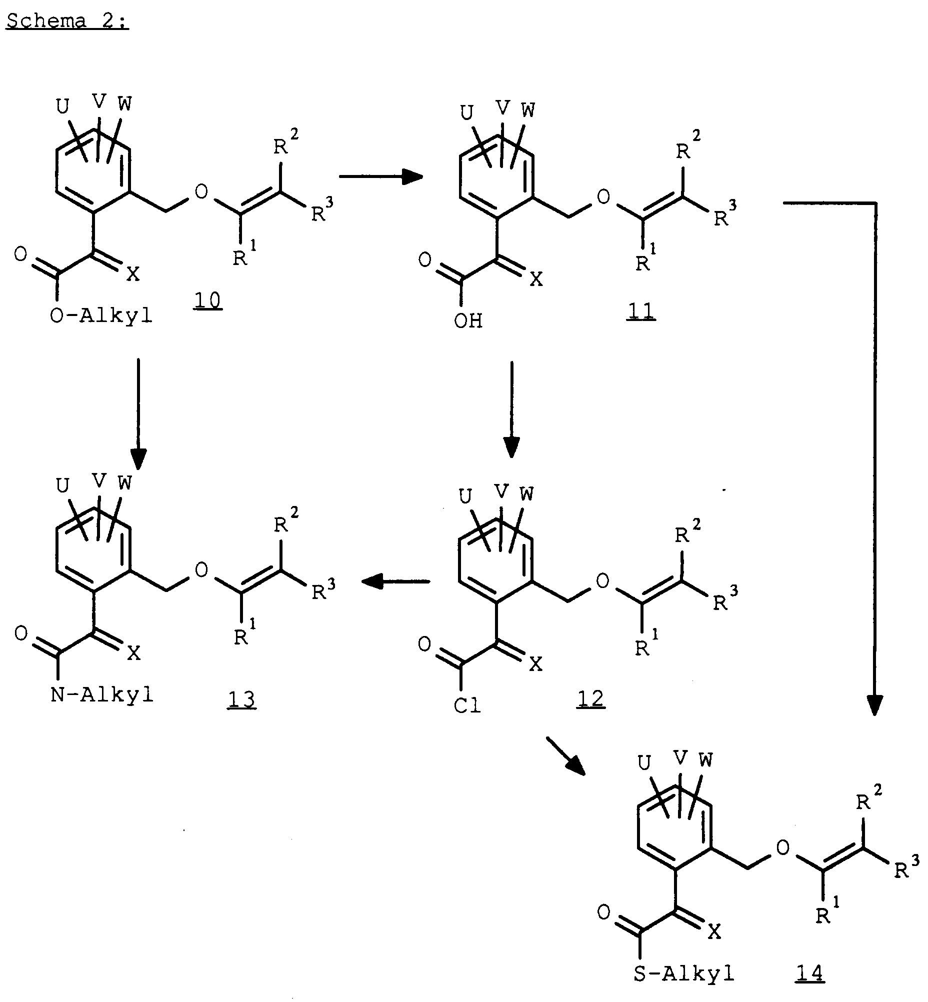
Aus den so erhaltenen Carbonsäuren 11 lassen sich in an sich bekannter Weise die Säurechloride 12 herstellen (vgl. Organikum 16. Auflage, S. 423f B. (1985)). Die Umwandlung von 12 in die Amide 13 erfolgt analog zu Organikum 16. Auflage S. 412 (1985).

Die Thiolester 14 erhält man aus den Säurechloriden 12 (analog zu Houben-Weyl Bd. 8, S. 464ff (1952)).

Alternativ können die Thiolester 14 auch aus den Säuren 11 hergestellt werden (analog zu Houben-Weyl Bd. E5, S. 855f (1985)).

Die Herstellung der Amide 13 kann auch nach literaturbekannten Verfahren aus dem Ester 10 erfolgen.

Die Herstellung der Verbindung der allgemeinen Formeln 2 und 7 mit ortho-Methylsubstitution am Aromaten ist bekannt (B = O-Alkyl; U, V, W = H; s. EP 178 826, EP 260 832).

Die zur Herstellung der Verbindungen der allgemeinen Formel I benötigten Enole sind entweder bekannt oder können nach an sich bekannten Methoden hergestellt werden, beispielsweise durch Umsetzung einer aktivierten Methyleneinheit 15 mit einem Ameisensäureester, in Gegenwart einer Base, wie z.B. NaH, oder Natrium-methanolat (vgl. Schema 3). 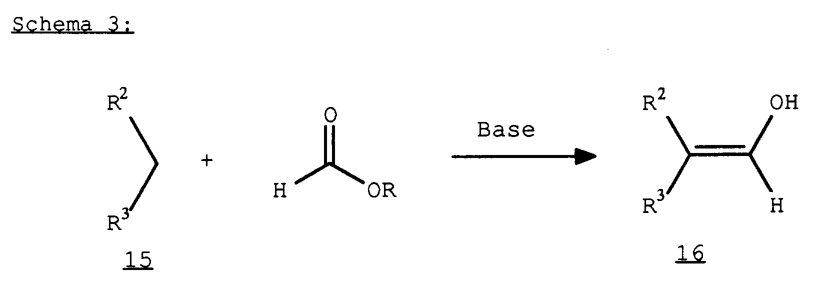
Weitere Methoden zur Darstellung finden sich in Houben-Weyl, "Methoden der Organischen Chemie", Bd 6/11, Seite 1-217 (1977).

Die Darstellung der neuen Verbindungen der allgemeinen Formel I gemäß Anspruch 1 erfolgt beispielsweise so, daß man ein Enol 16 a bzw. das tautomere Keton 17 a mit einer Benzylverbindung der Formel 6 umsetzt. 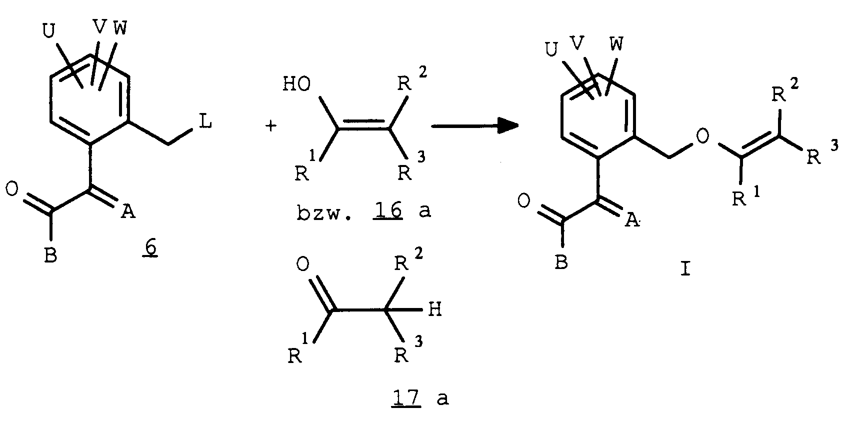
R¹ - R³, A, B, U, V, W haben die in Anspruch 1 beschriebene Bedeutung; L steht für eine Abgangsgruppe (z.B. Chlorid, Bromid, Jodid, Triflat, Methansulfonat, p-Toluolsulfonat).

Die beschriebenen Umsetzungen können z.B. in einem inerten Lösungs- oder Verdünnungsmittel (z.B. Aceton, Acetonitril, Dimethylformamid, Dimethylsulfoxid, N-Methylpyrrolidon) unter Verwendung einer Base (z.B. Natriumcarbonat, Kaliumcarbonat, Natriumhydroxid, Kaliumhydroxid, Natriumhydrid, Natriummethylat) durchgeführt werden.

Die Umsetzung kann auch im Zweiphasensystem (z.B. Dichlormethan, Wasser) unter Zusatz eines geeigneten Phasentransferkatalysators (z.B. Cetyltrimethylammoniumchlorid, Benzyltriethylammonimchlorid) durchgeführt werden.

### Beispiele

### E/Z 2-Methoximino-2-[2-cyano-2-(4-methyl)-phenylethen-1-yl-oxymethyl] phenylessigsäuremethylester (Verb. 2.100; Tabelle 2)

Zu einer Lösung von 5 g (17,5 mmol) 2-Methoximino-2-(2'-Brommethyl)phenylessigsäuremethylester in 80ml Aceton gibt man 2,8 g (17,5 mmol) 3-Hydroxy-2-(4-methylphenyl)acrylnitril, 2,6 g Kaliumcarbonat und 100 mg Kaliumjodid und rührt 16 Stunden bei Raumtemperatur (20°C). Anschließend gießt man die Mischung auf Wasser, extrahiert mit Essigester, trocknet die organische Phase und engt ein. Es verbleiben 6,2 g (97 %) farbloser Feststoff, mit einem Schmelzpunkt von 93-95°C. Isomerenverteilung laut GC: 7:1. E- zu Z-Isomer
¹H-NMR (CDCl₃): δ = 2,34 (s, 3H, Isomer 1 + 2); 3,73; 4,03 (s, OCH₃, Isomer 1); 3,87; 4,03 (s, OCH₃, Isomer 2); 4,99 (s, CH₂, Isomer 2); 5,00 (s, CH₂, Isomer 1); 6,97 - 7,61 ppm (m, Aryl-H und =CH).

### E/Zα-[2{2-Cyano-2-(4-Methyl)phenyl-ethen-1-yloxy}methylphenyl]-β-methoxy-acrylsäuremethylester (Verb. 1.100; Tabelle 1)

Zu einer Lösung von 3 g (11 mmol) 3-Methoxy-2-(2'-Brommethyl)phenyl-acrylsäuremethylester in 50 ml Aceton gibt man 1,7 g (11mmol) 3-Hydroxy-2-(4-Methylphenyl)acrylonitril, 1,6 g Kaliumcarbonat und 100 mg Kaliumjodid und rührt 16 Stunden bei Raumtemperatur. Man gießt auf Wasser, extrahiert mit Essigester, trocknet die org. Phase und engt ein. Es verbleiben 3,6 g (92,7 %) eines gelben Öls. Isomerenverhältnis laut GC: 5:1 E- zu Z-Isomer.
¹H-NMR (CDCl₃) : δ = 2,55 (s, 2x CH₃); 3,66; 3,77 (s, OCH₃; Isomer 1); 3,71; 3,82 (s, OCH₃; Isomer 2); 5,04 (s, CH₂; Isomer 2); 5,05 (s, CH₂; Isomer 1); 7,03 - 7,65 (m, Aryl-H; =CH).

Die folgenden Verbindungen können in entsprechender Weise hergestellt werden.

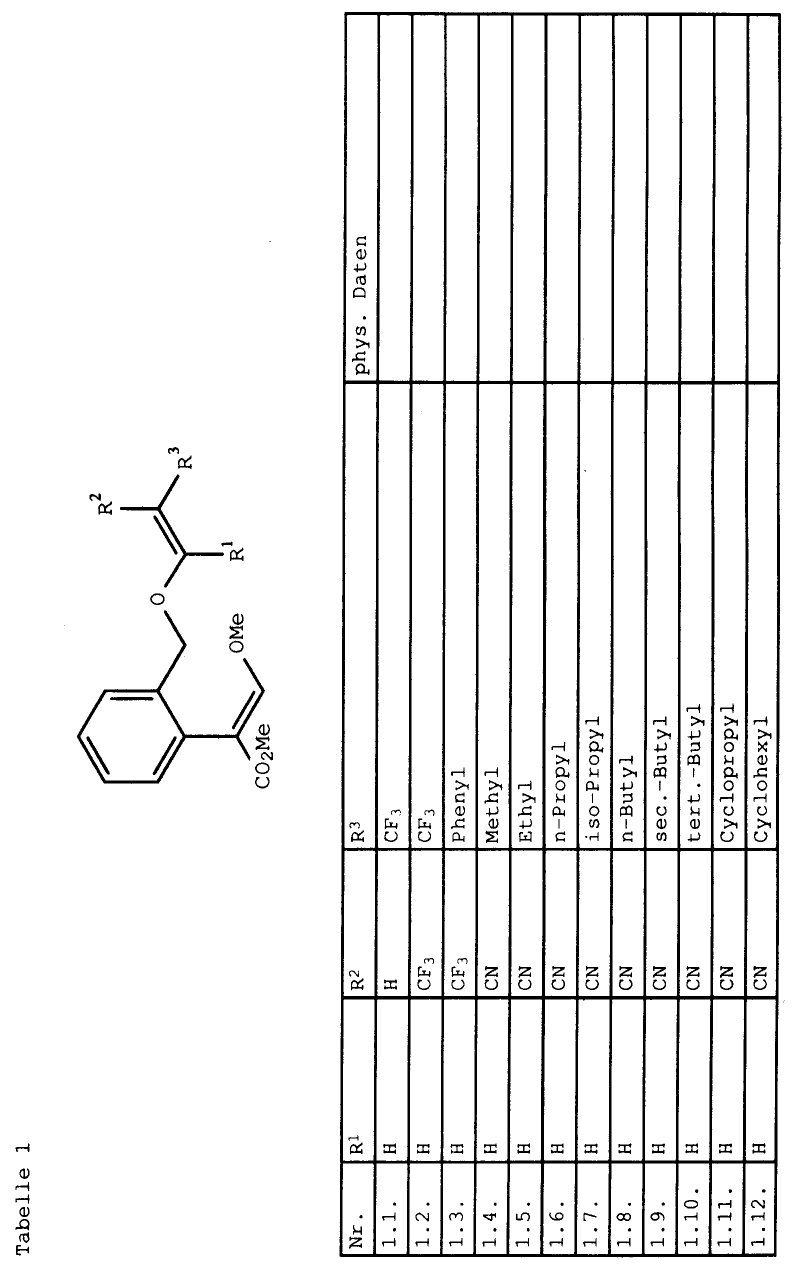

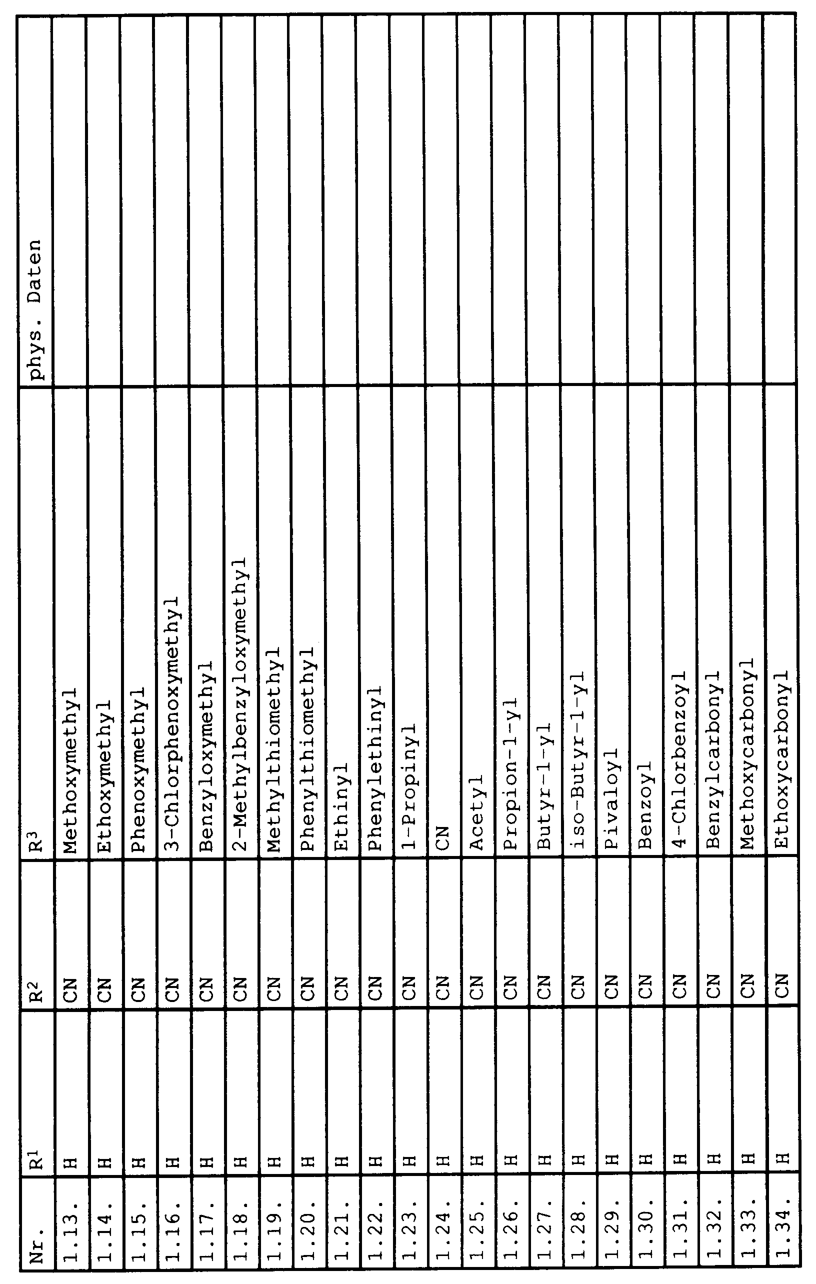

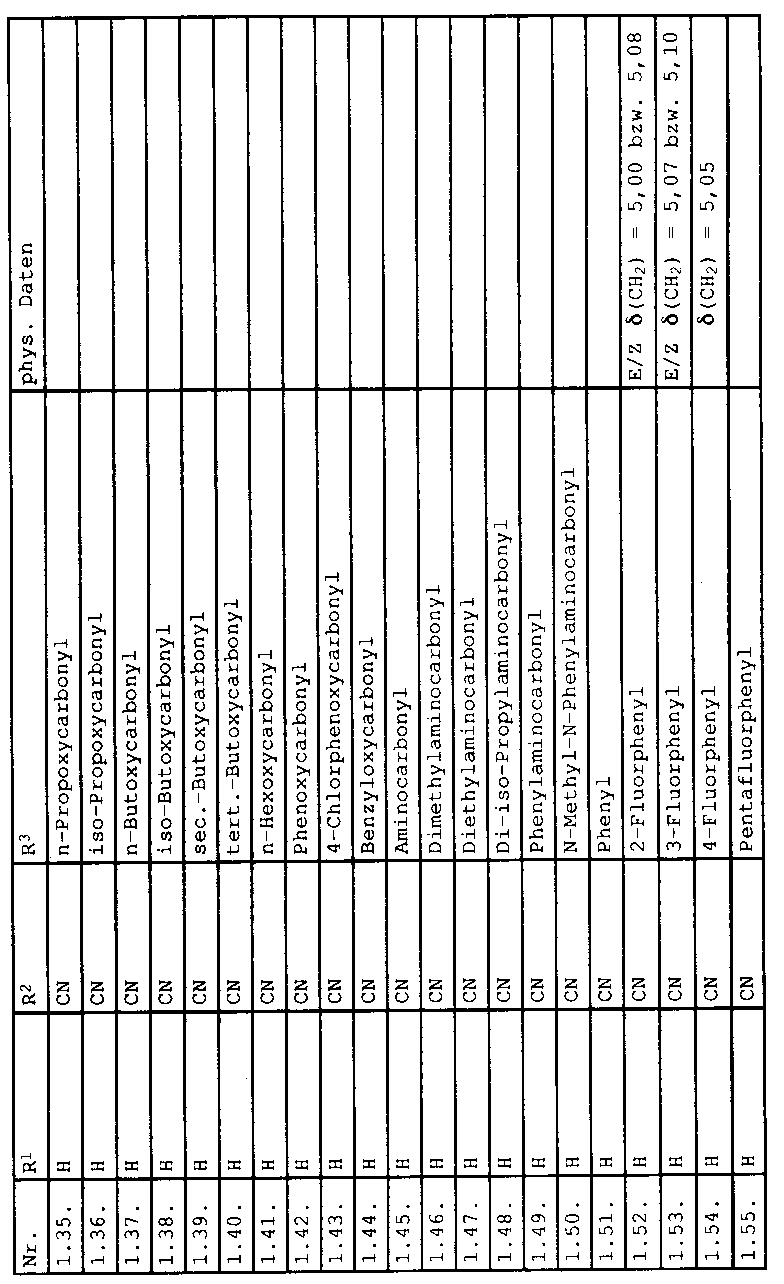

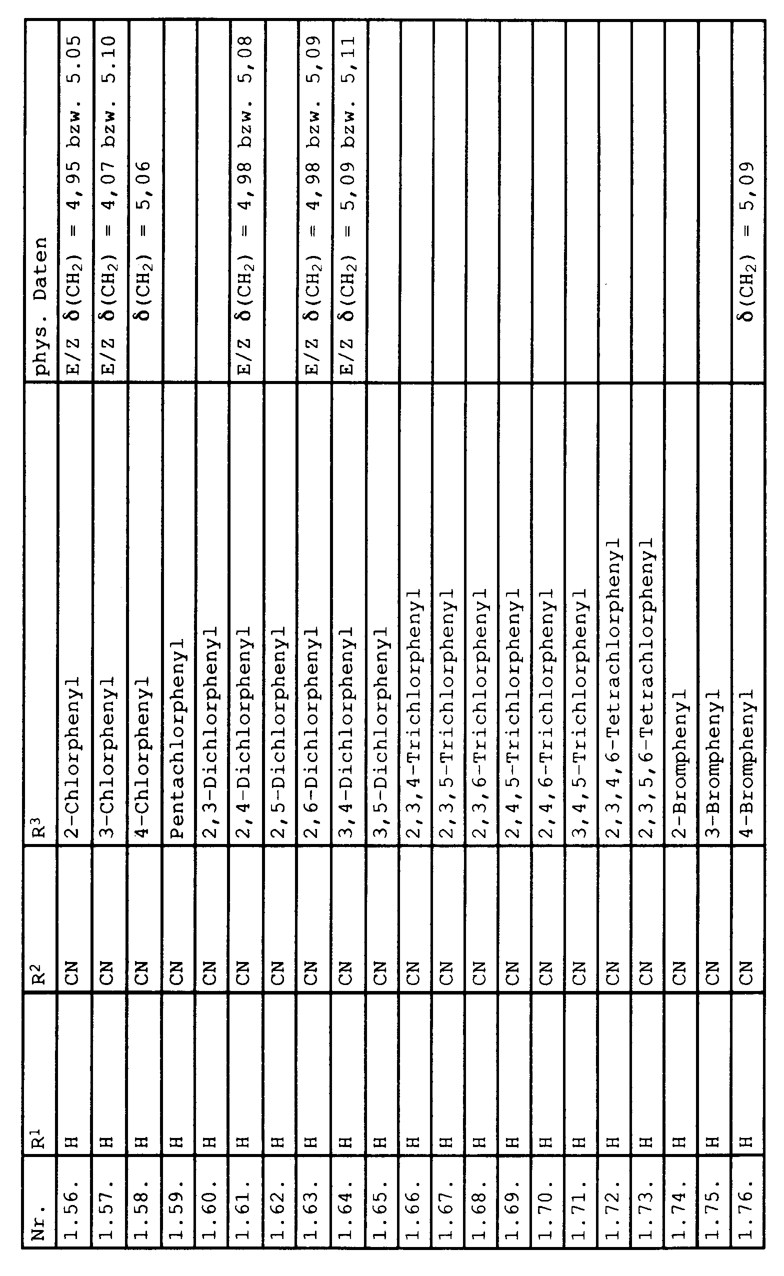

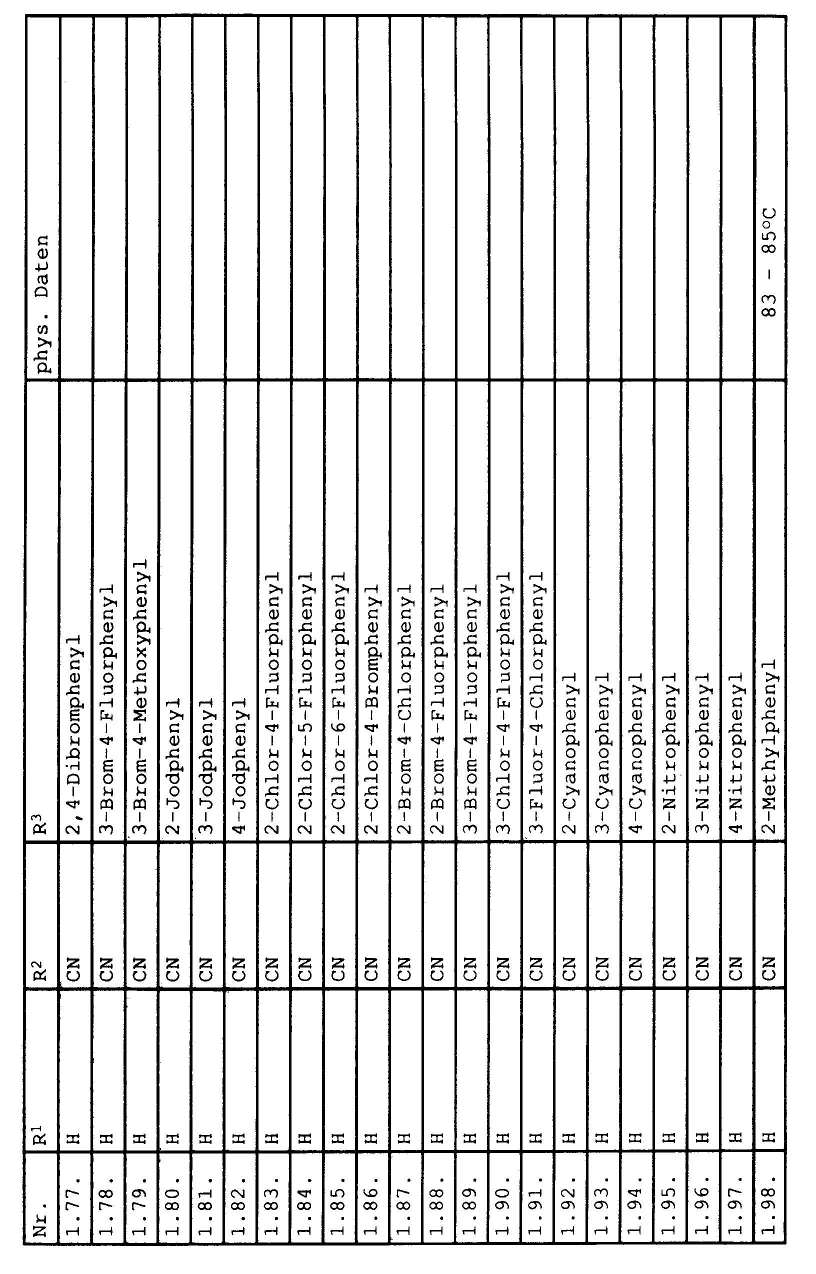

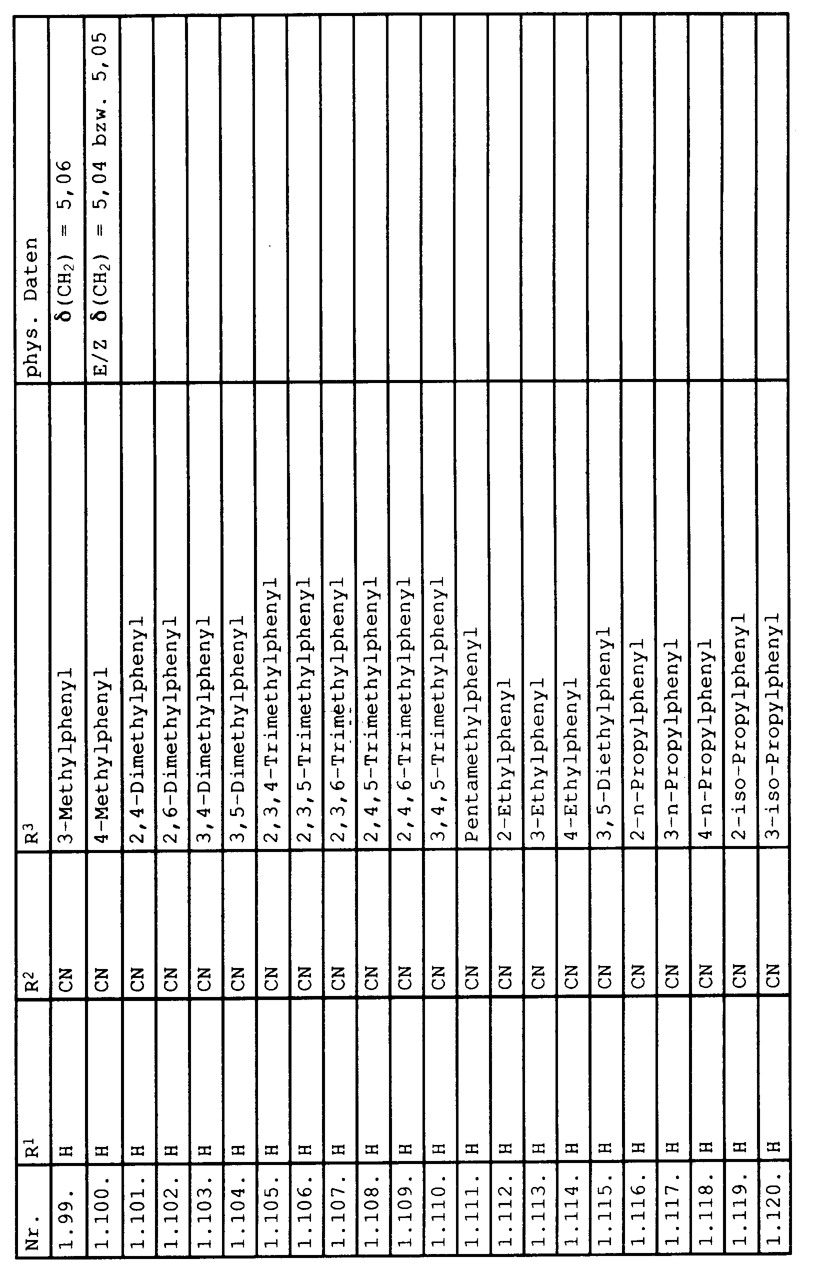

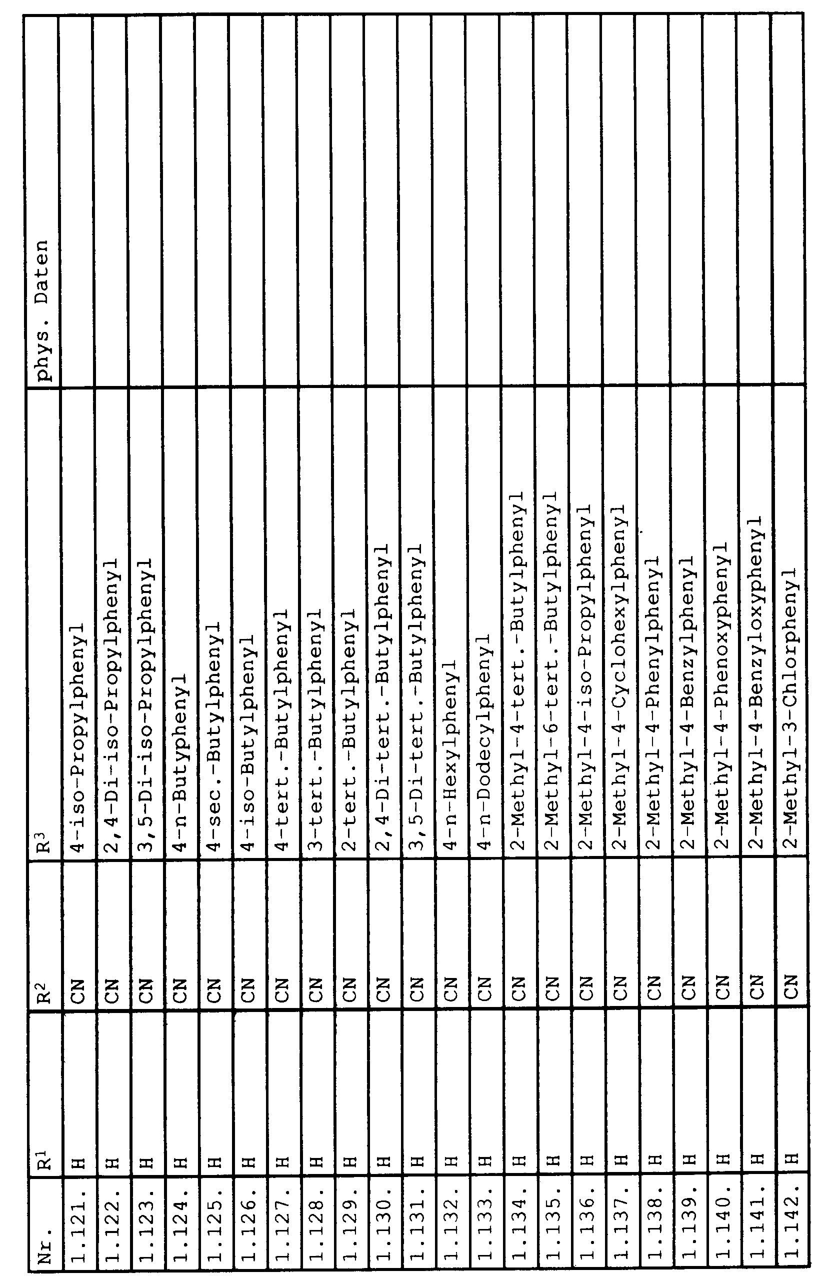

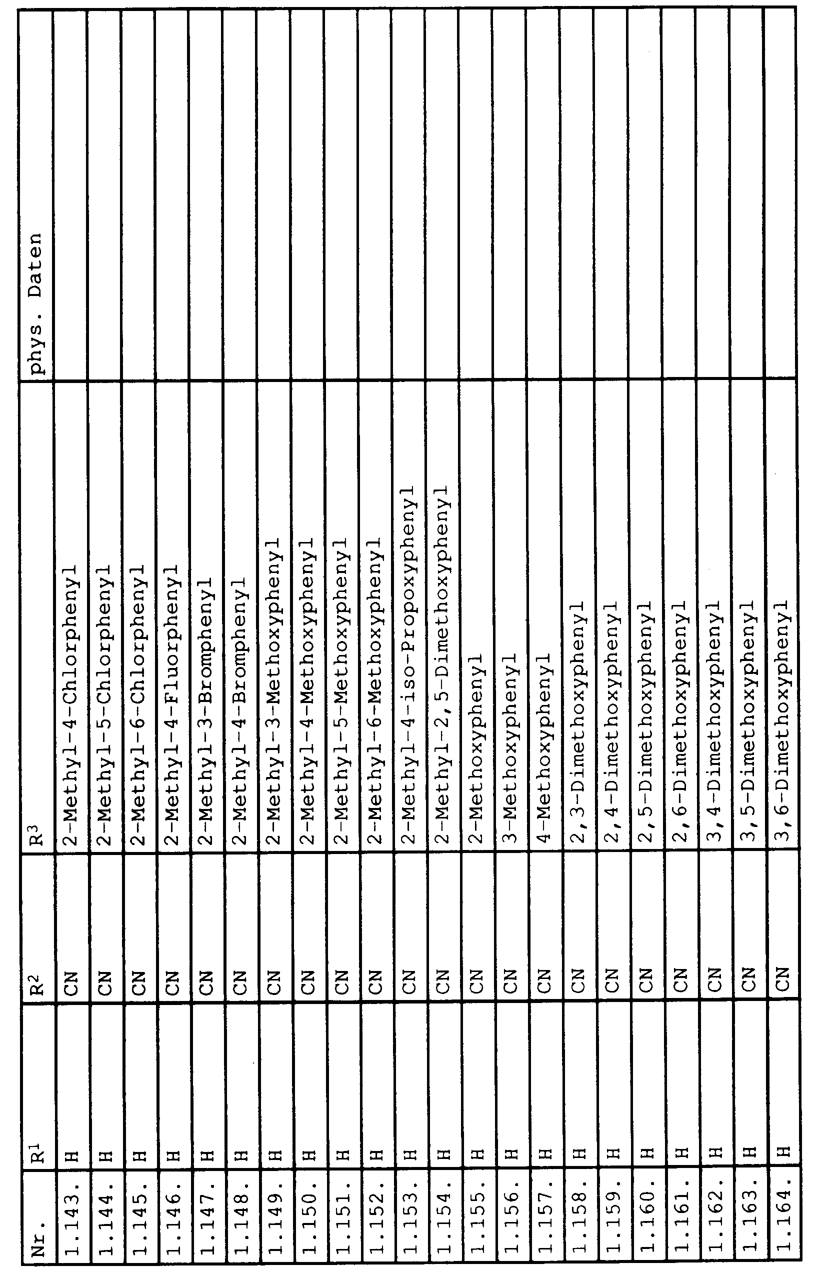

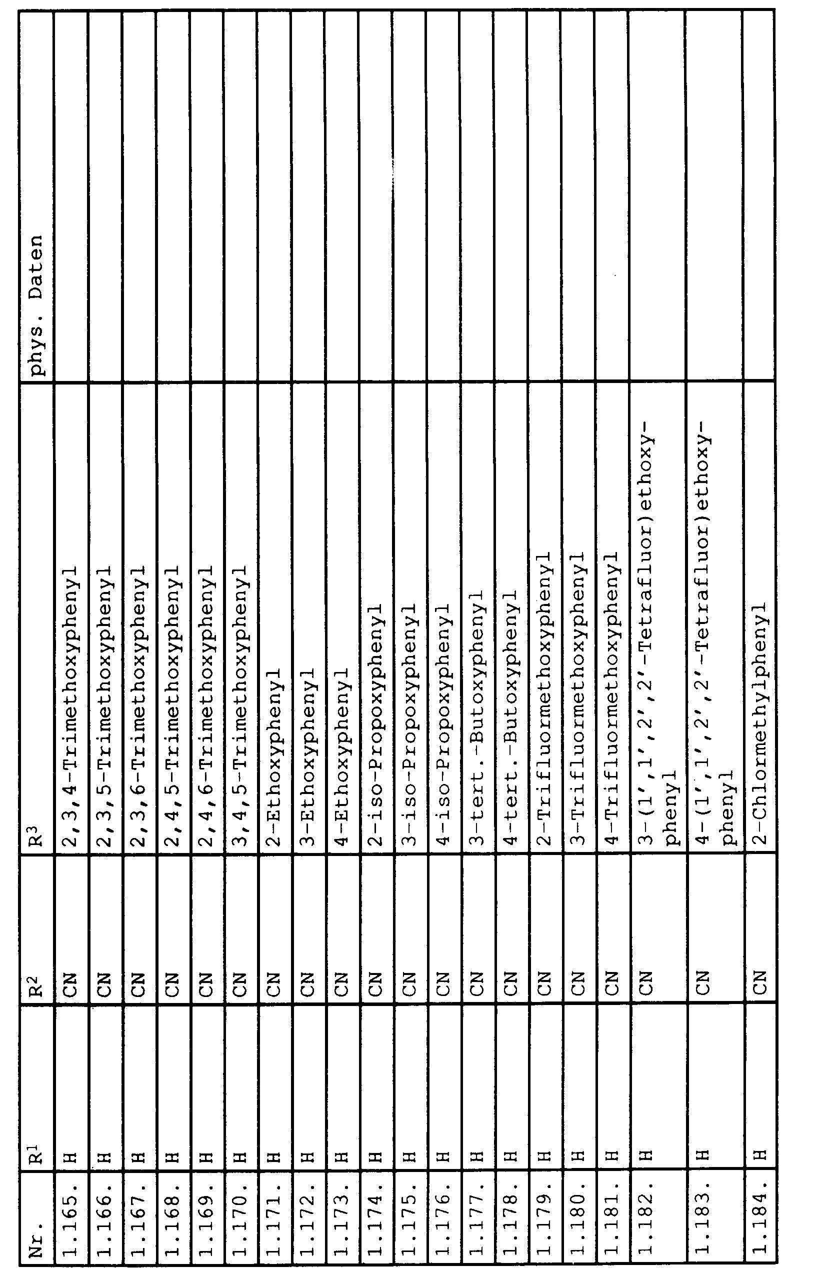

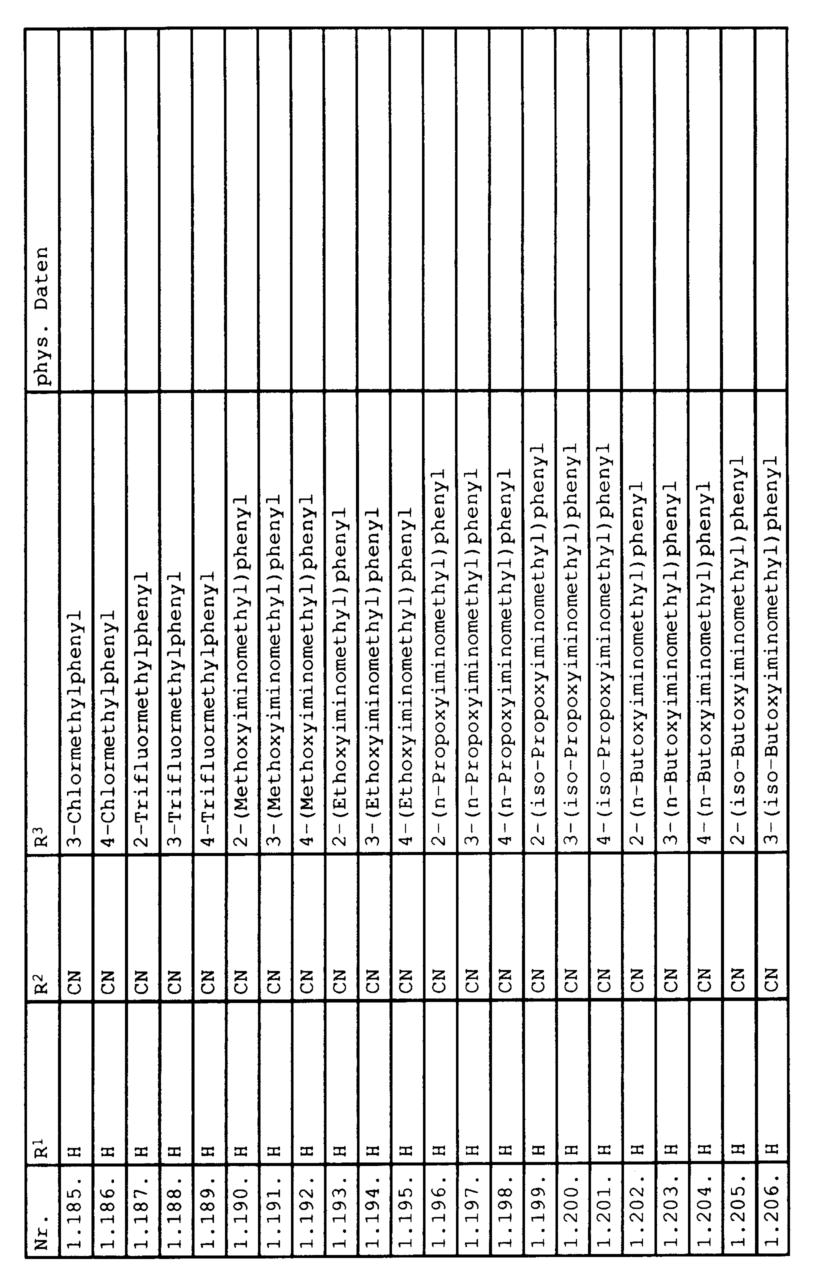

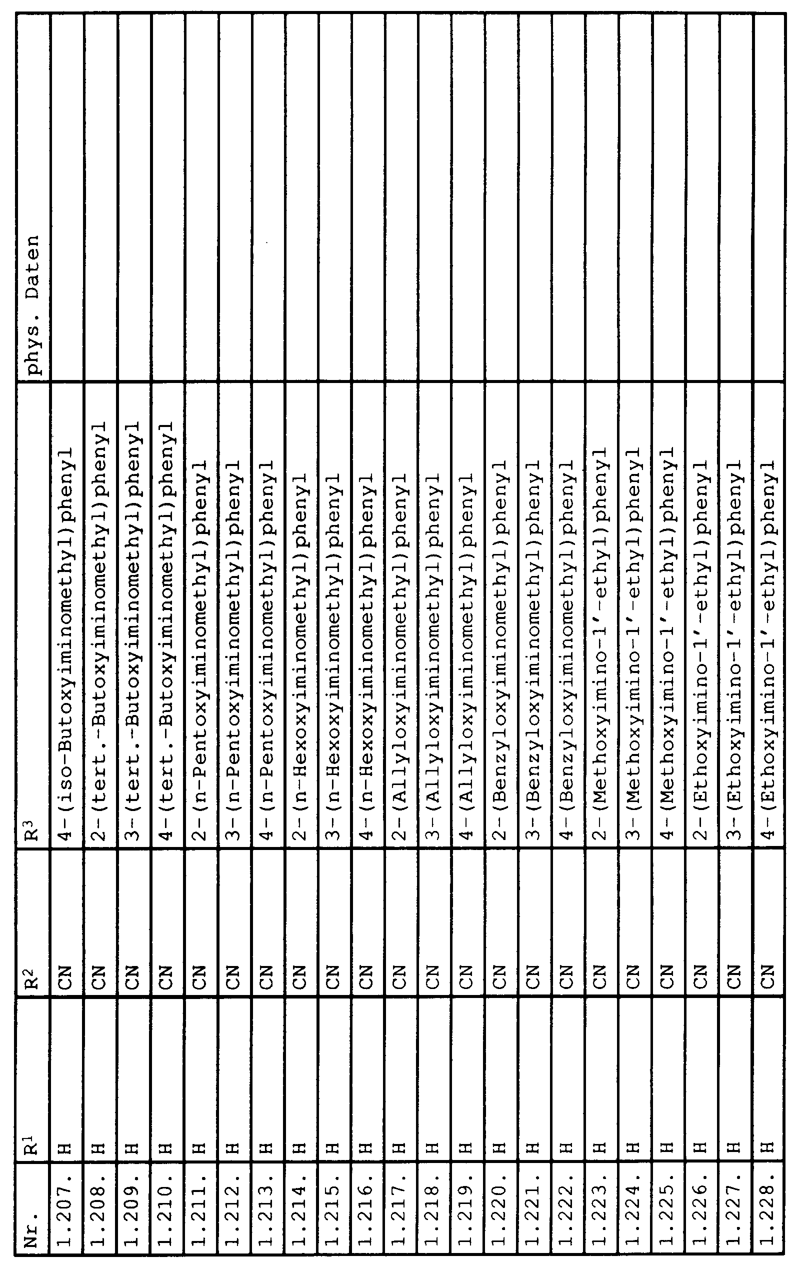

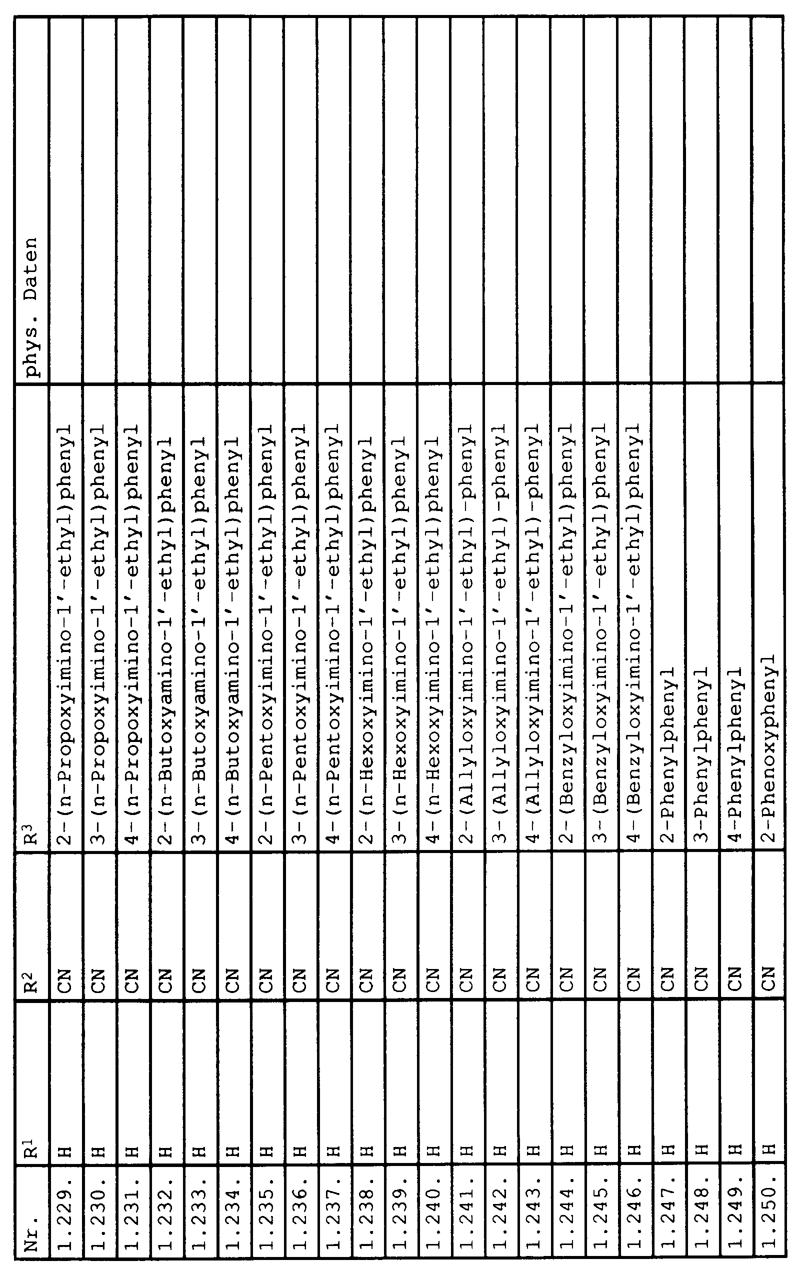

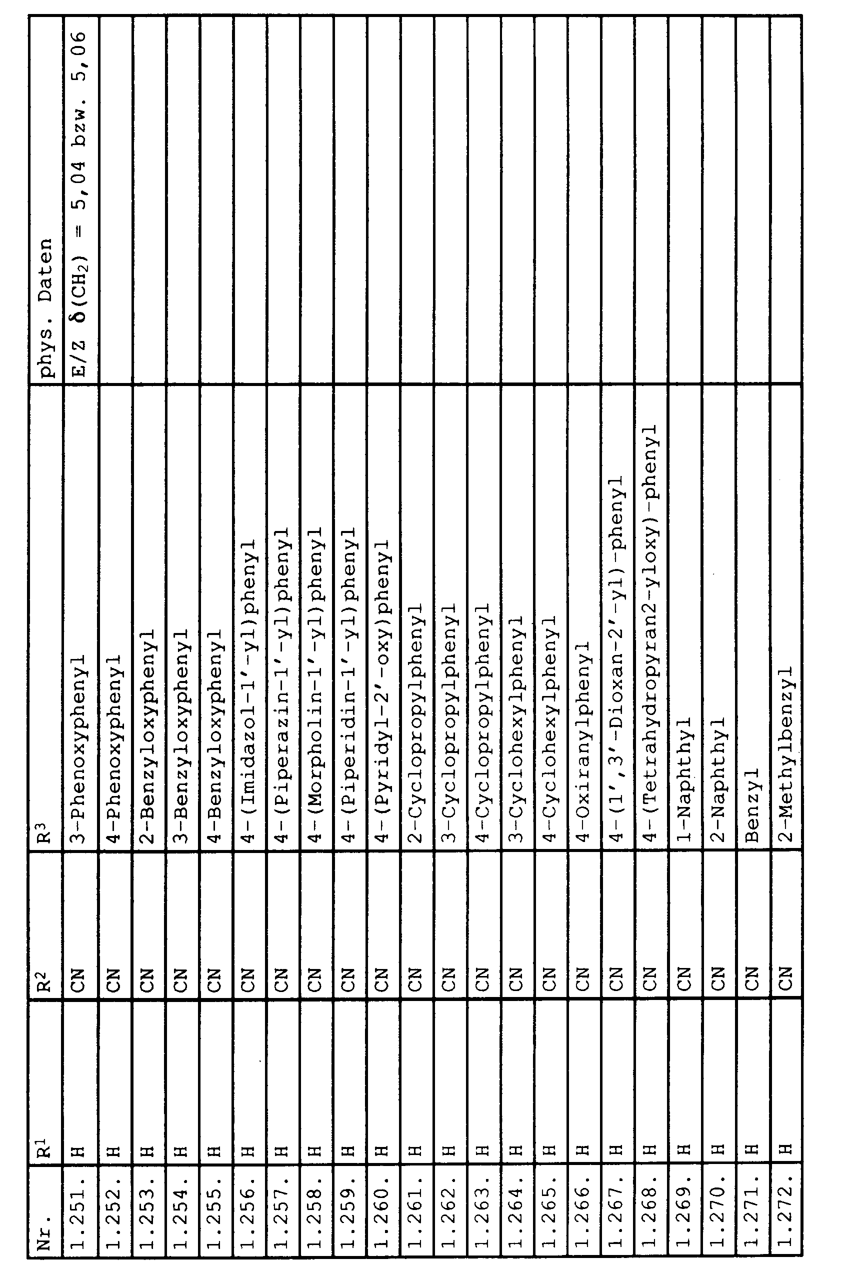

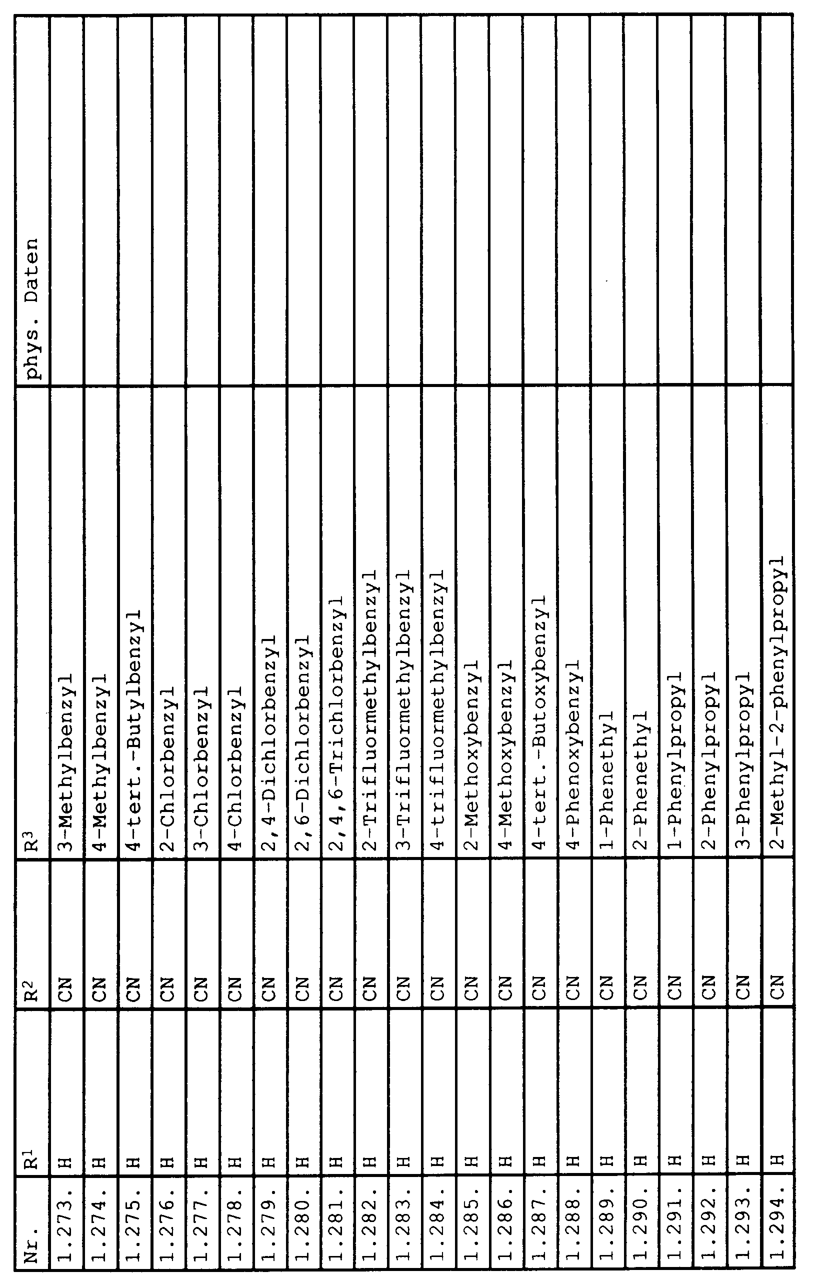

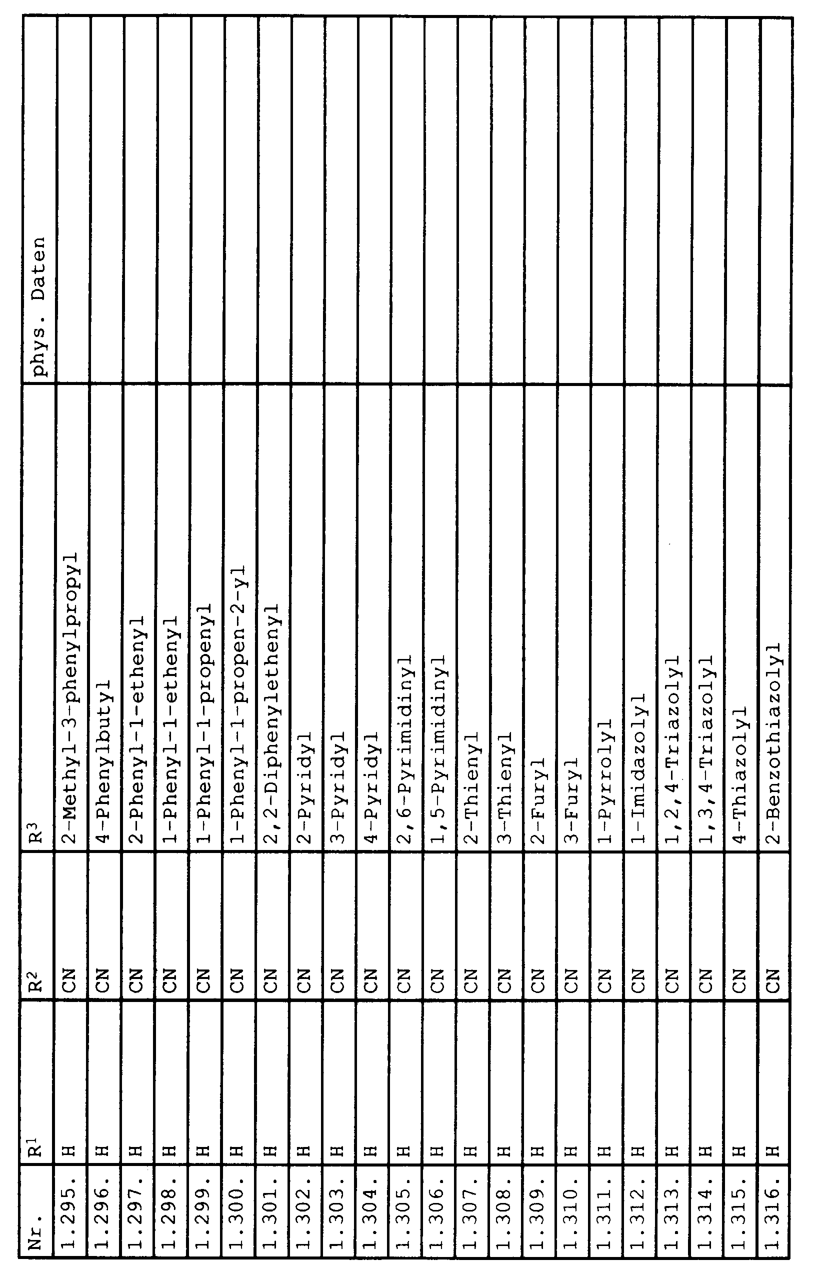

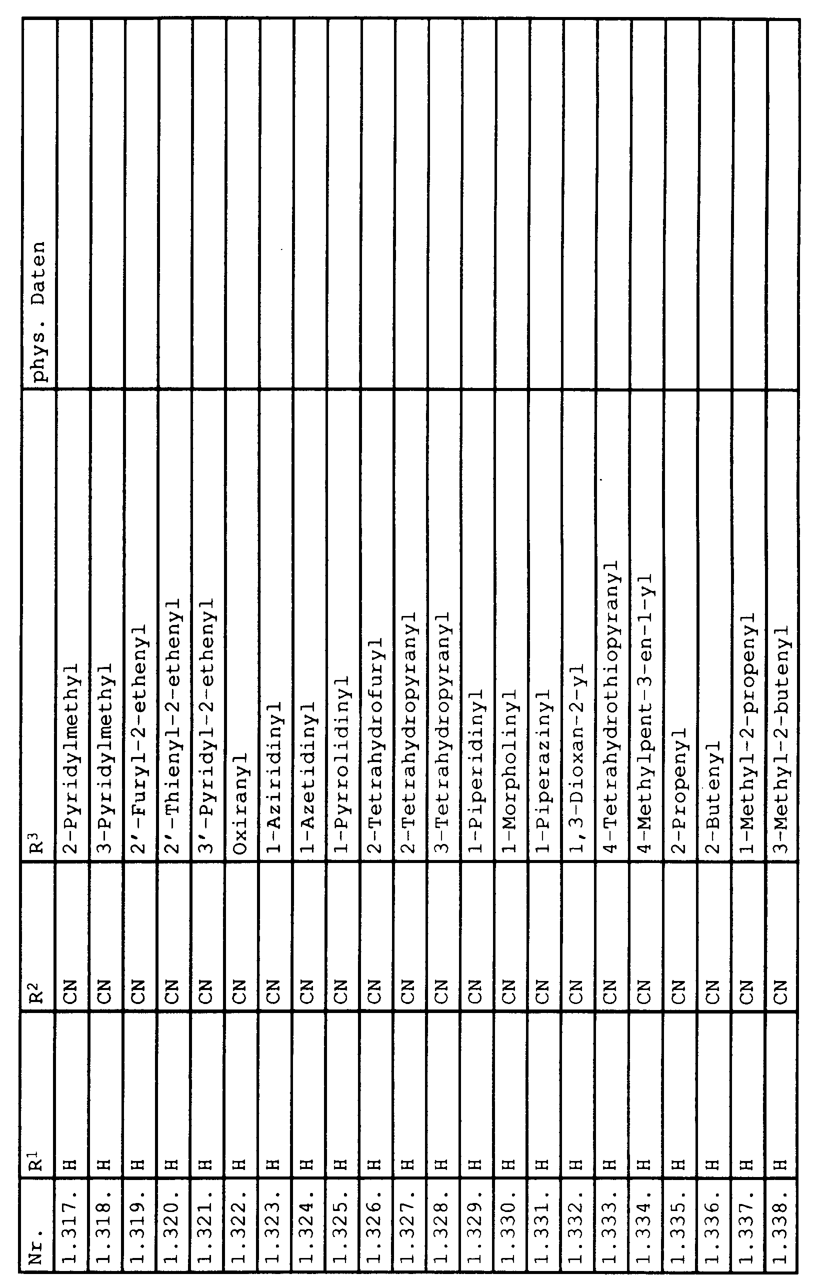

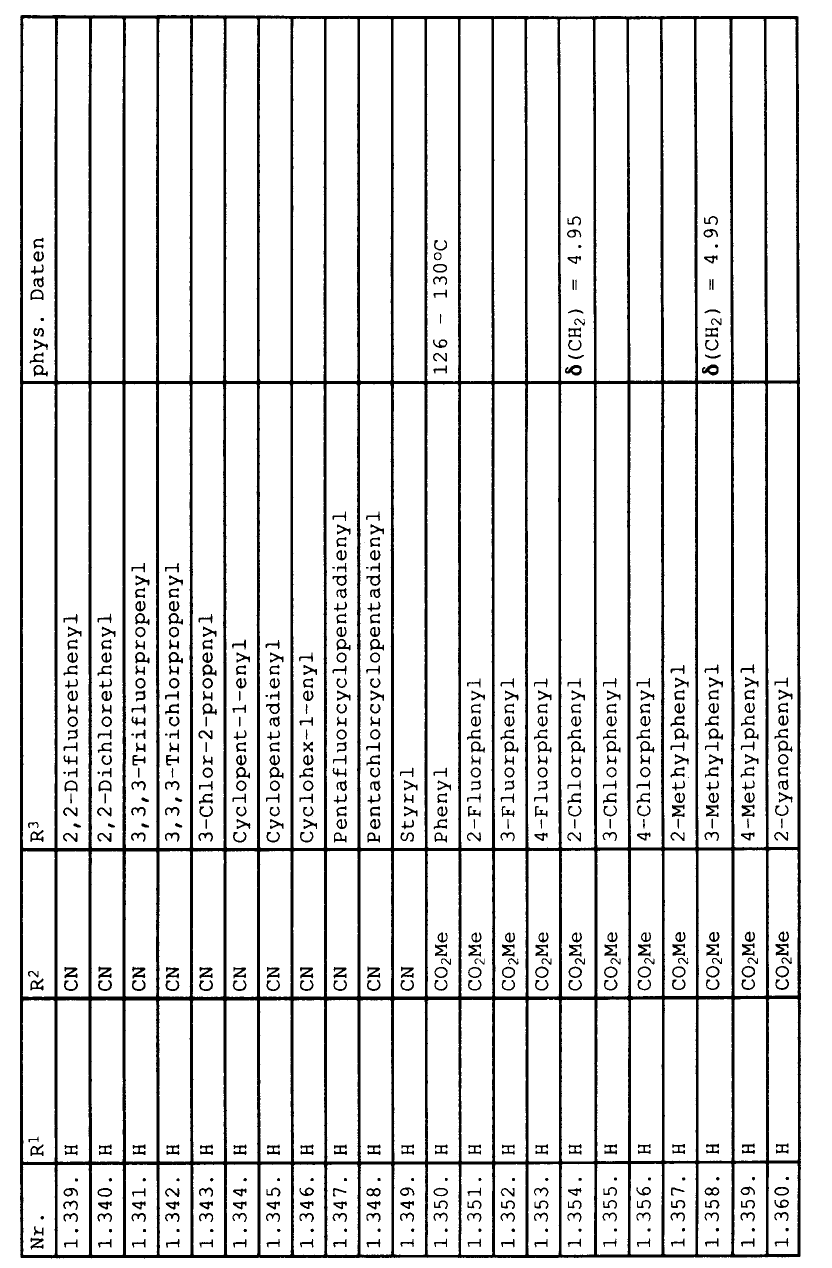

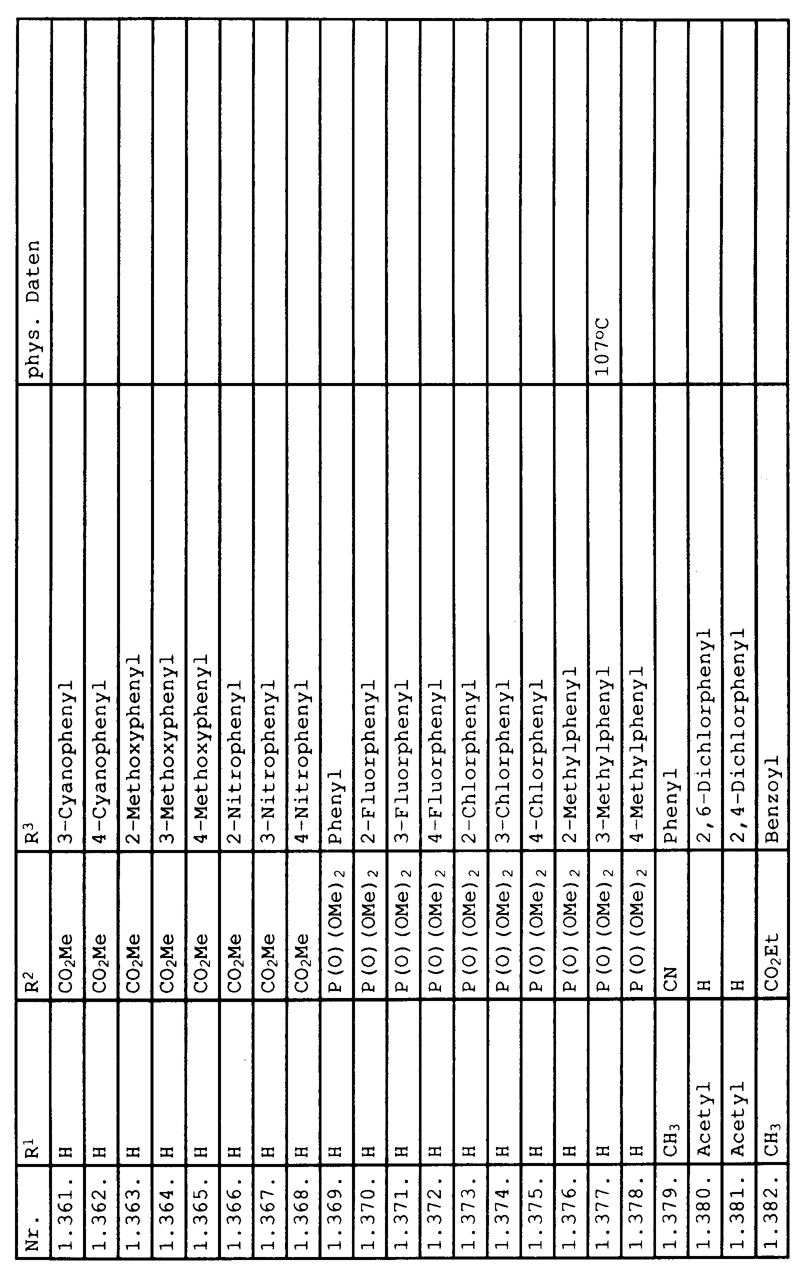

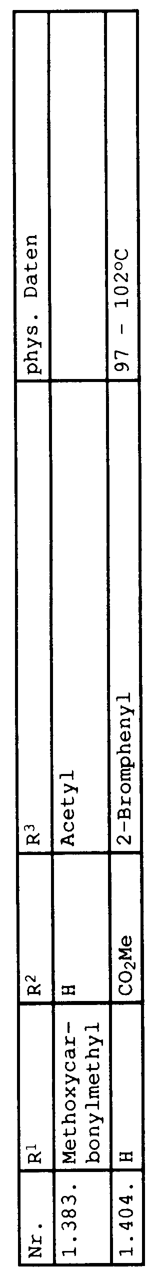

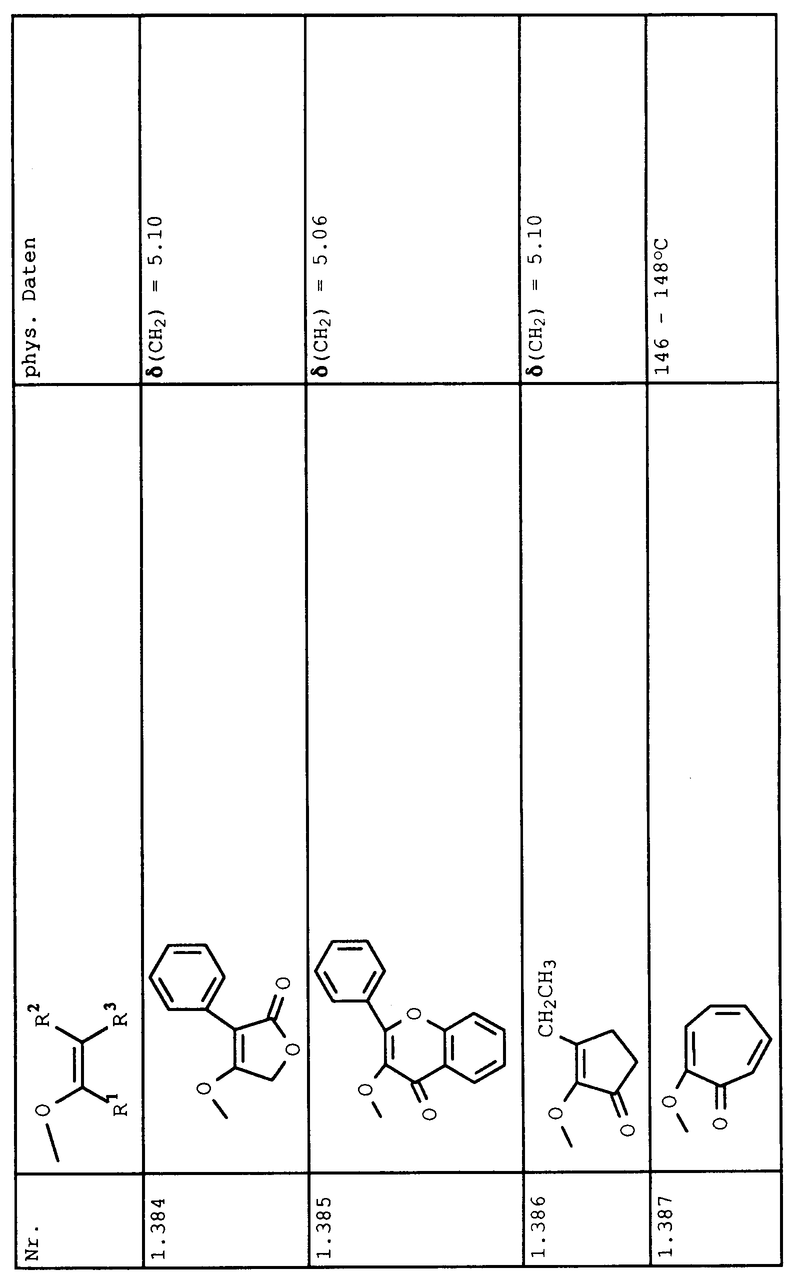

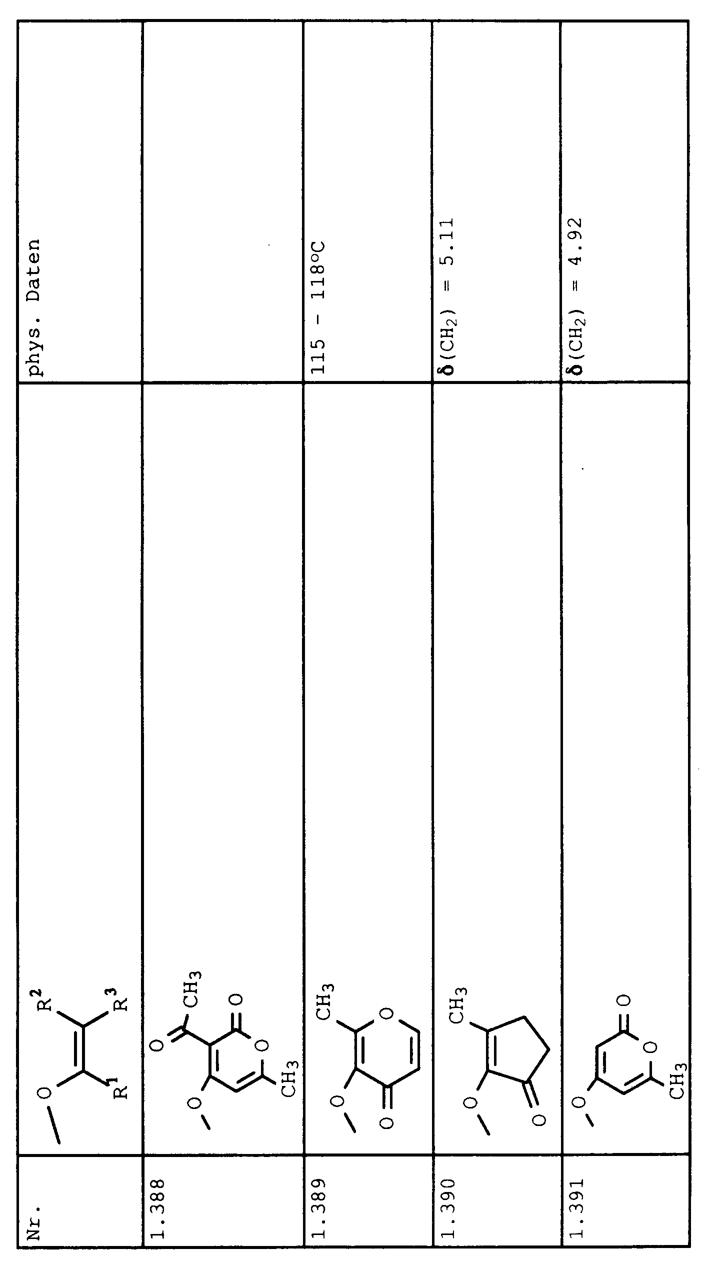

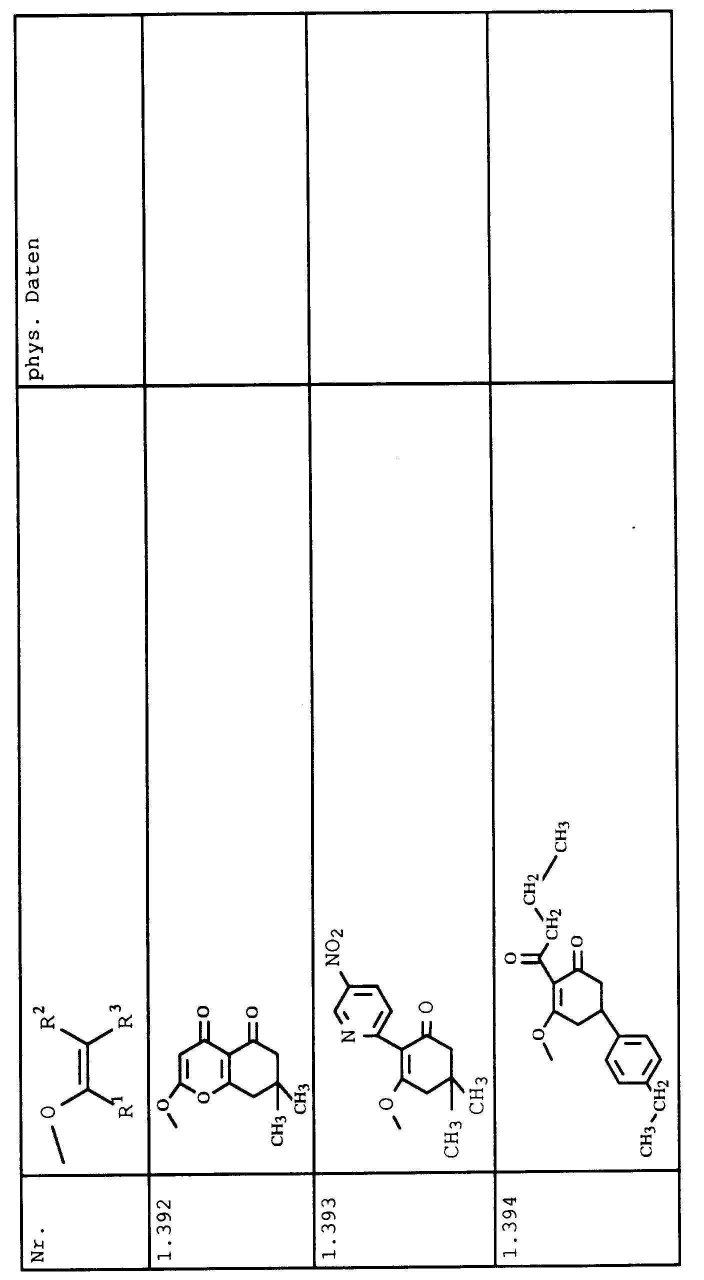

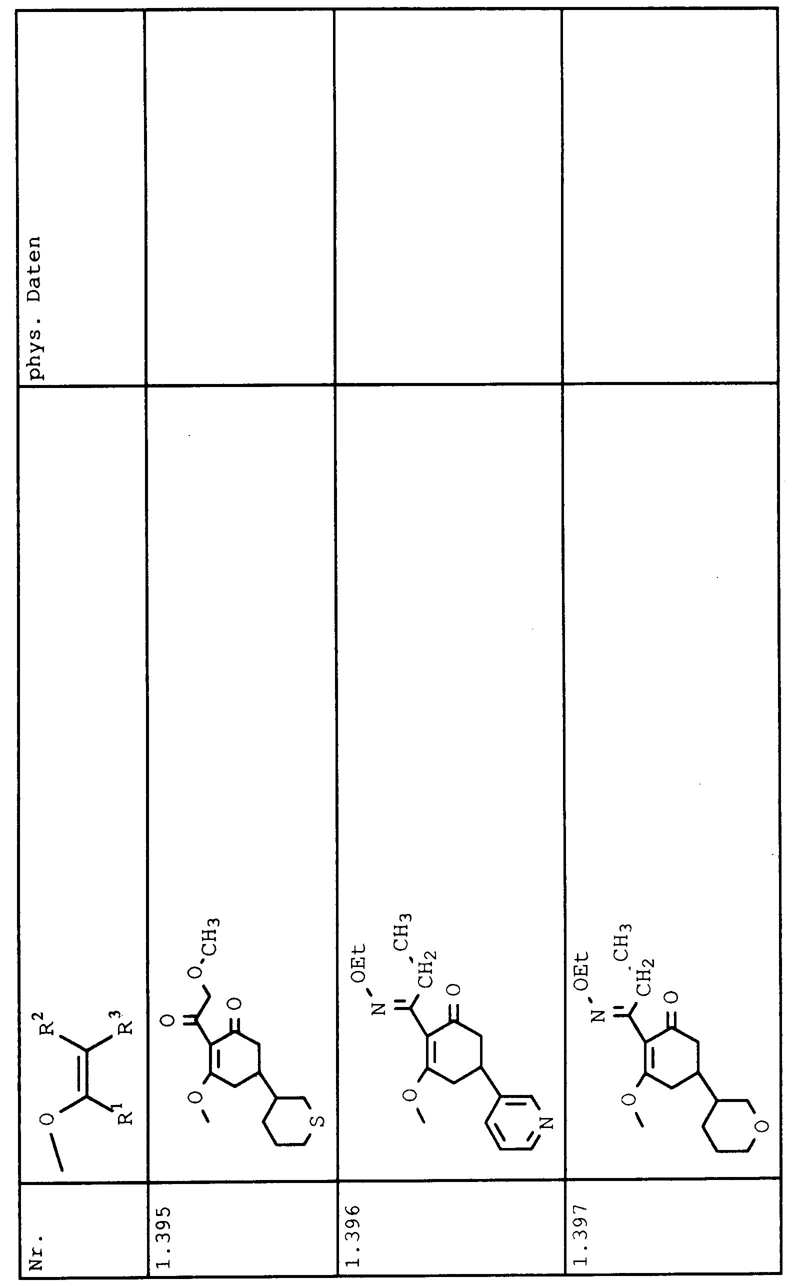

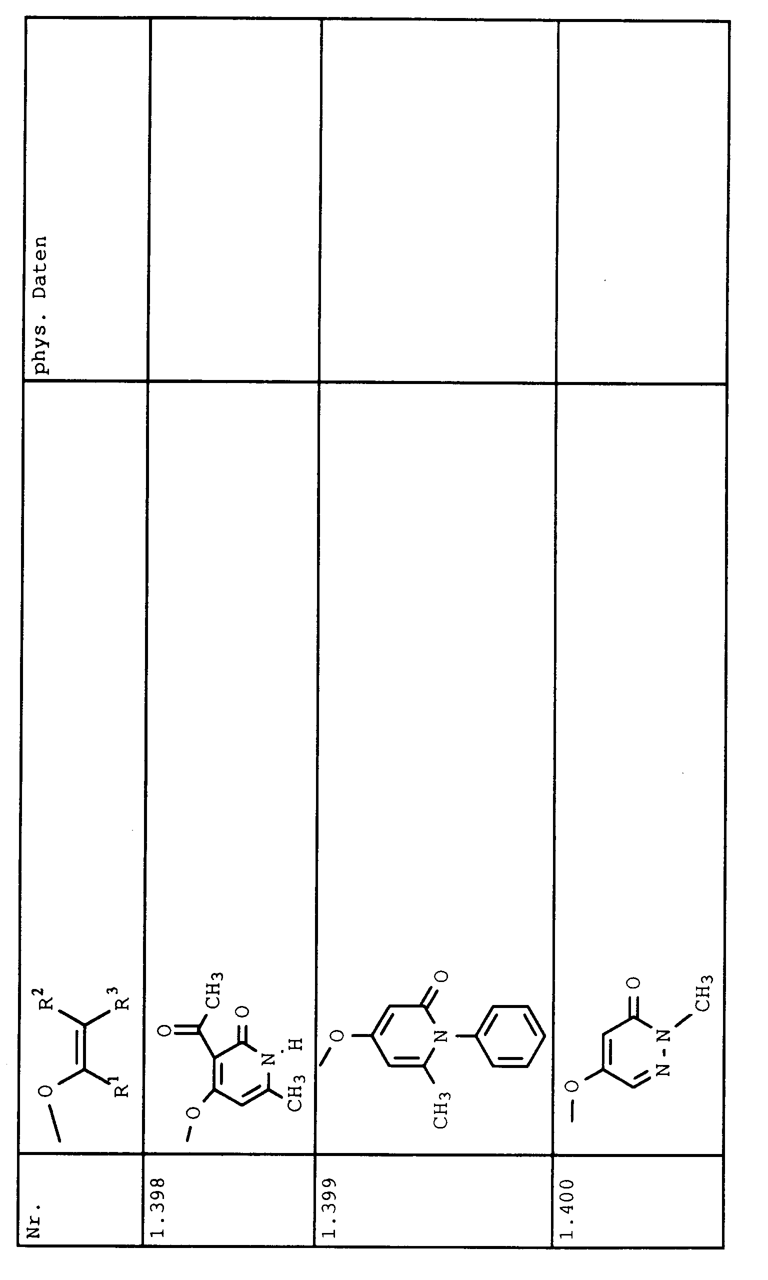

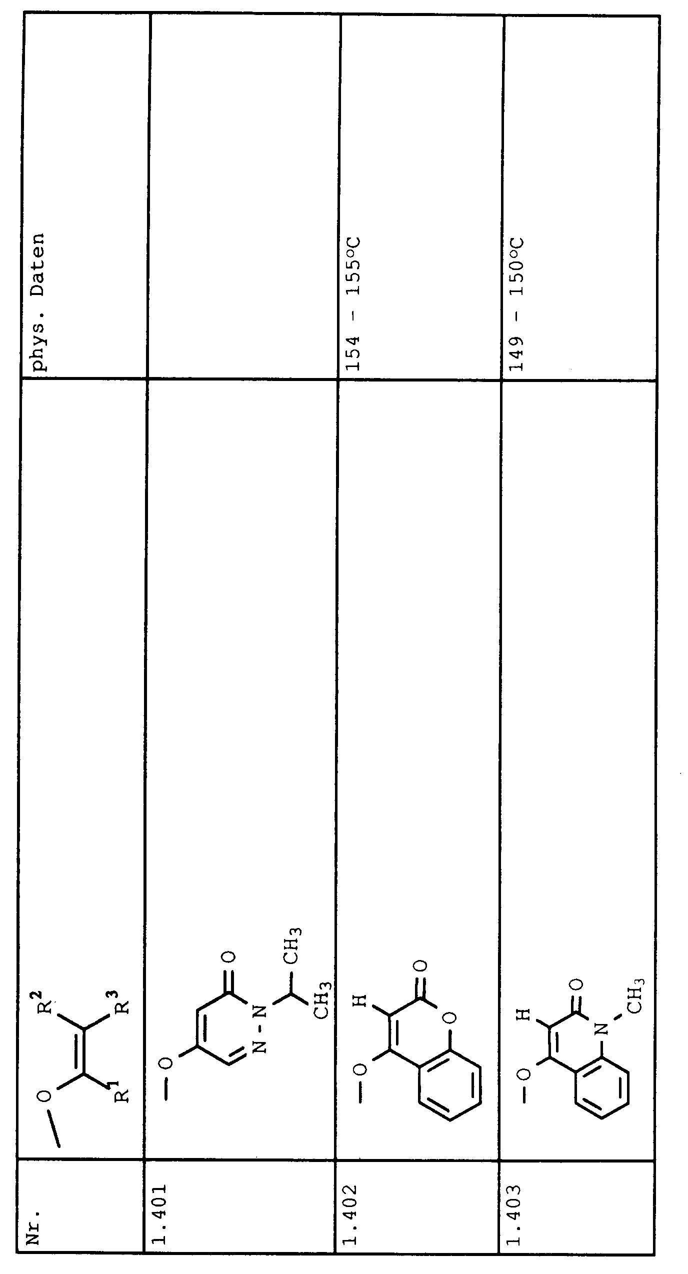

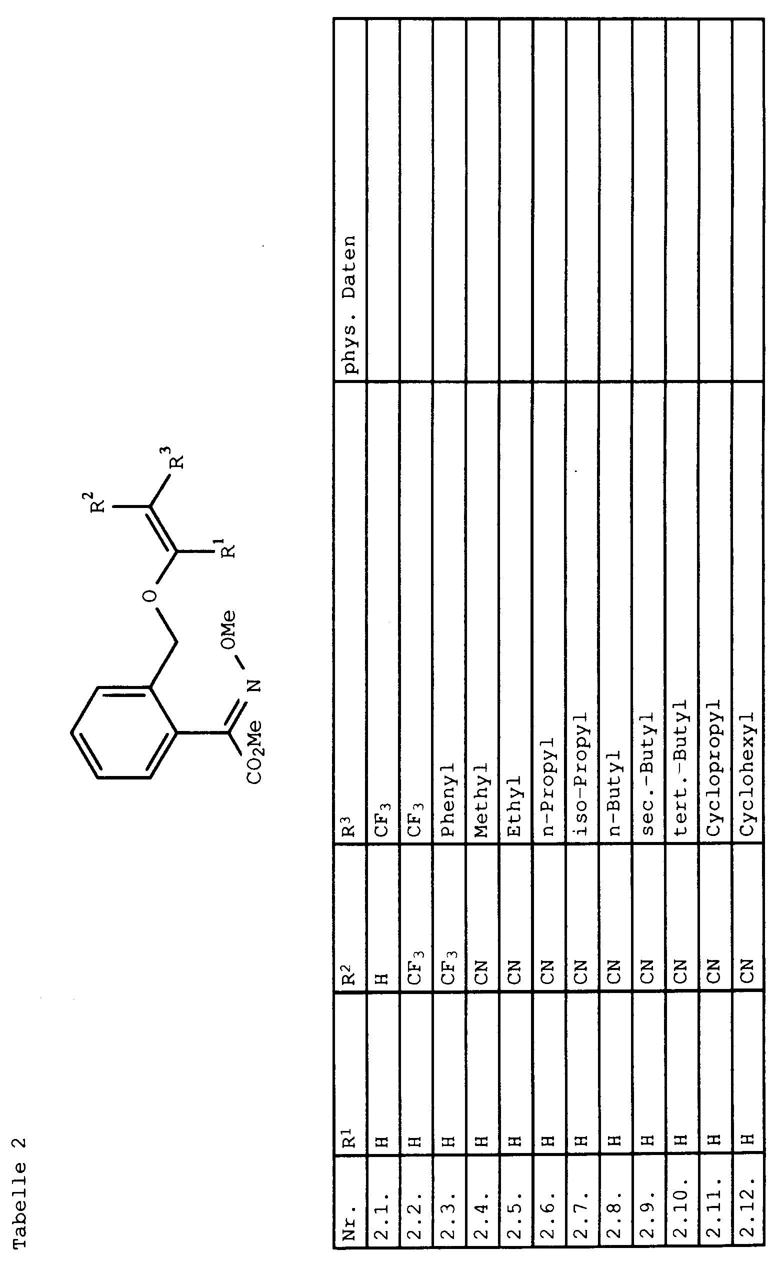

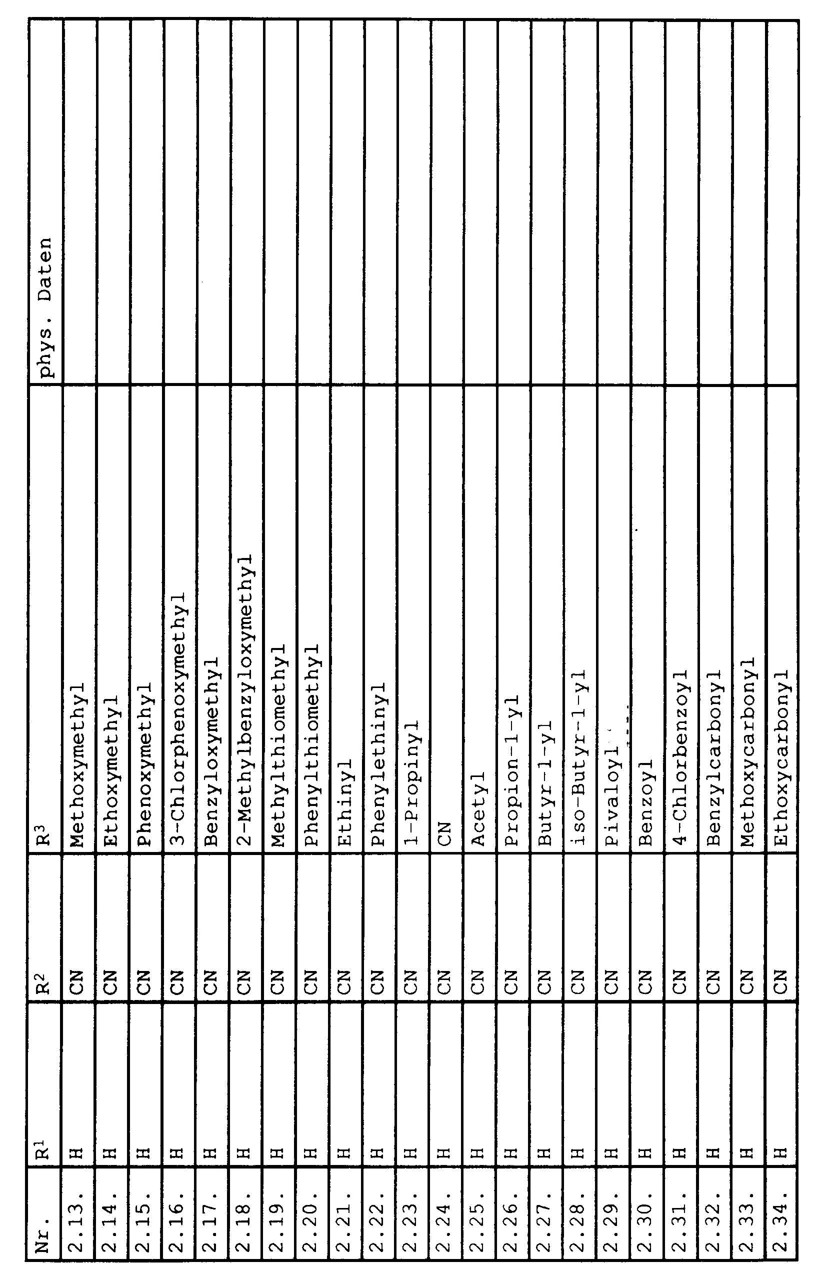

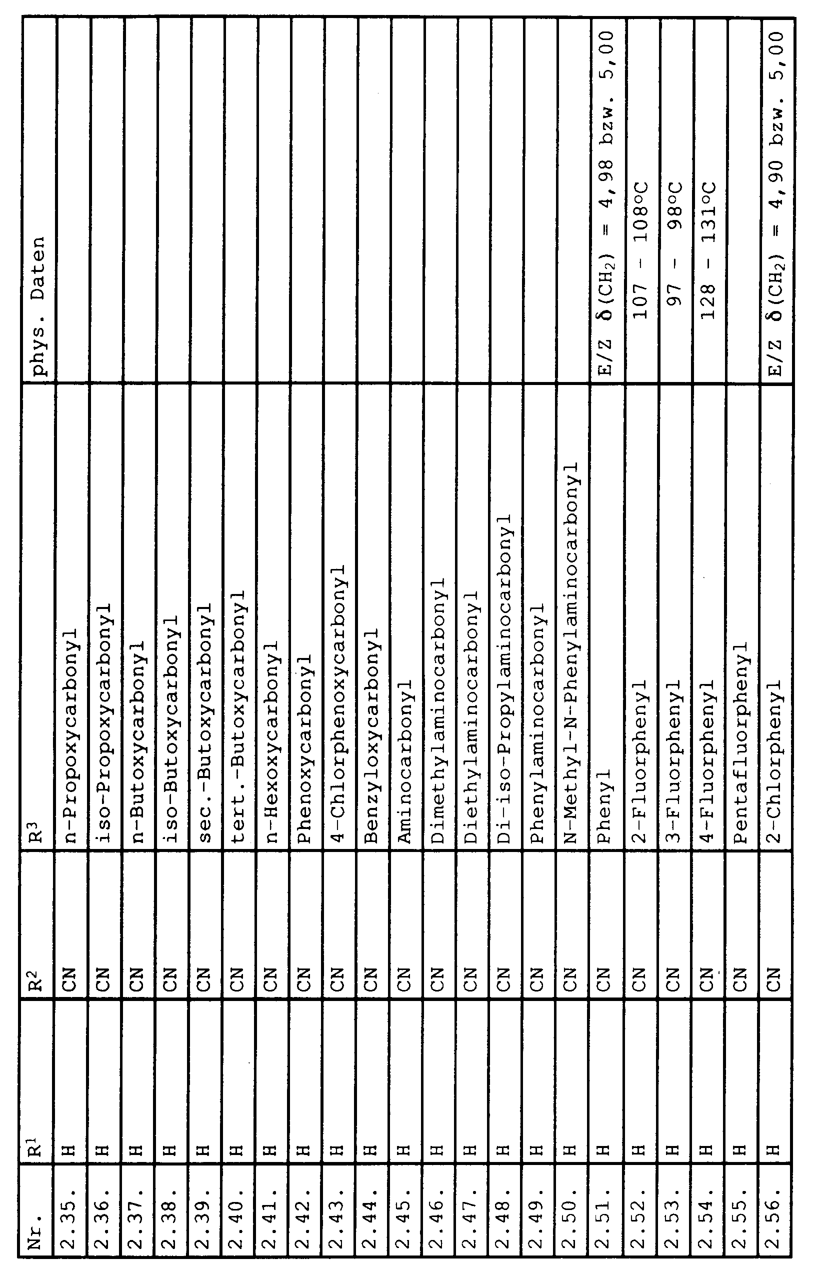

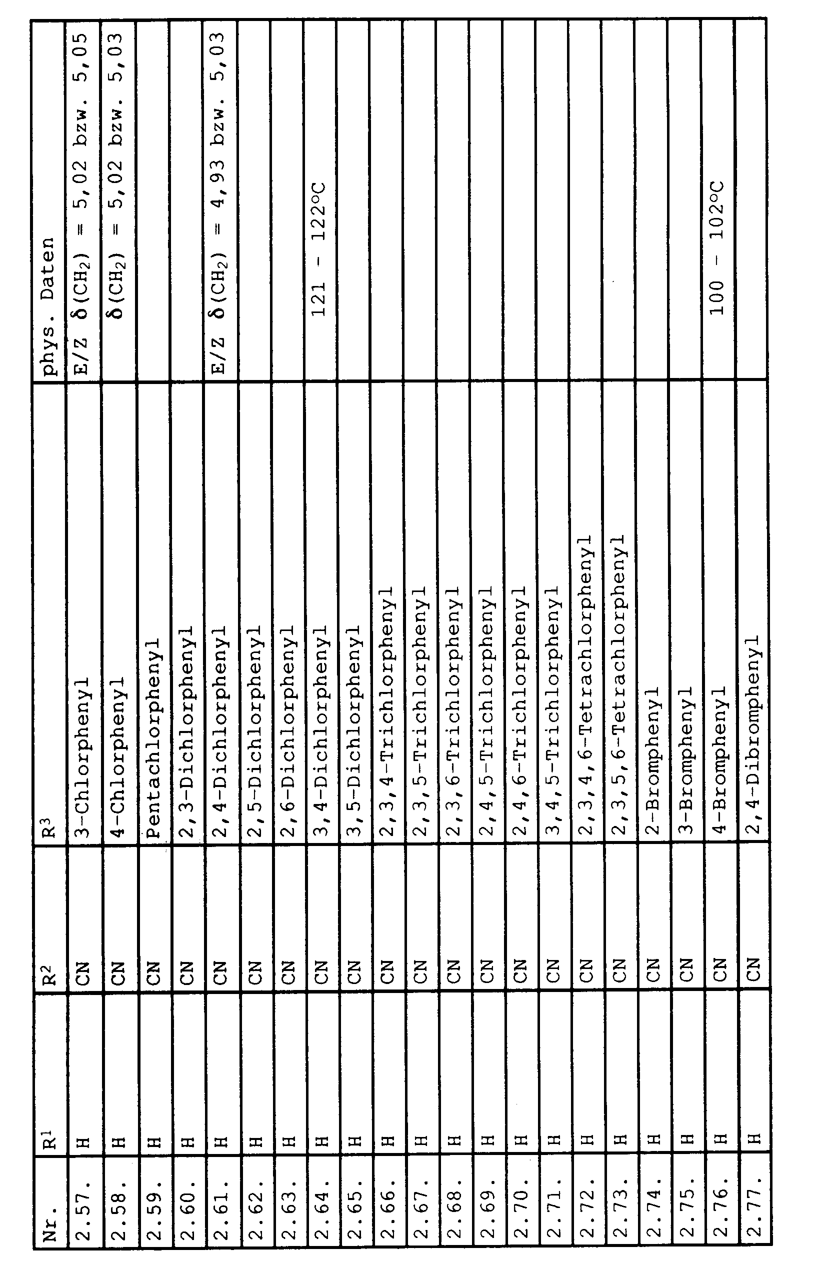

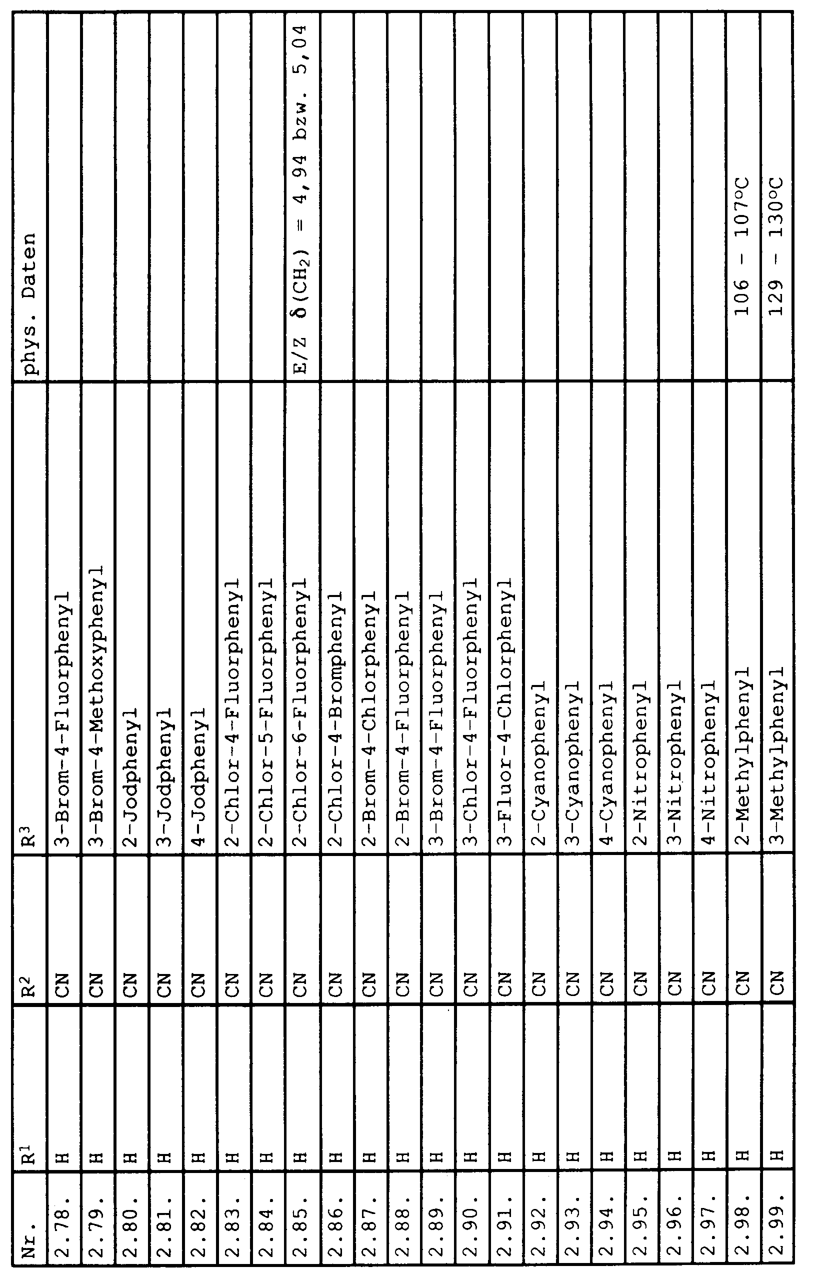

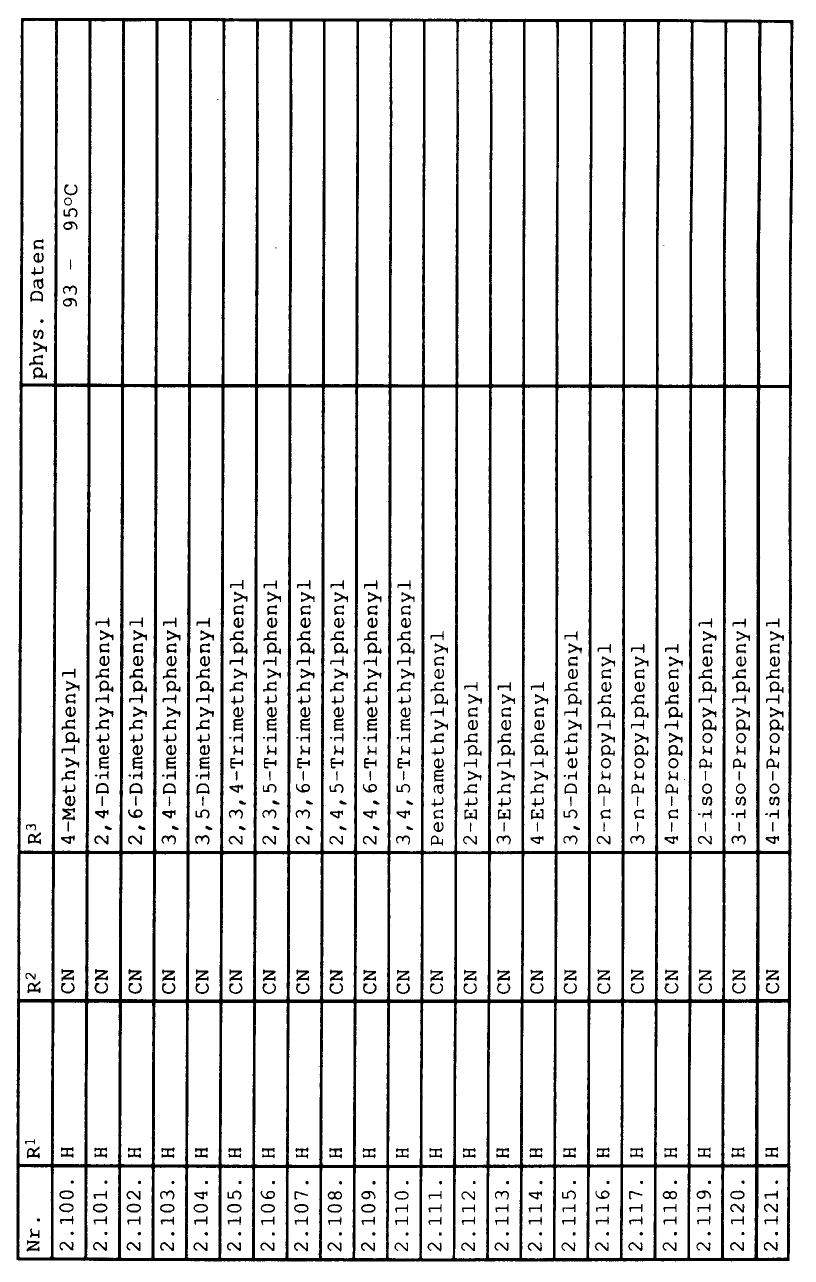

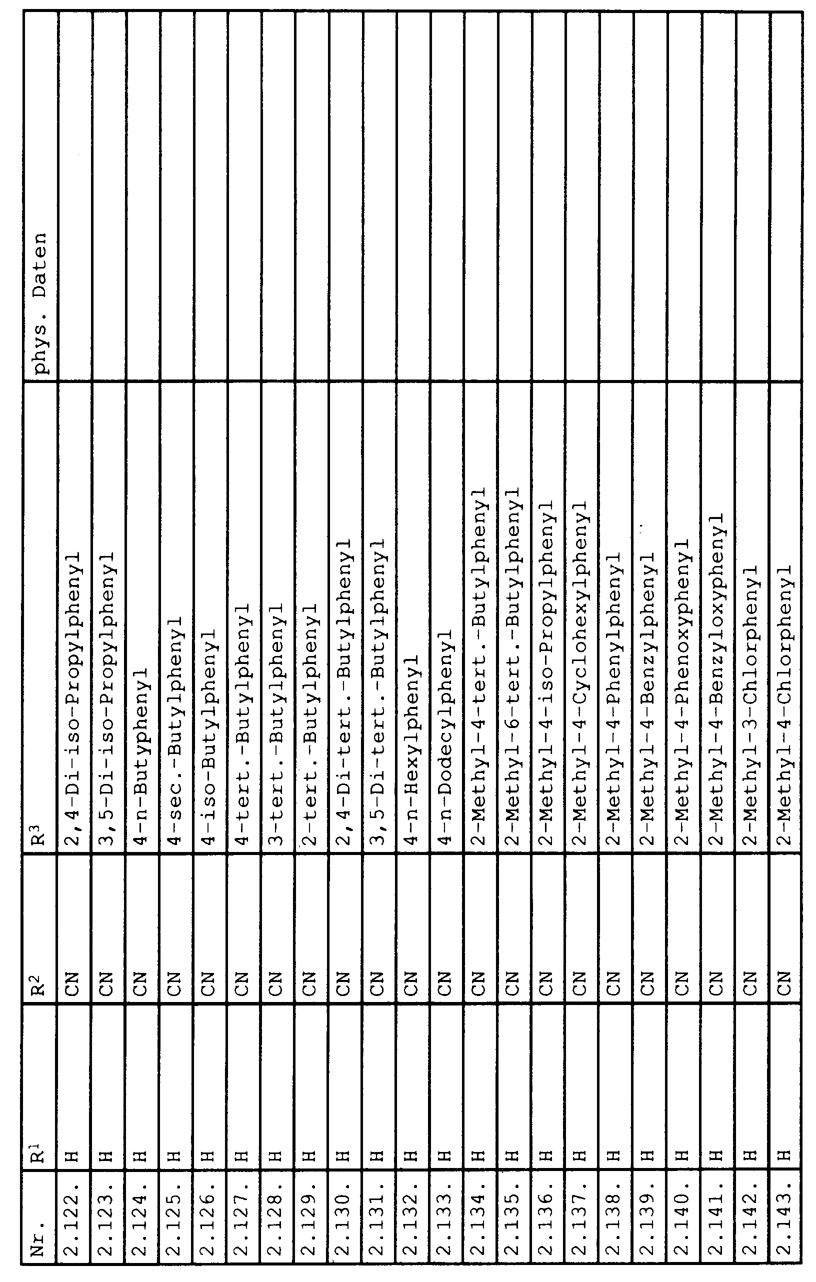

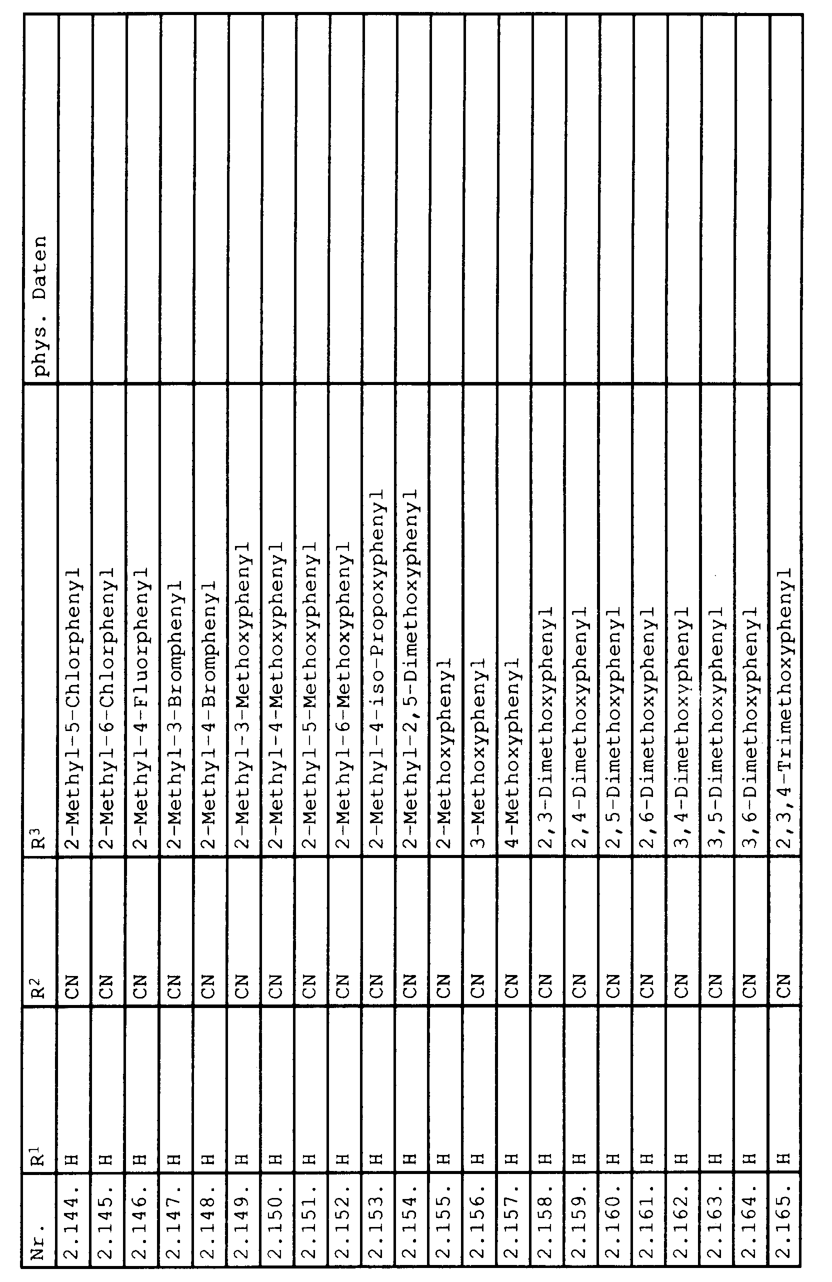

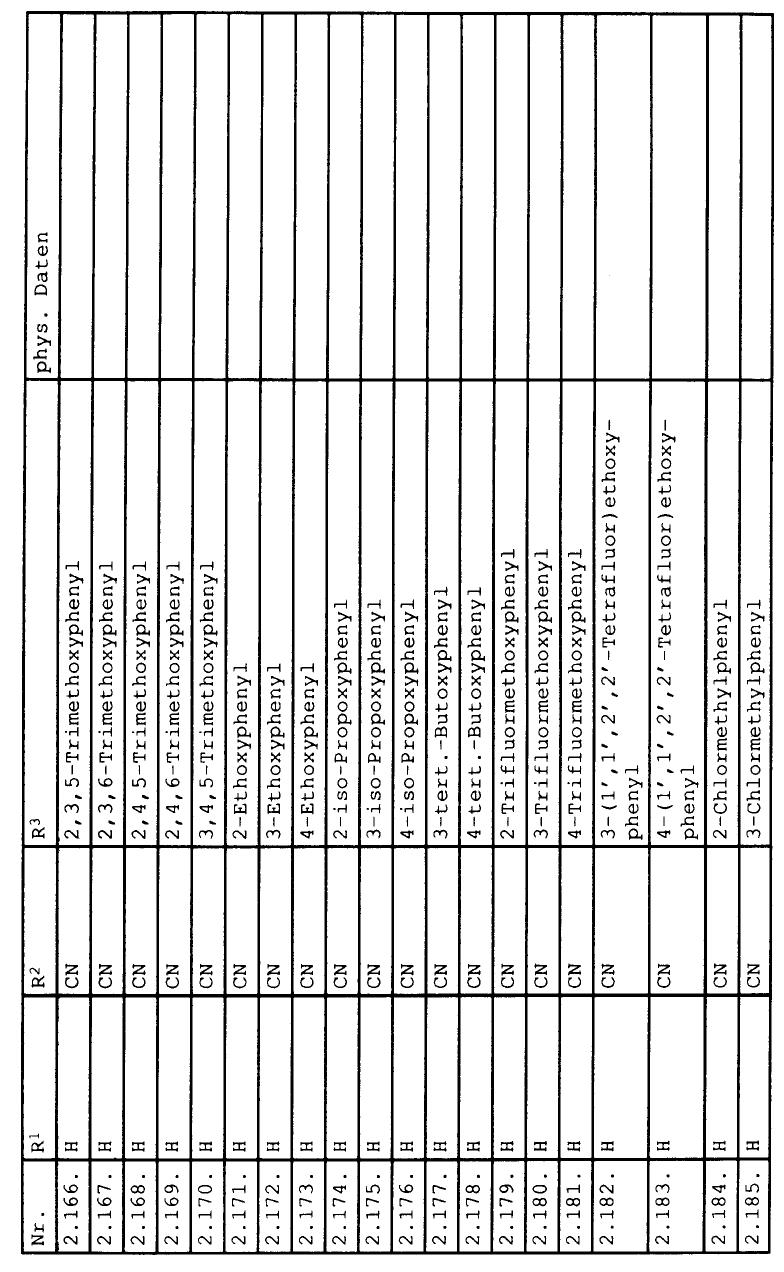

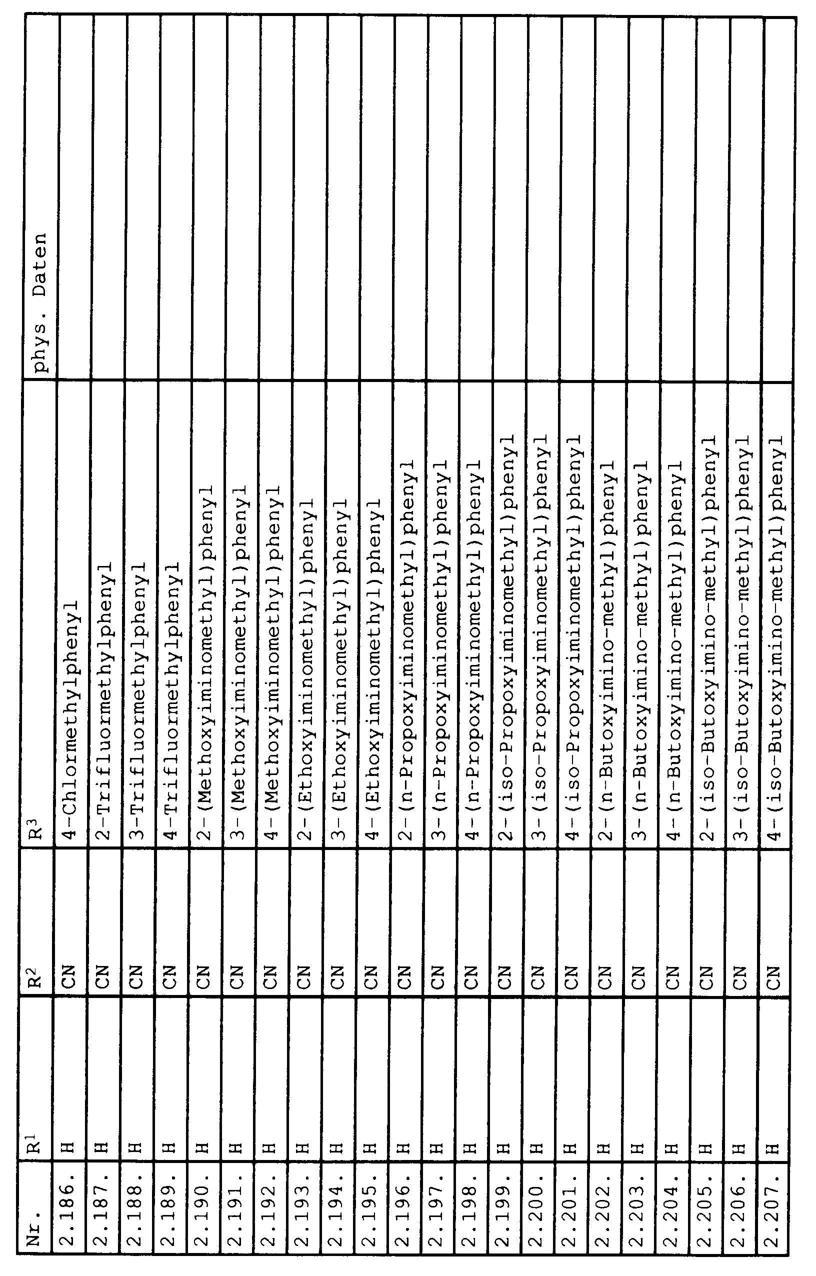

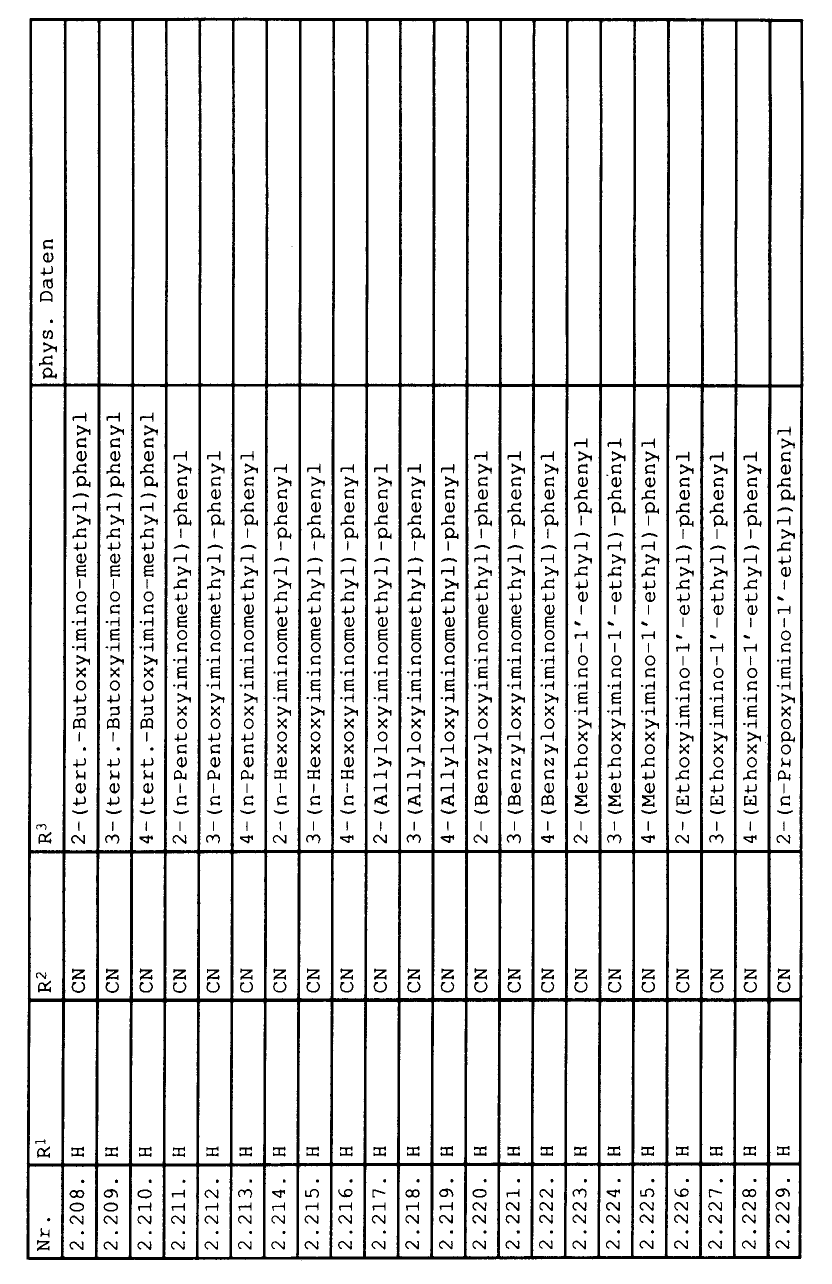

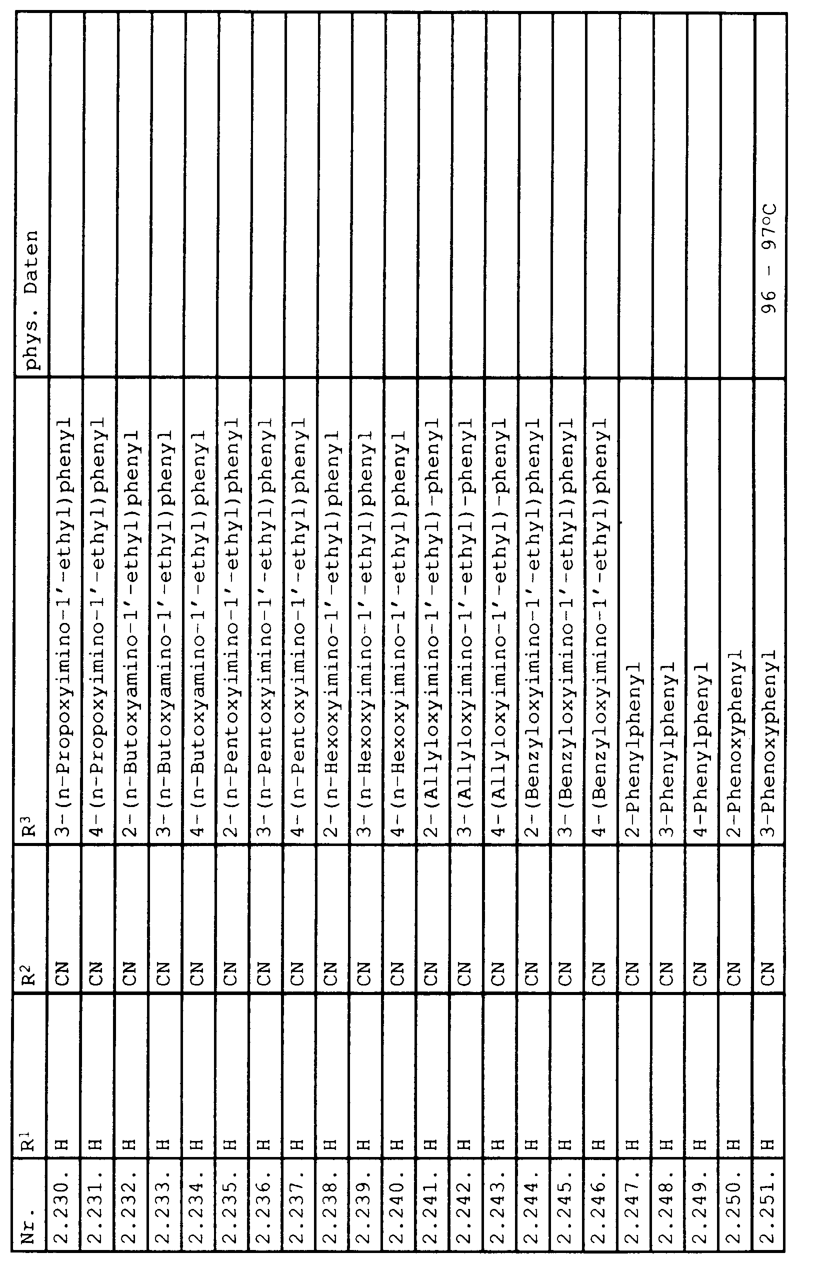

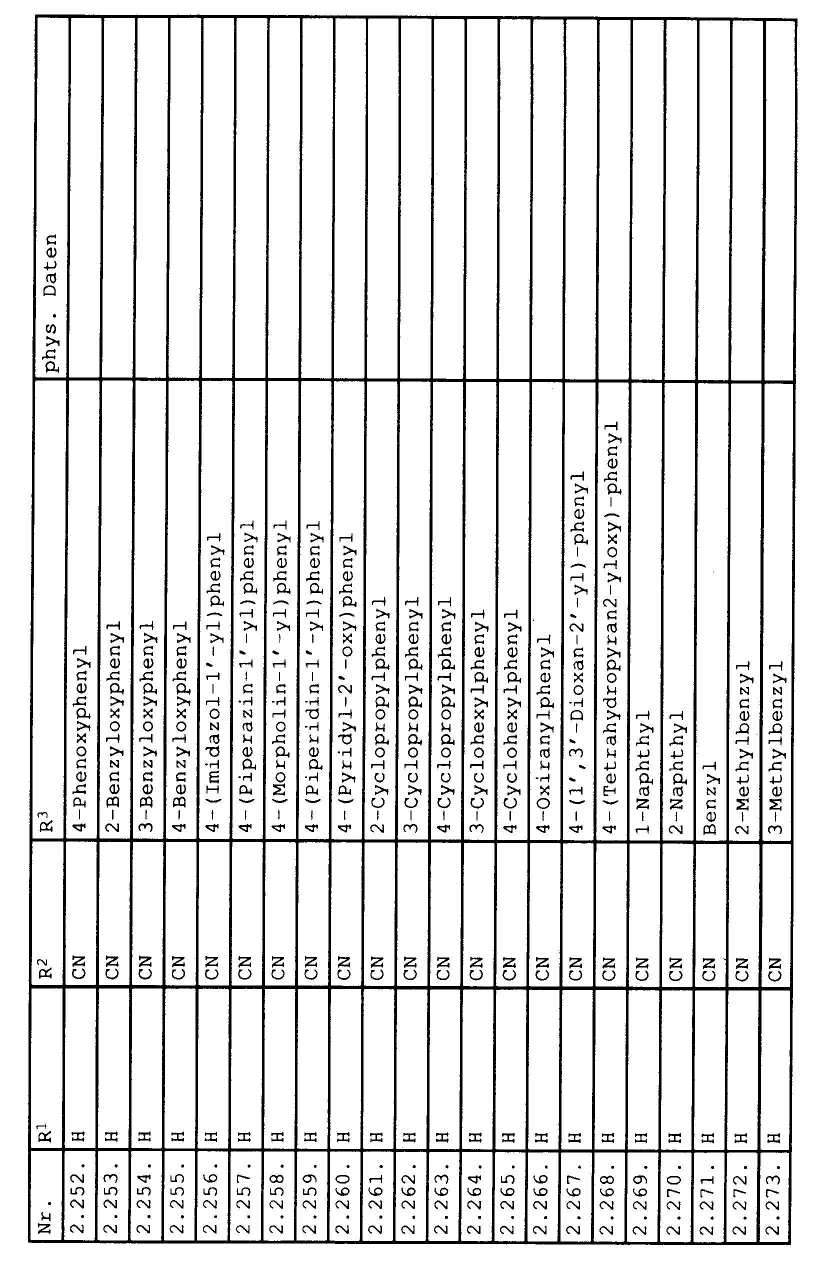

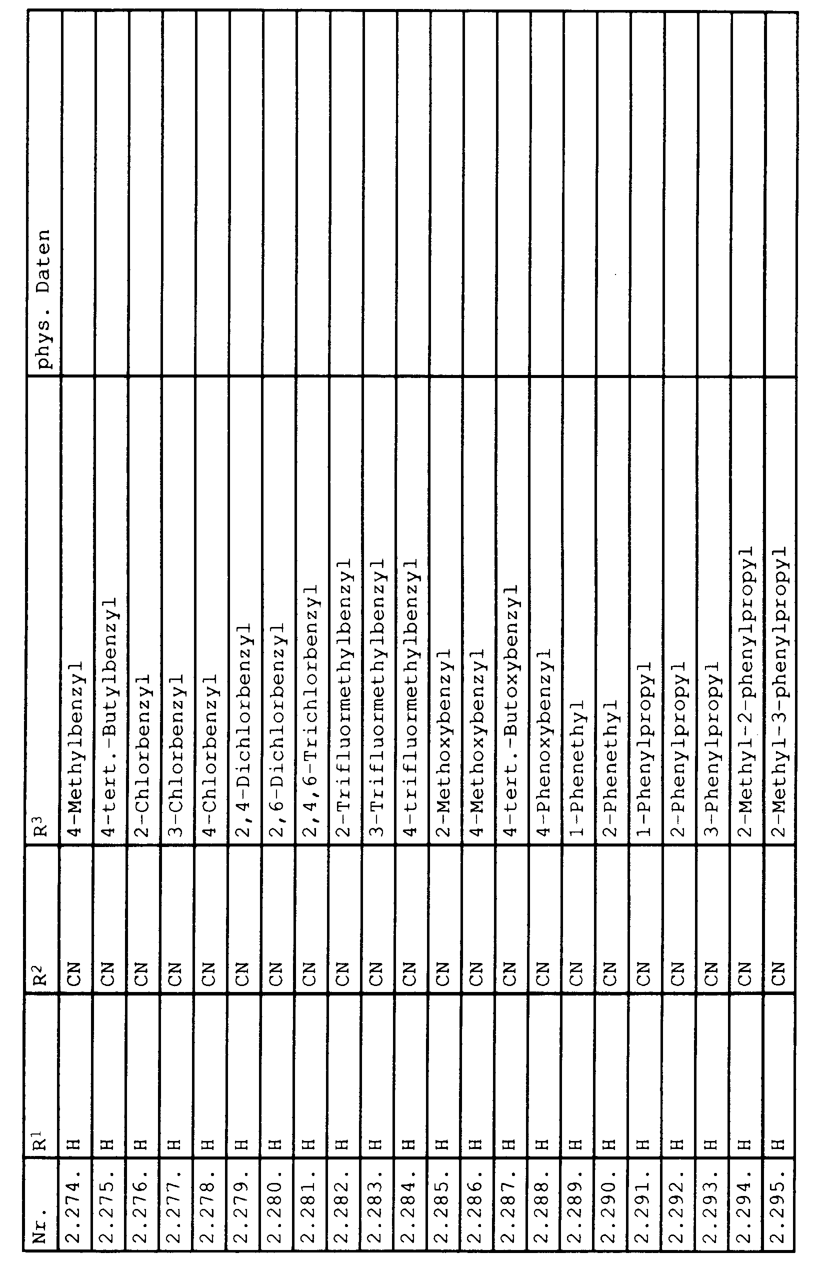

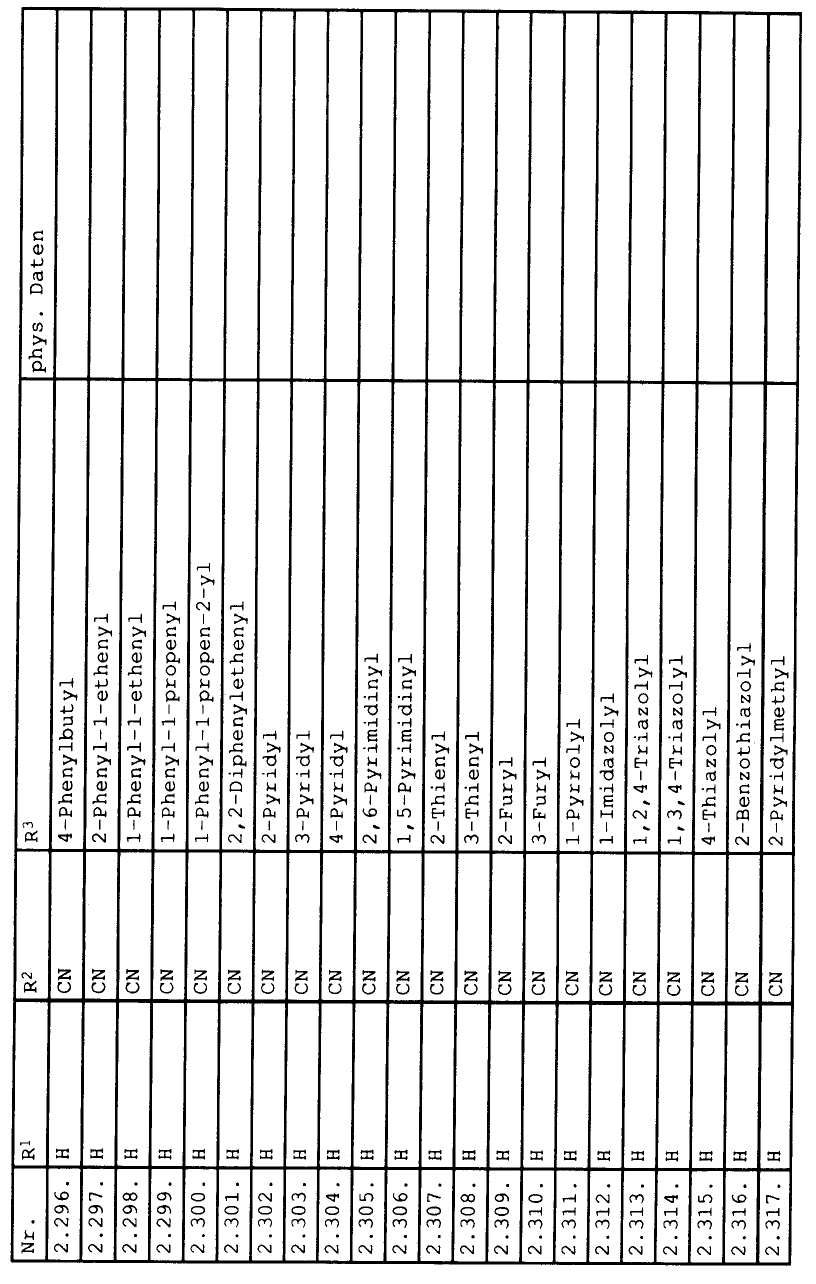

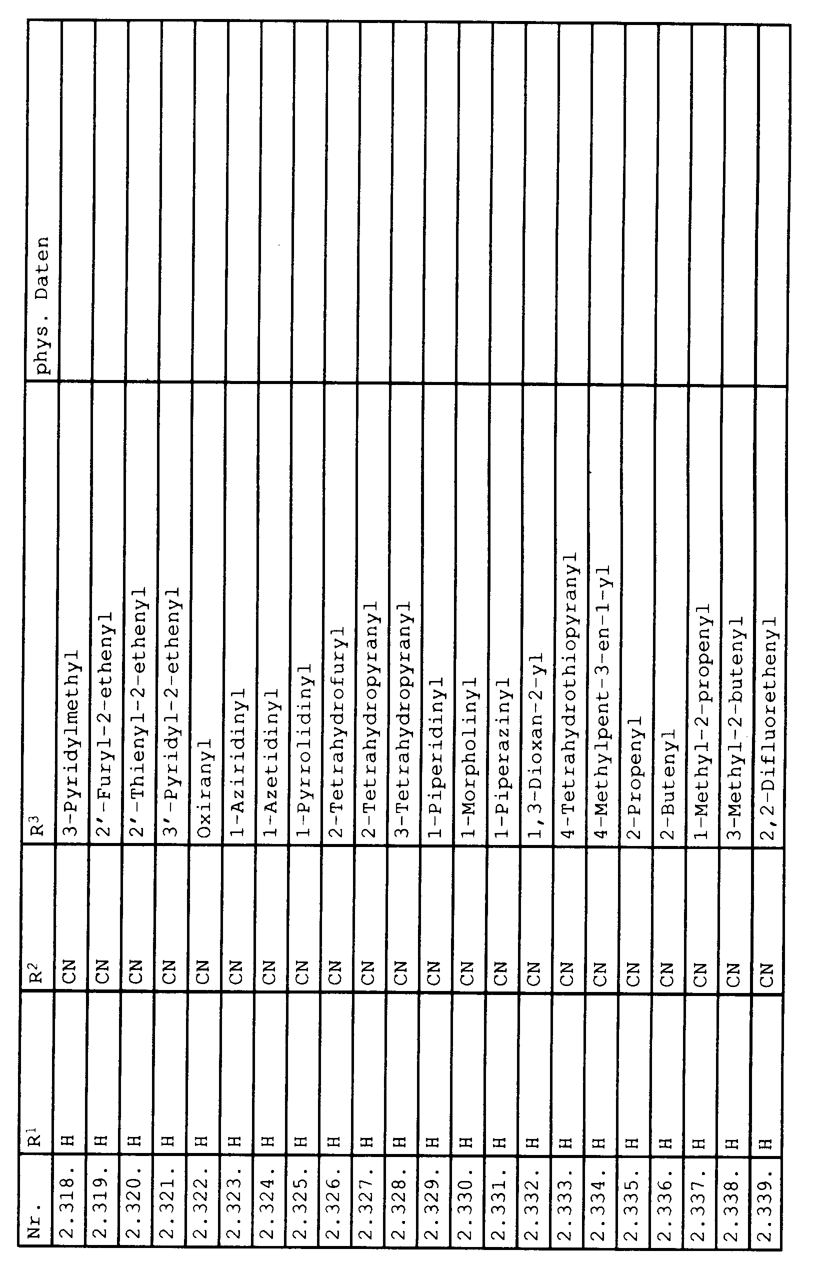

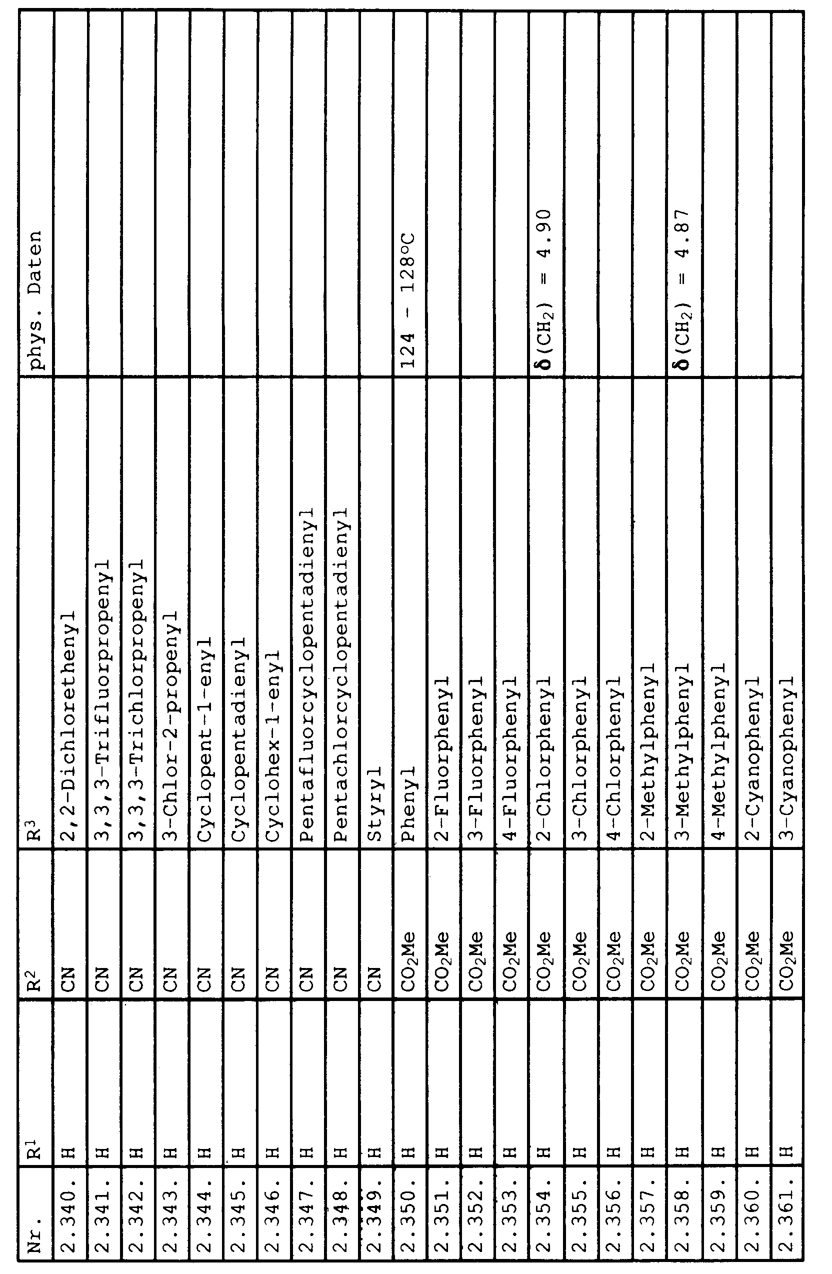

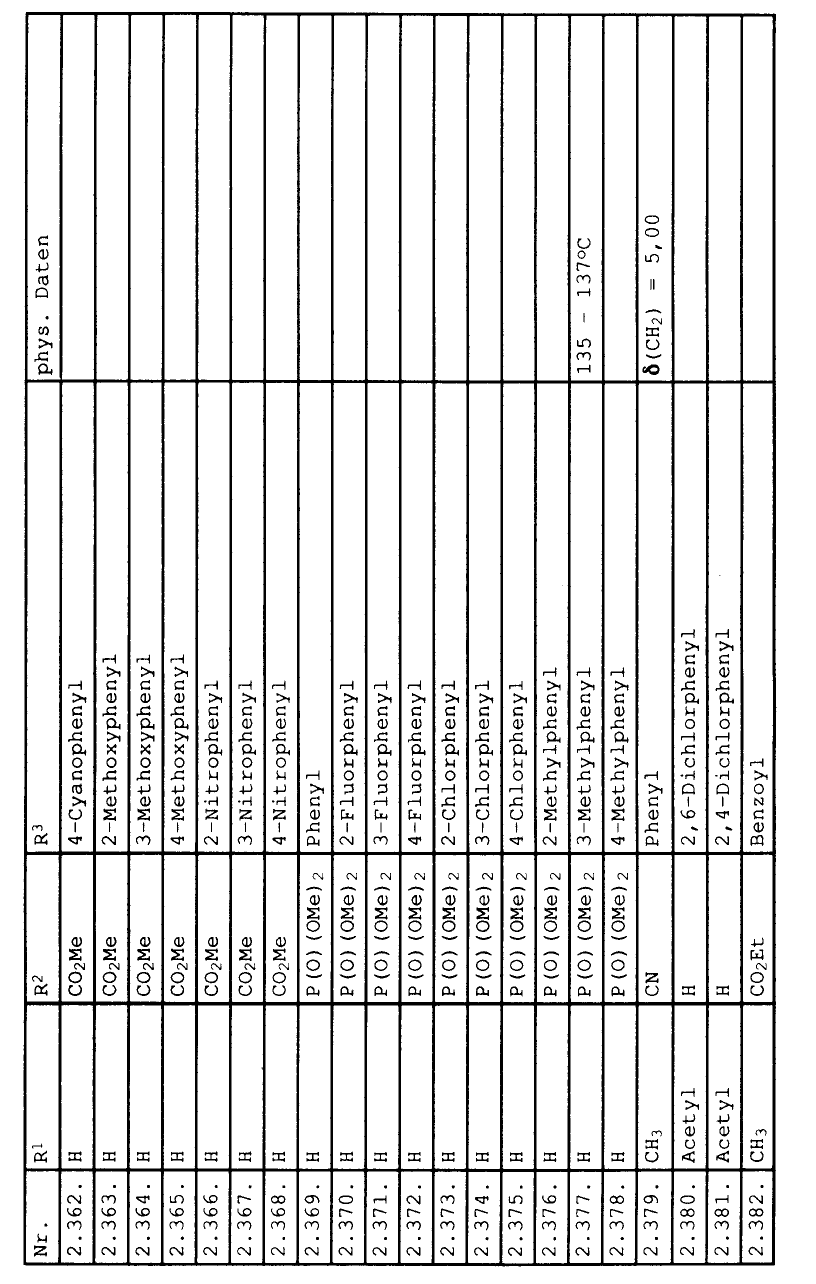

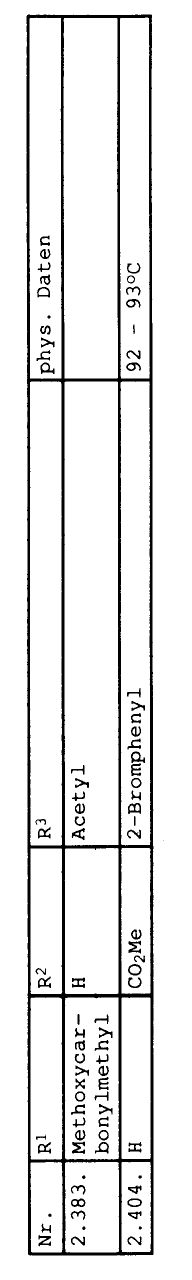

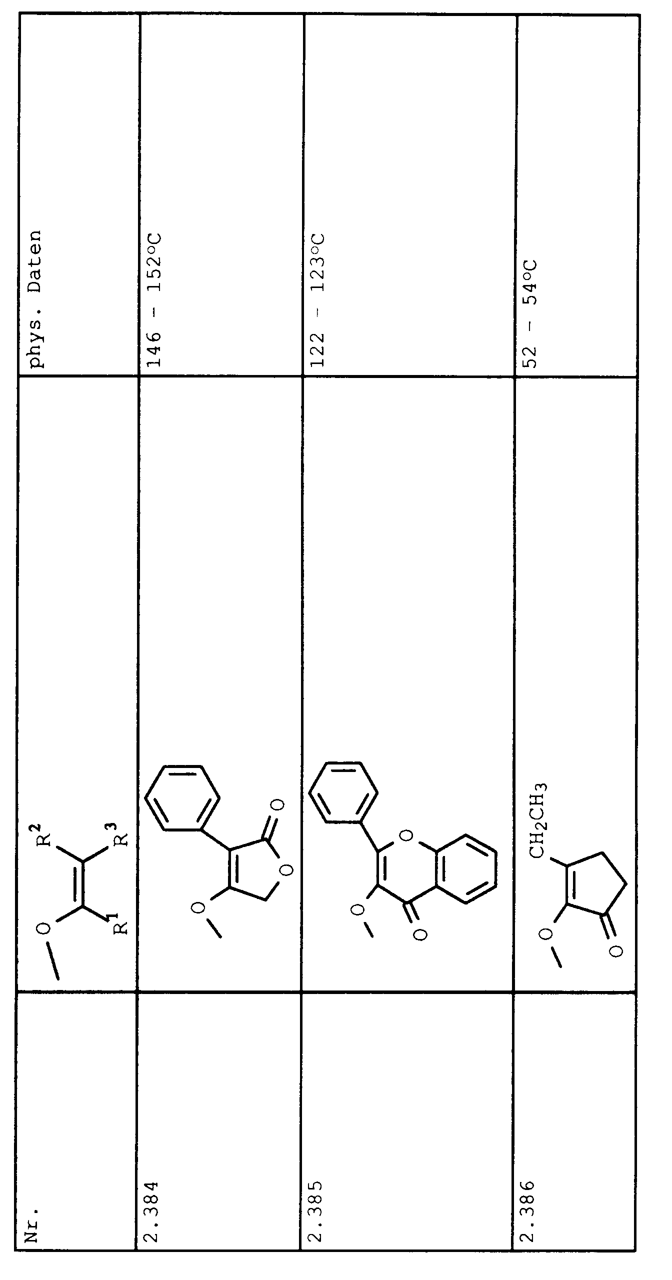

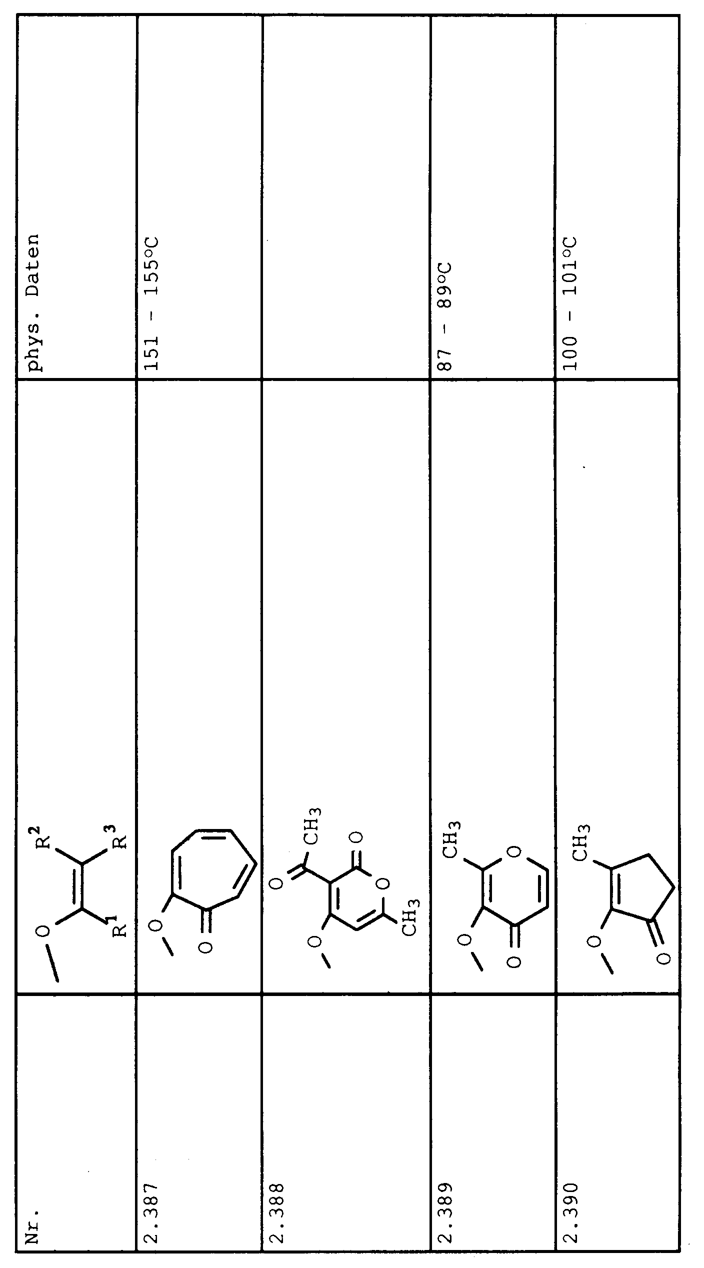

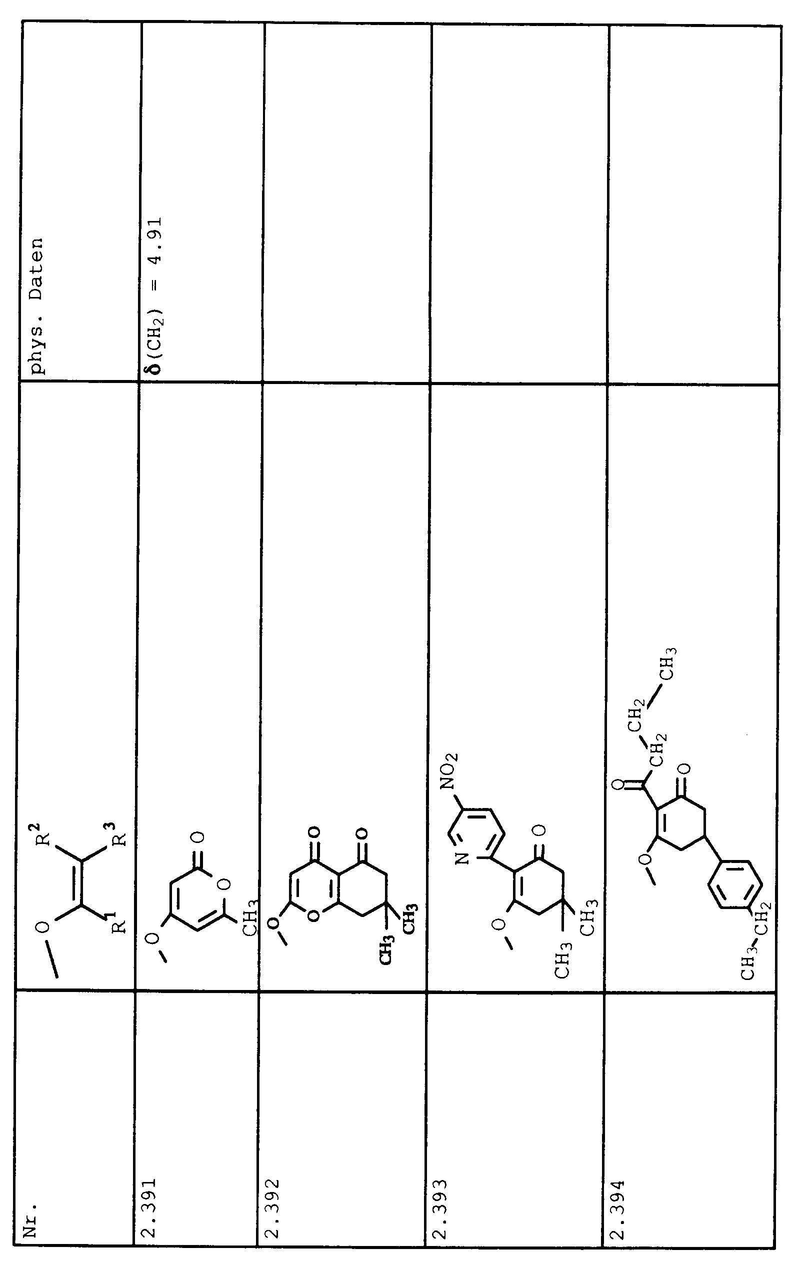

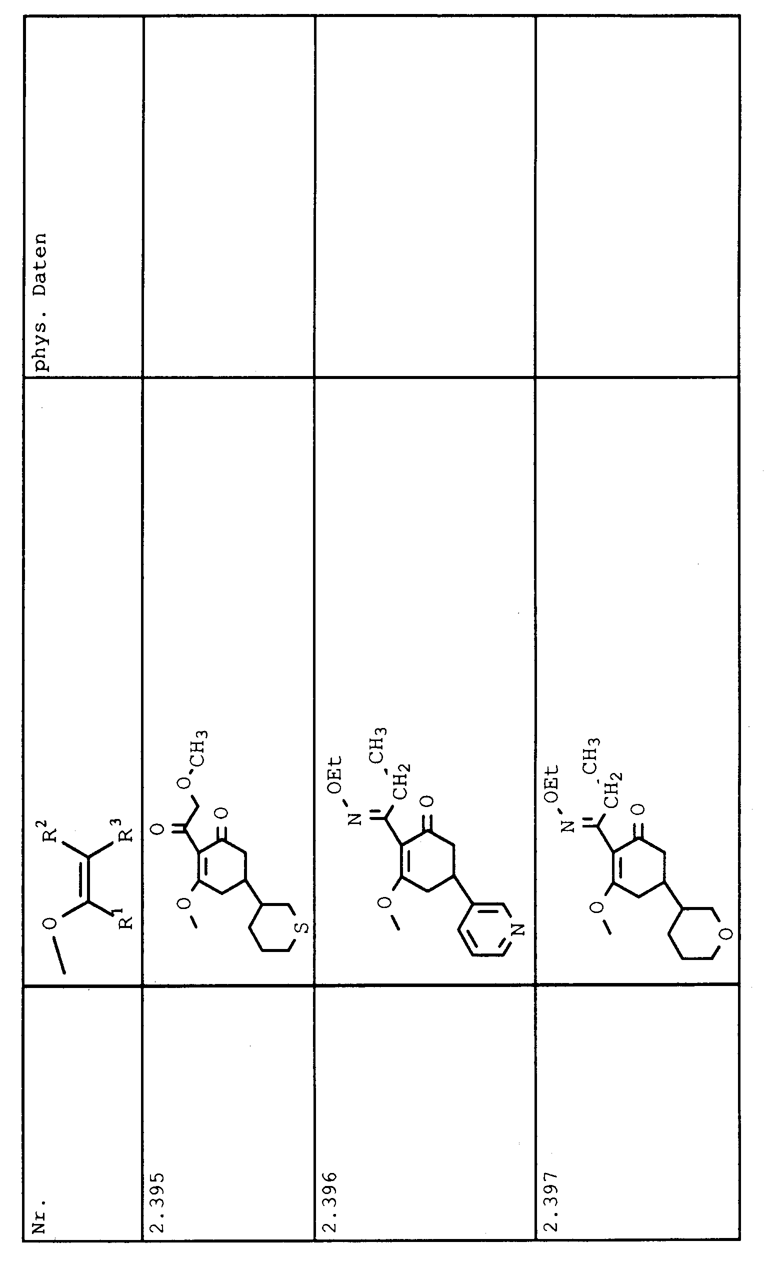

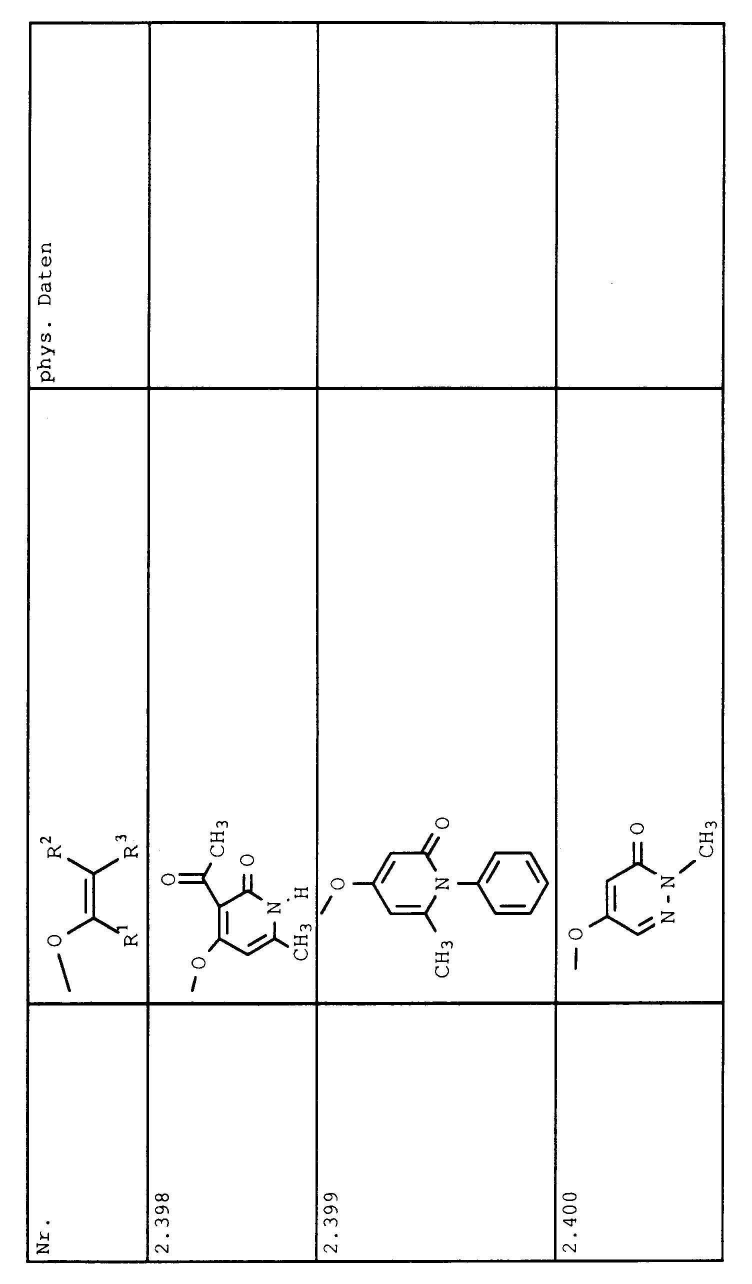

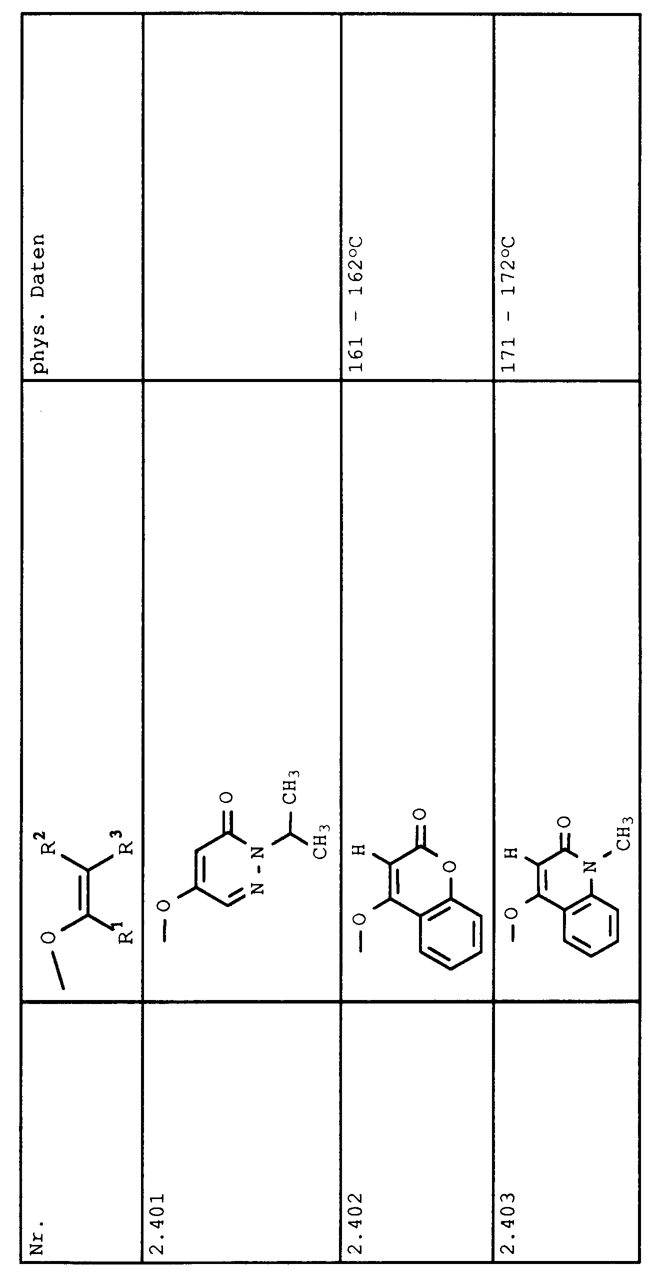

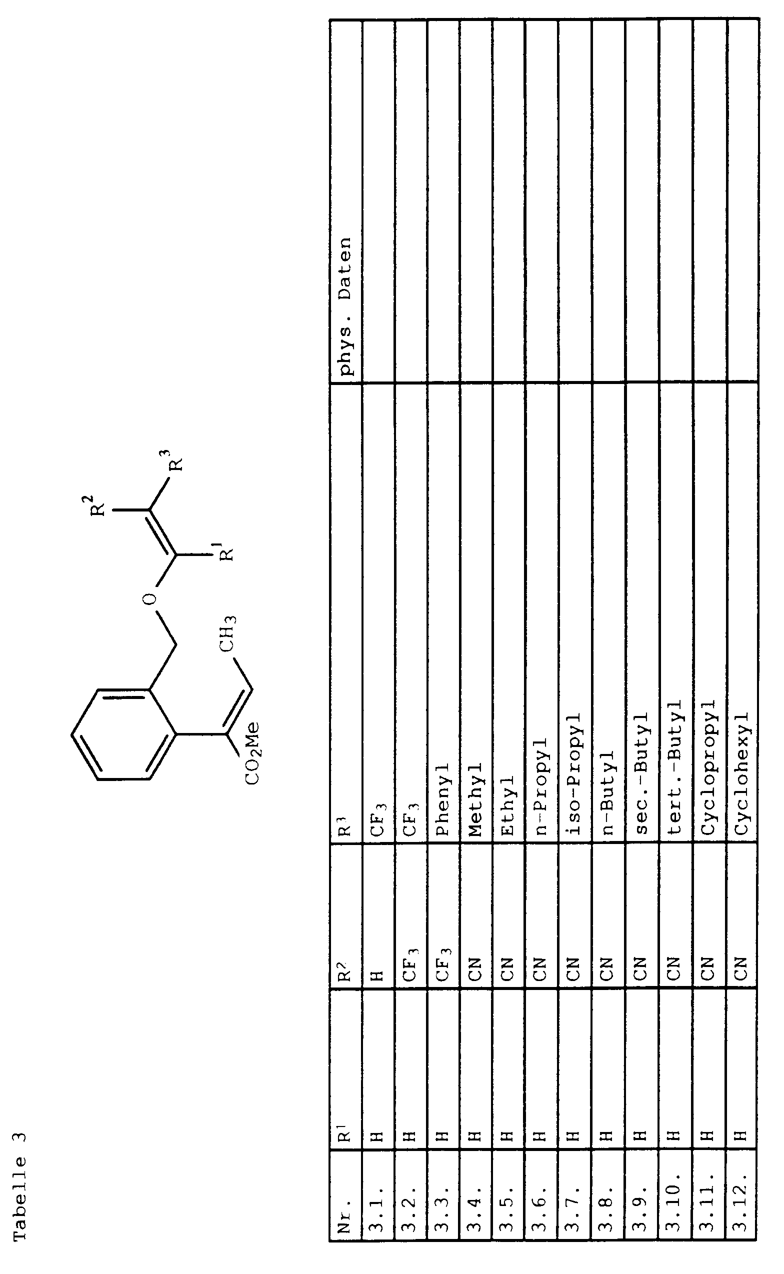

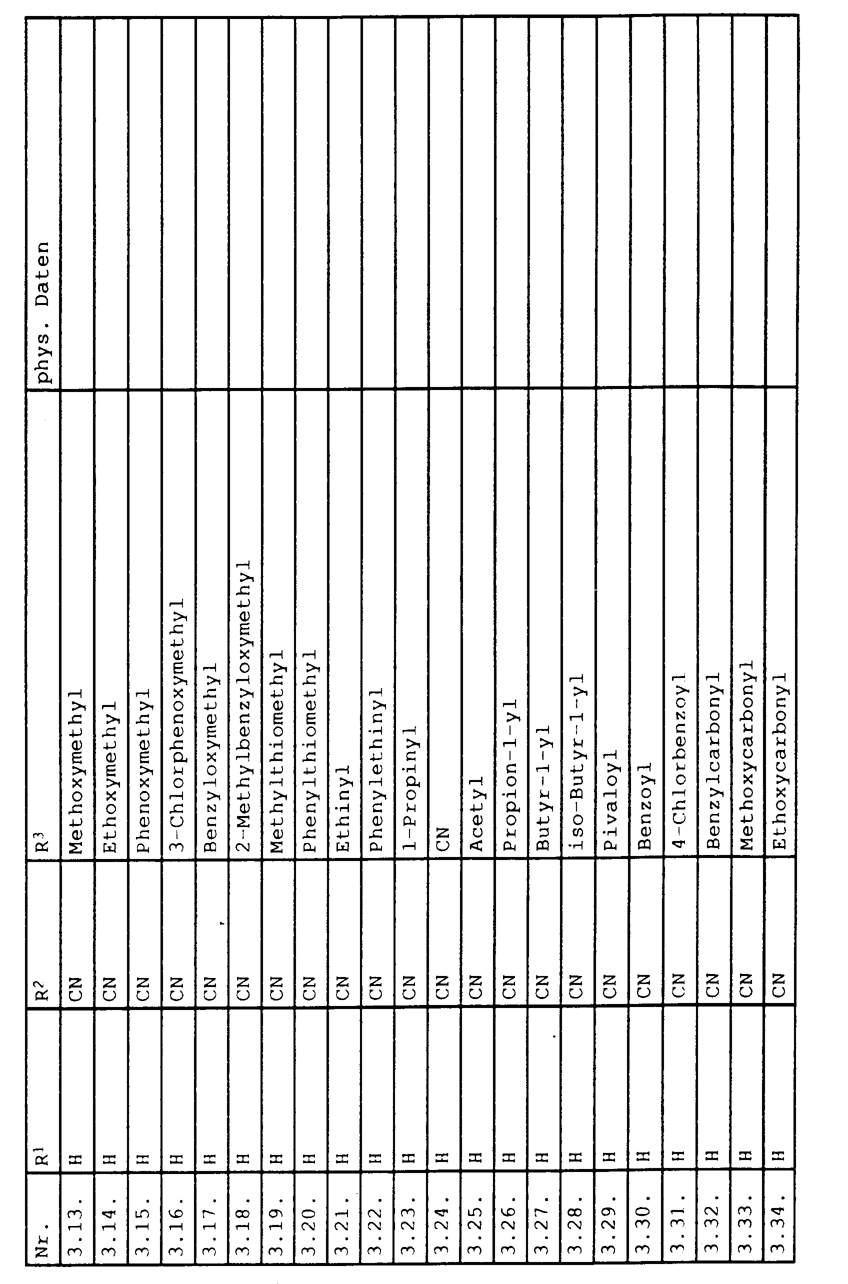

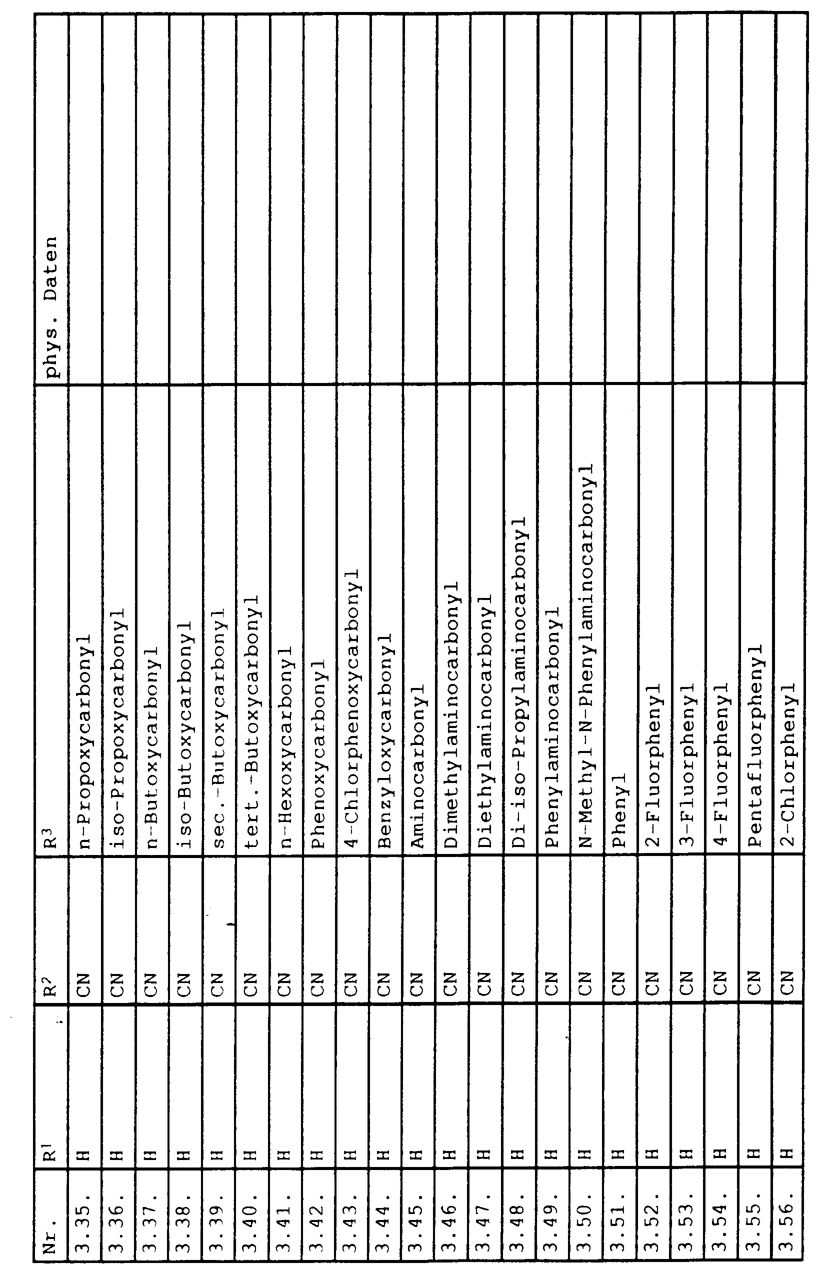

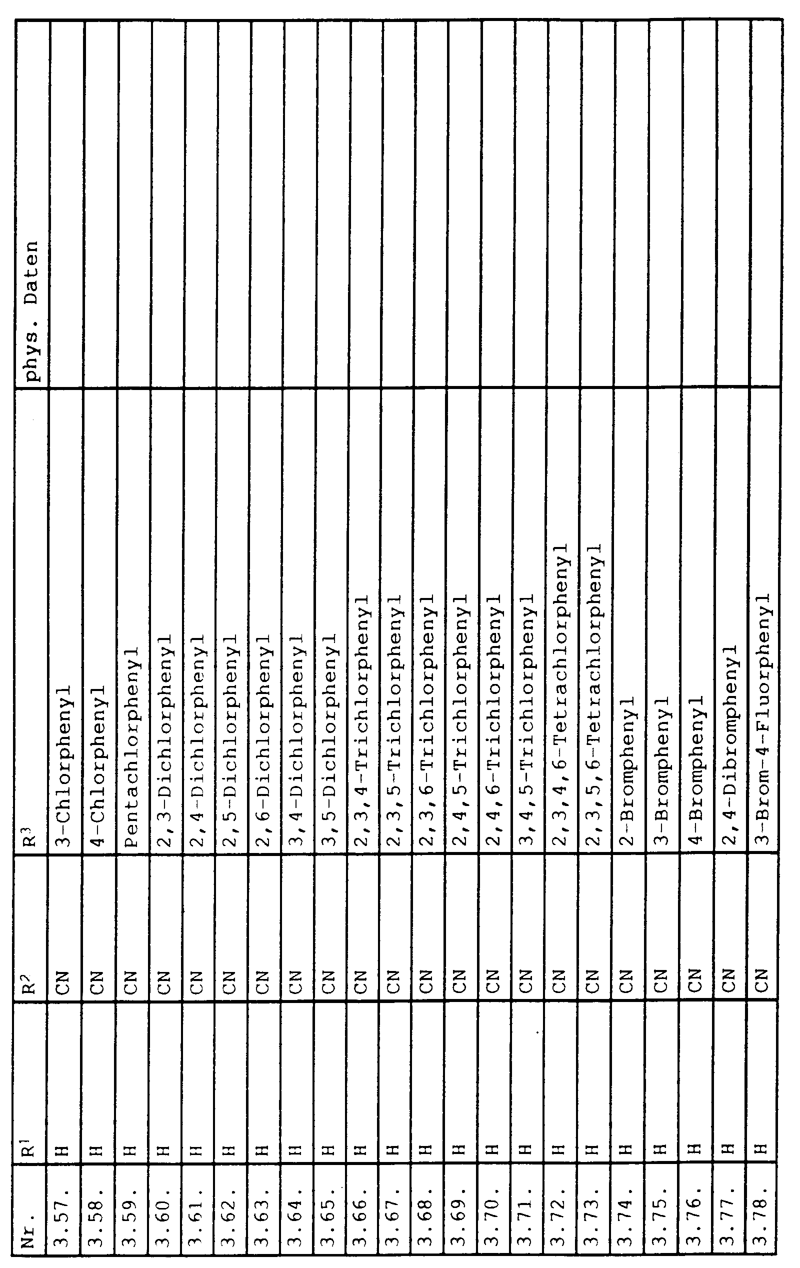

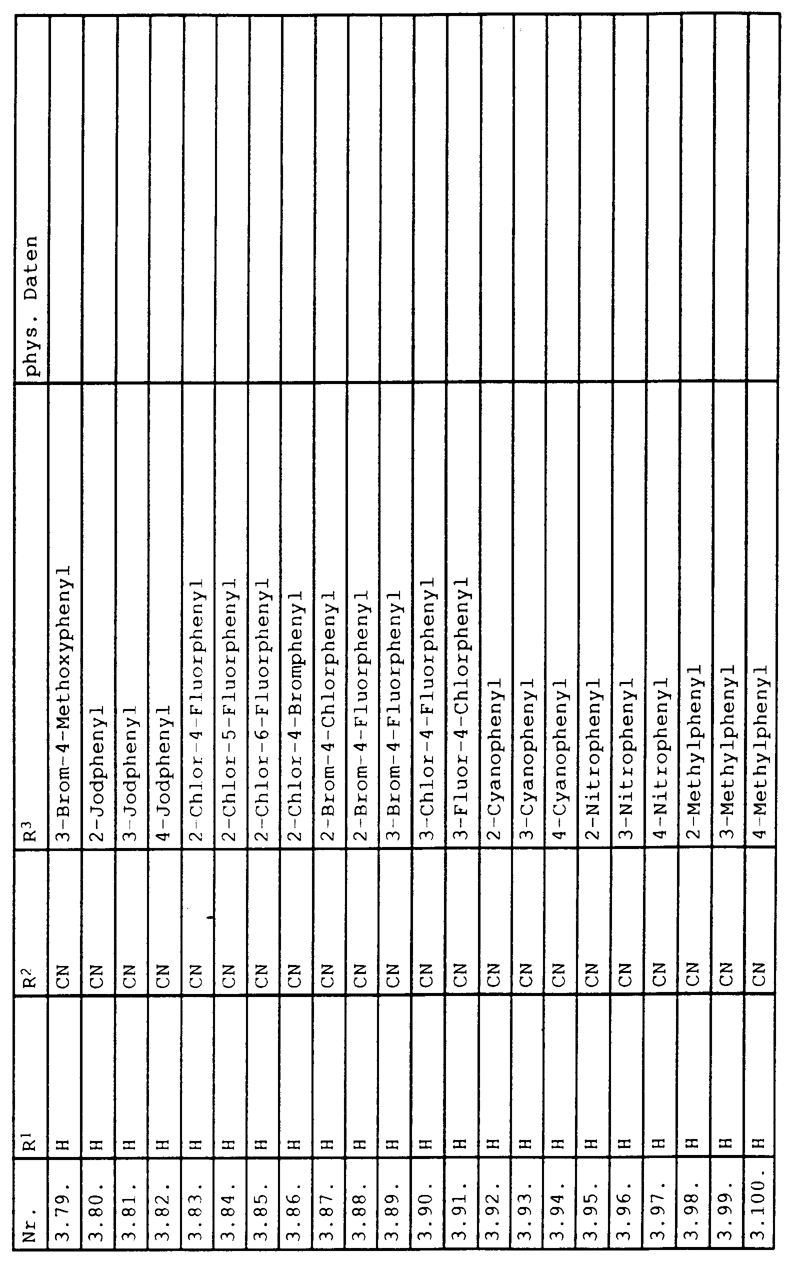

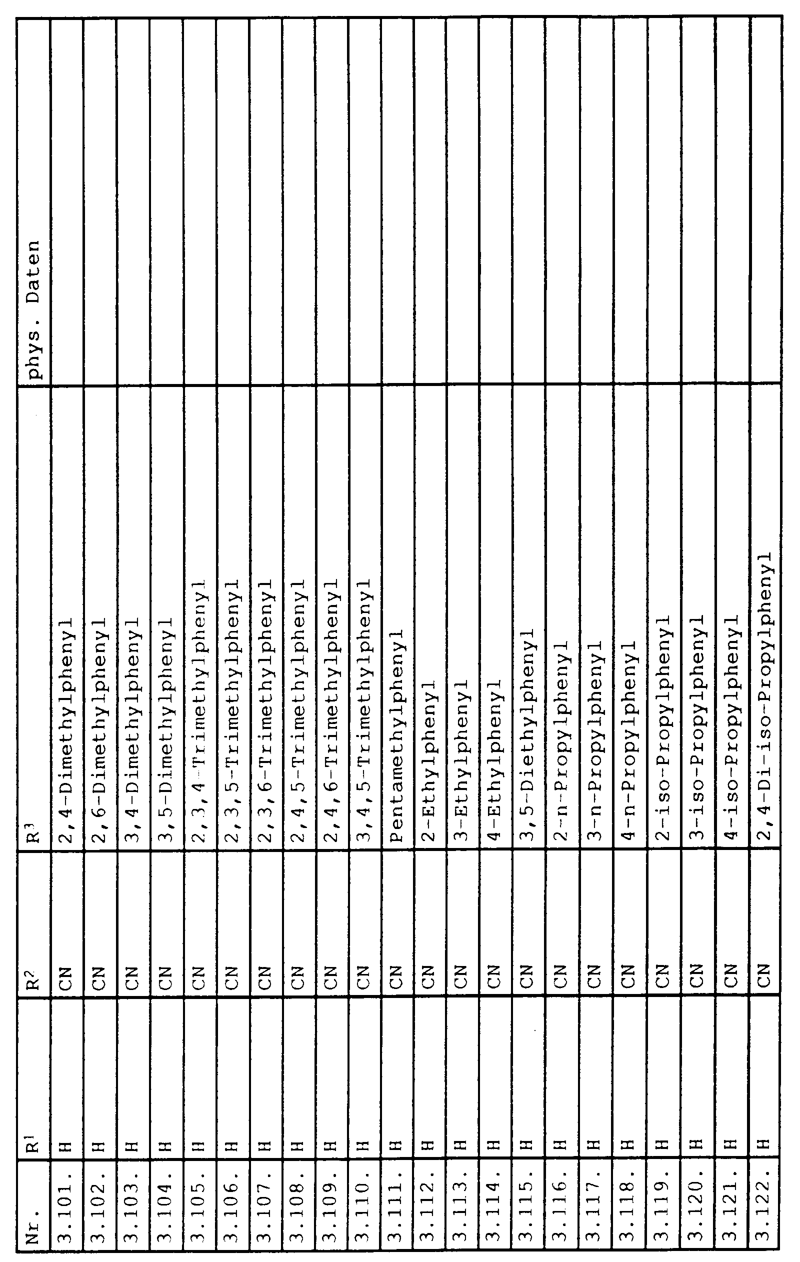

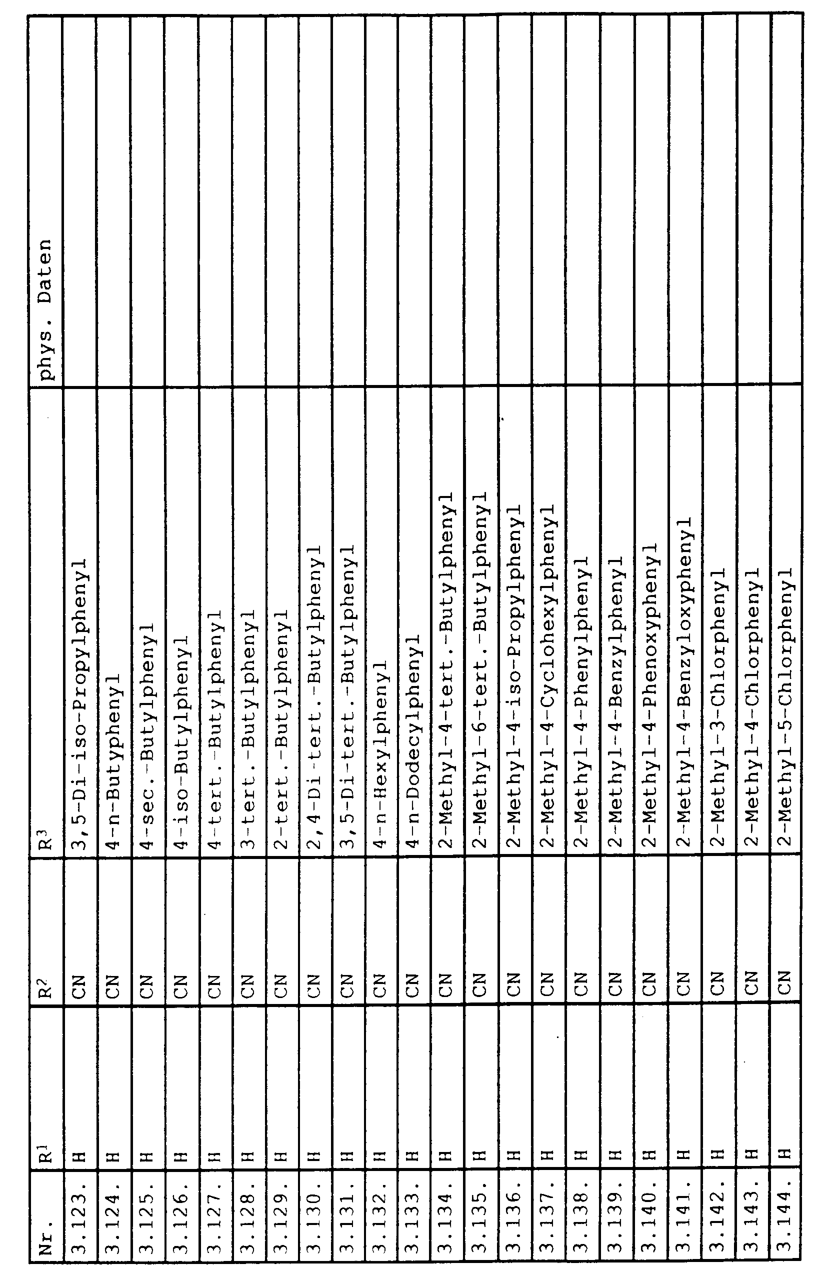

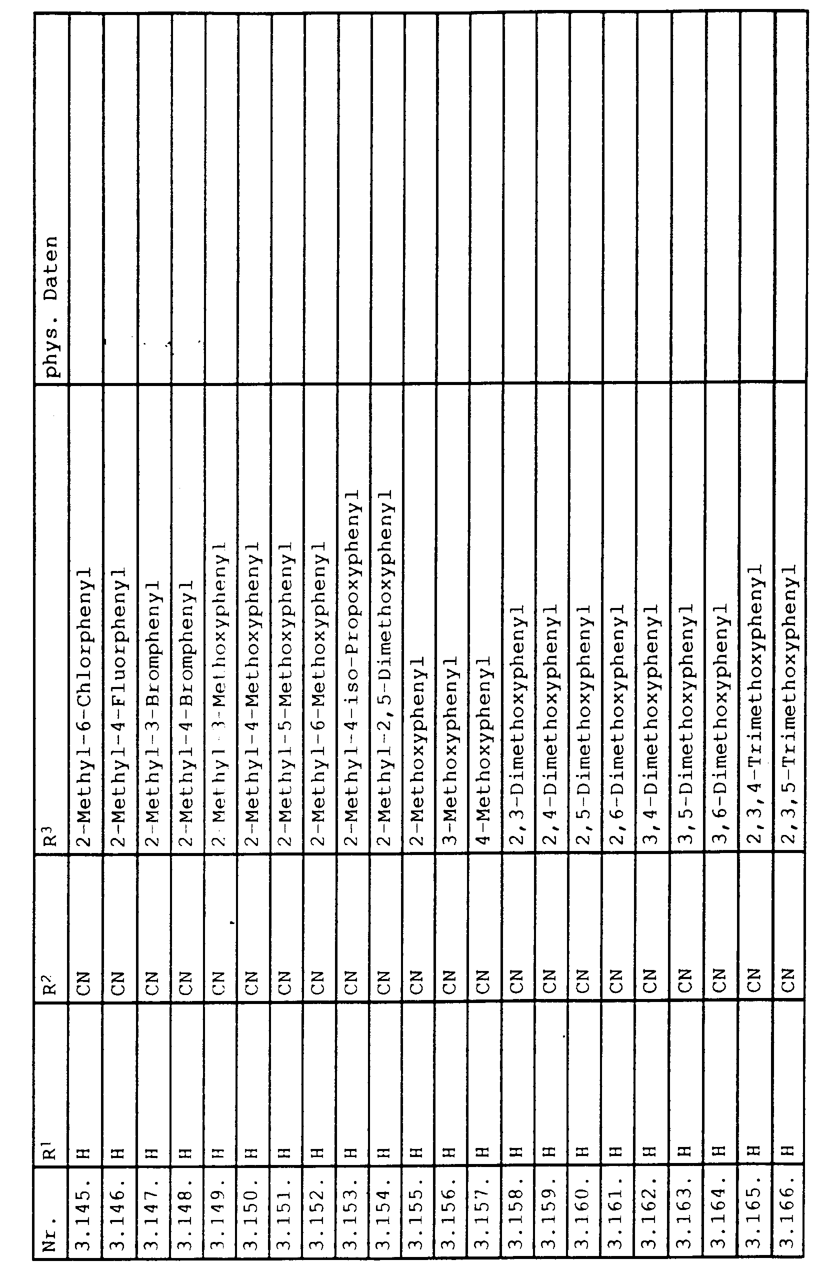

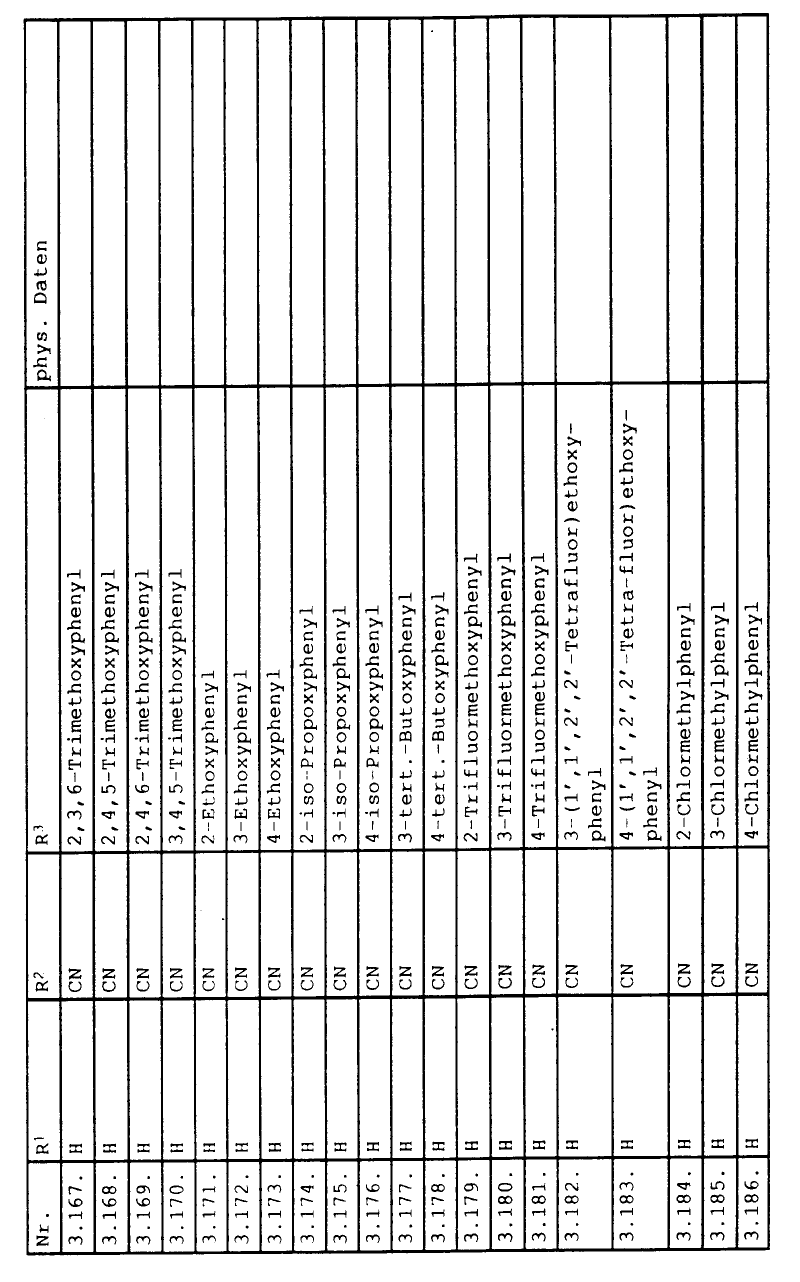

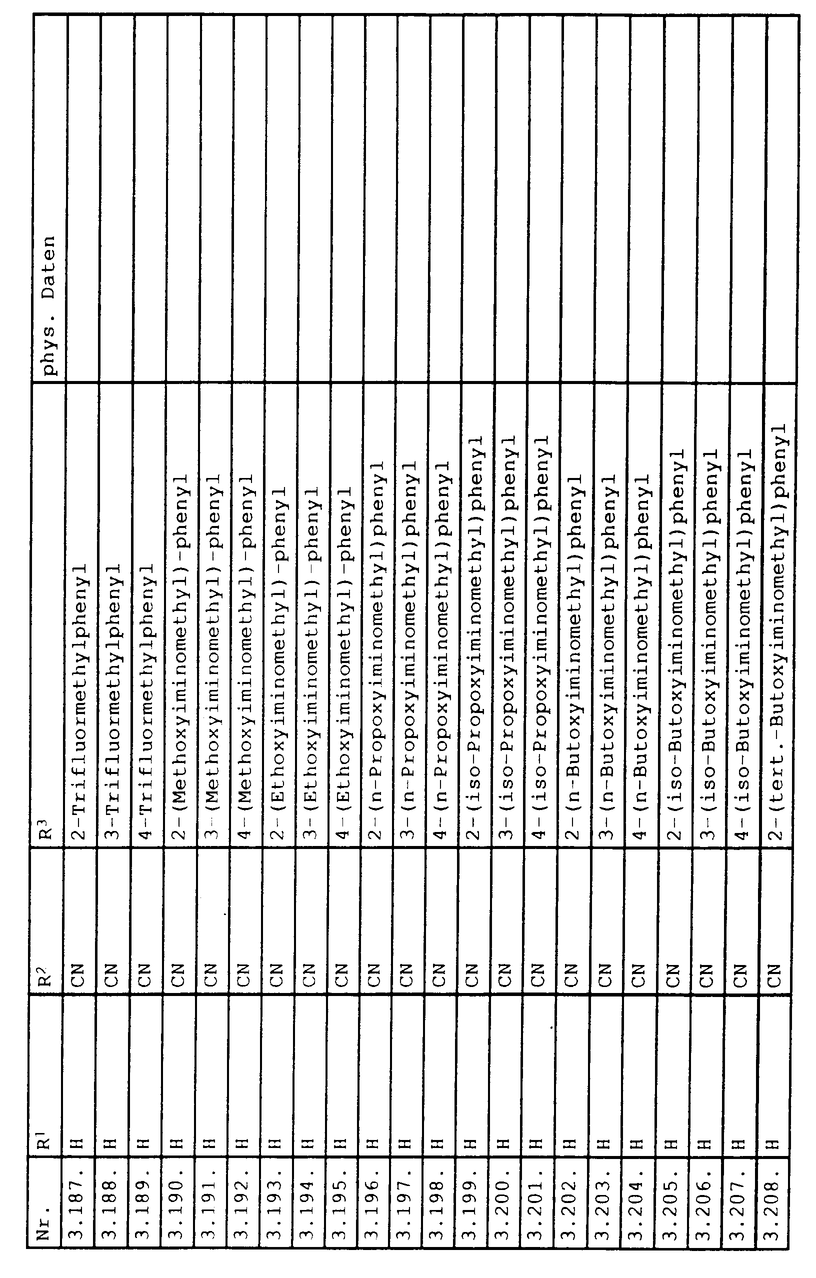

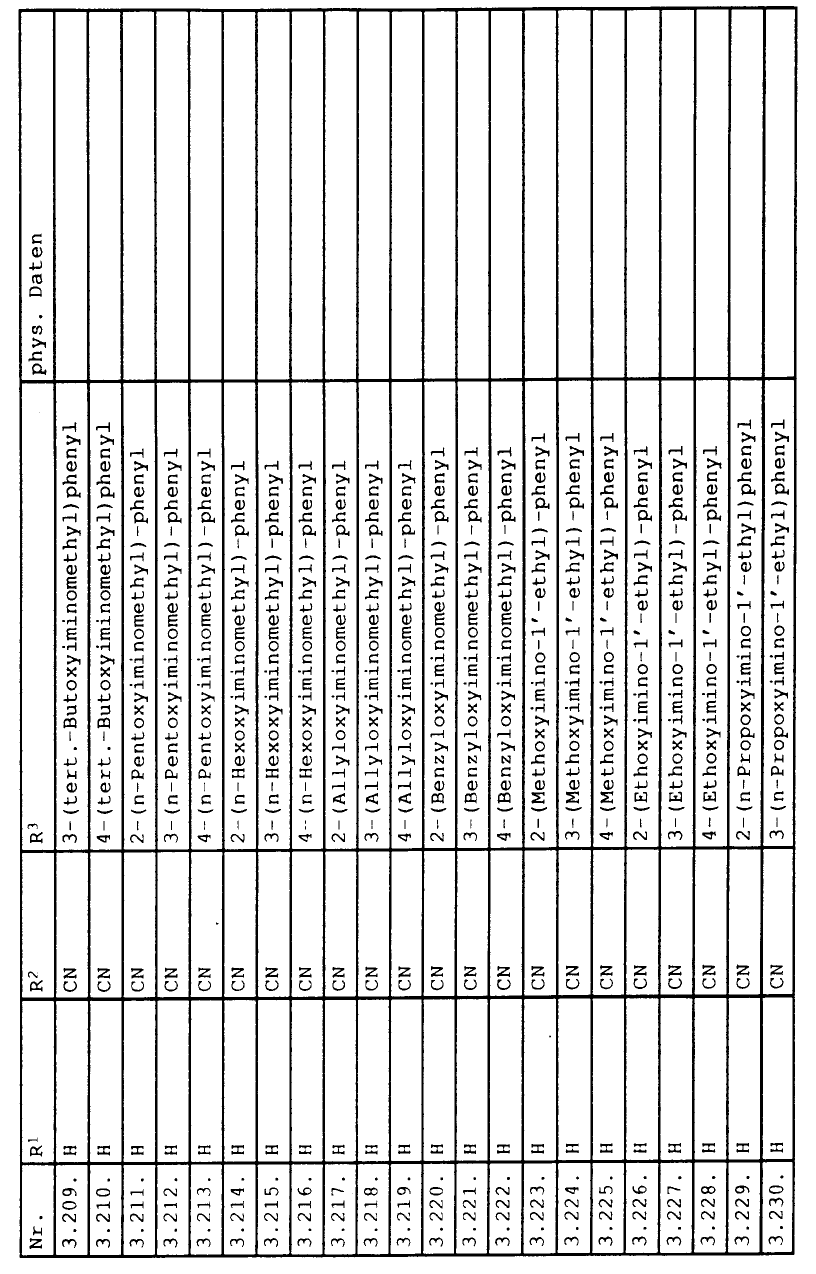

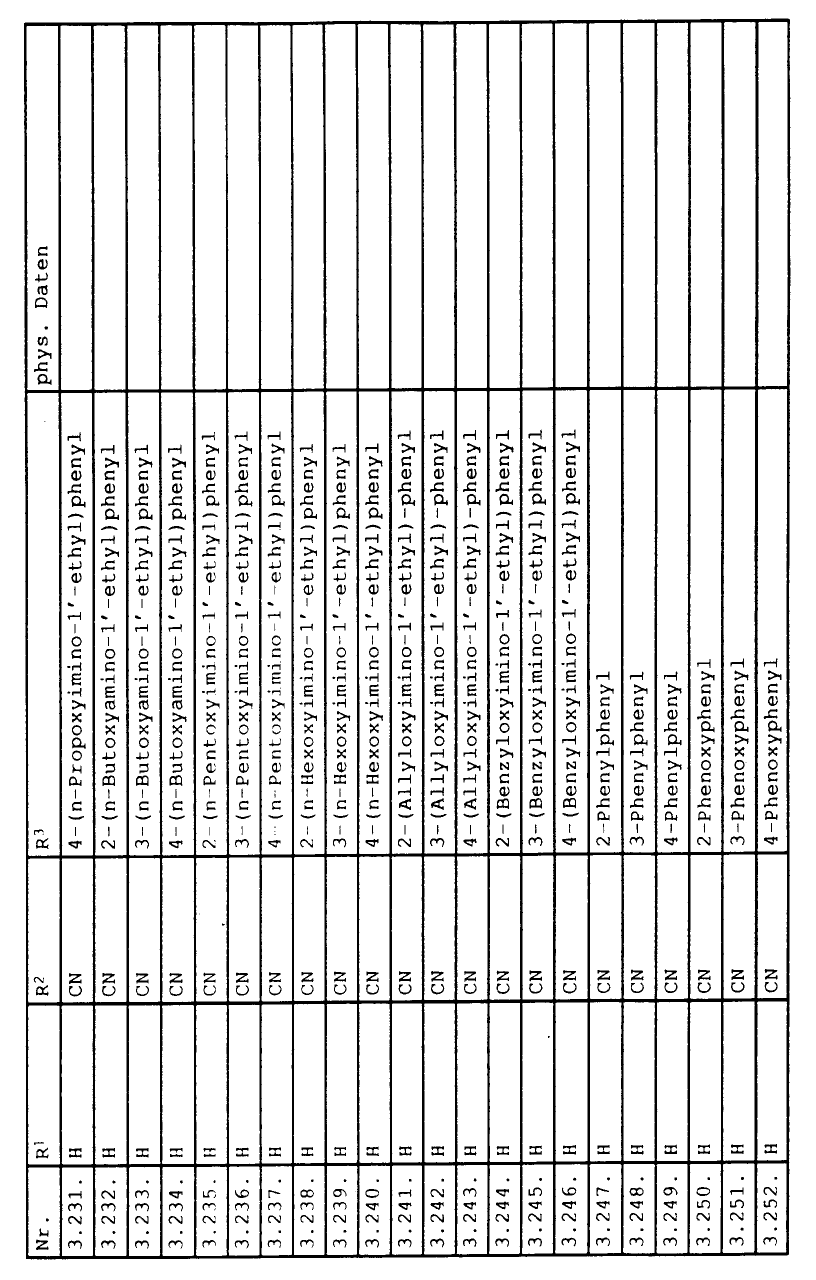

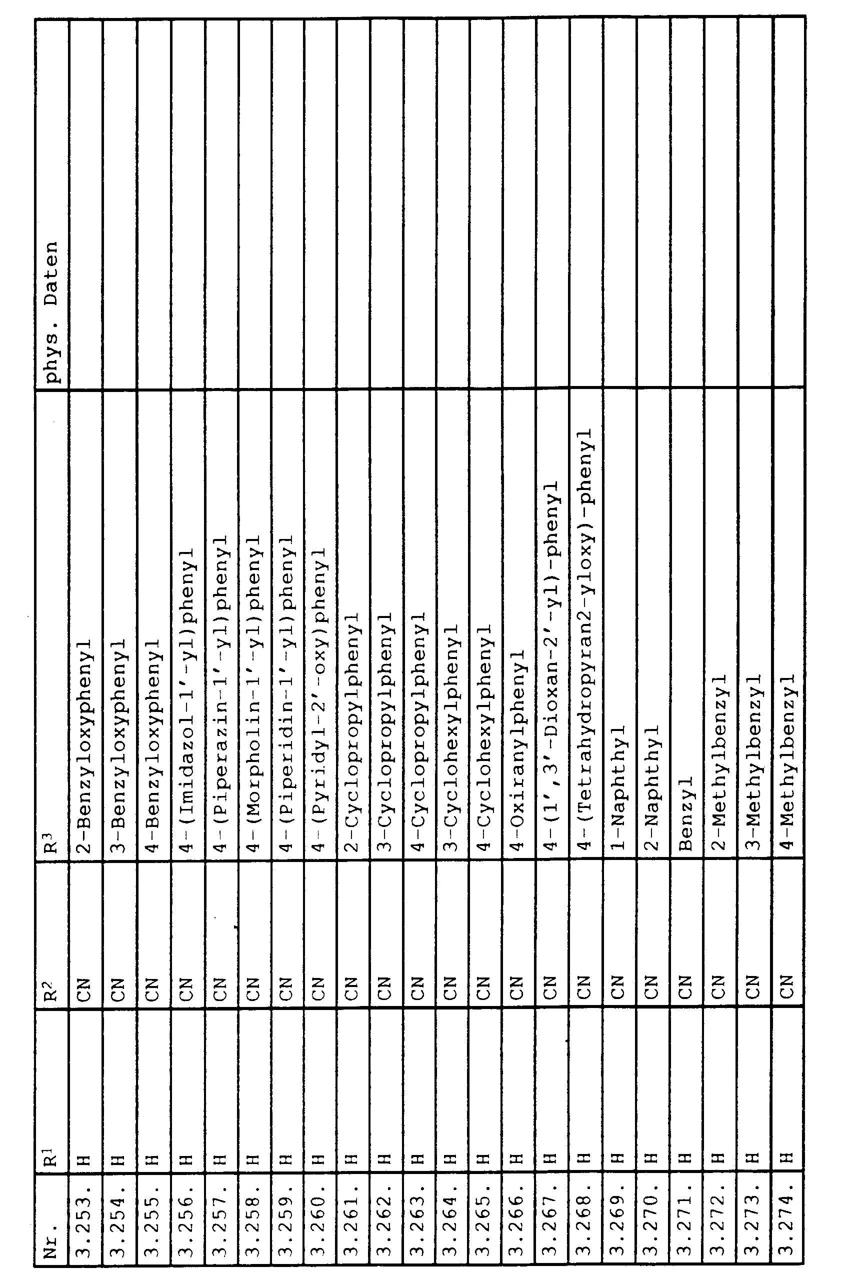

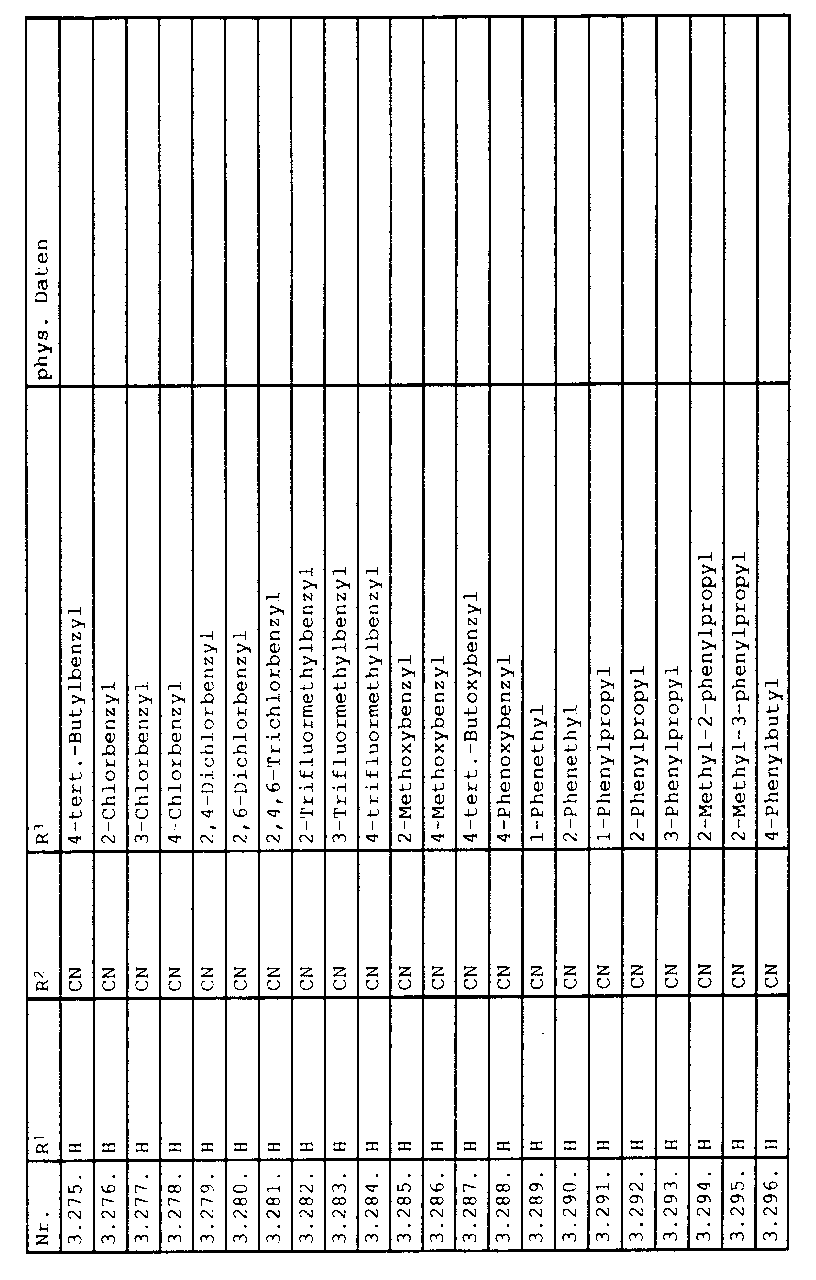

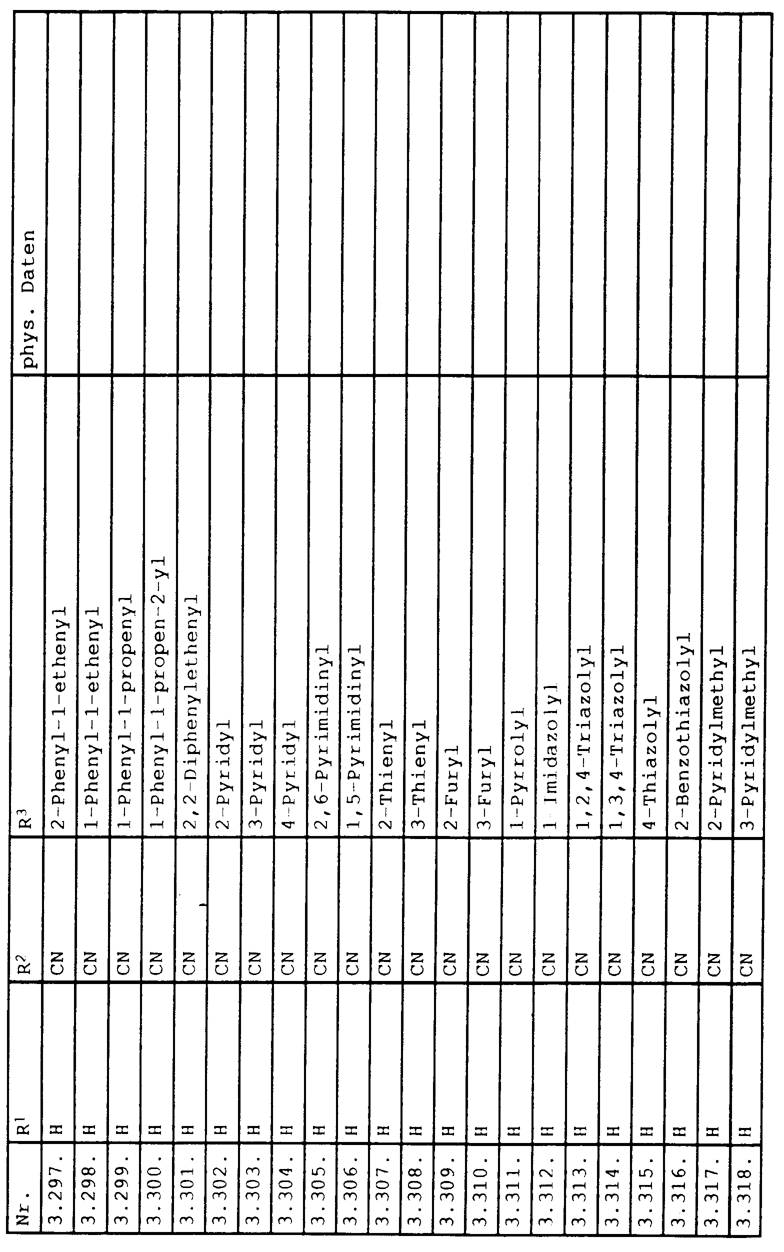

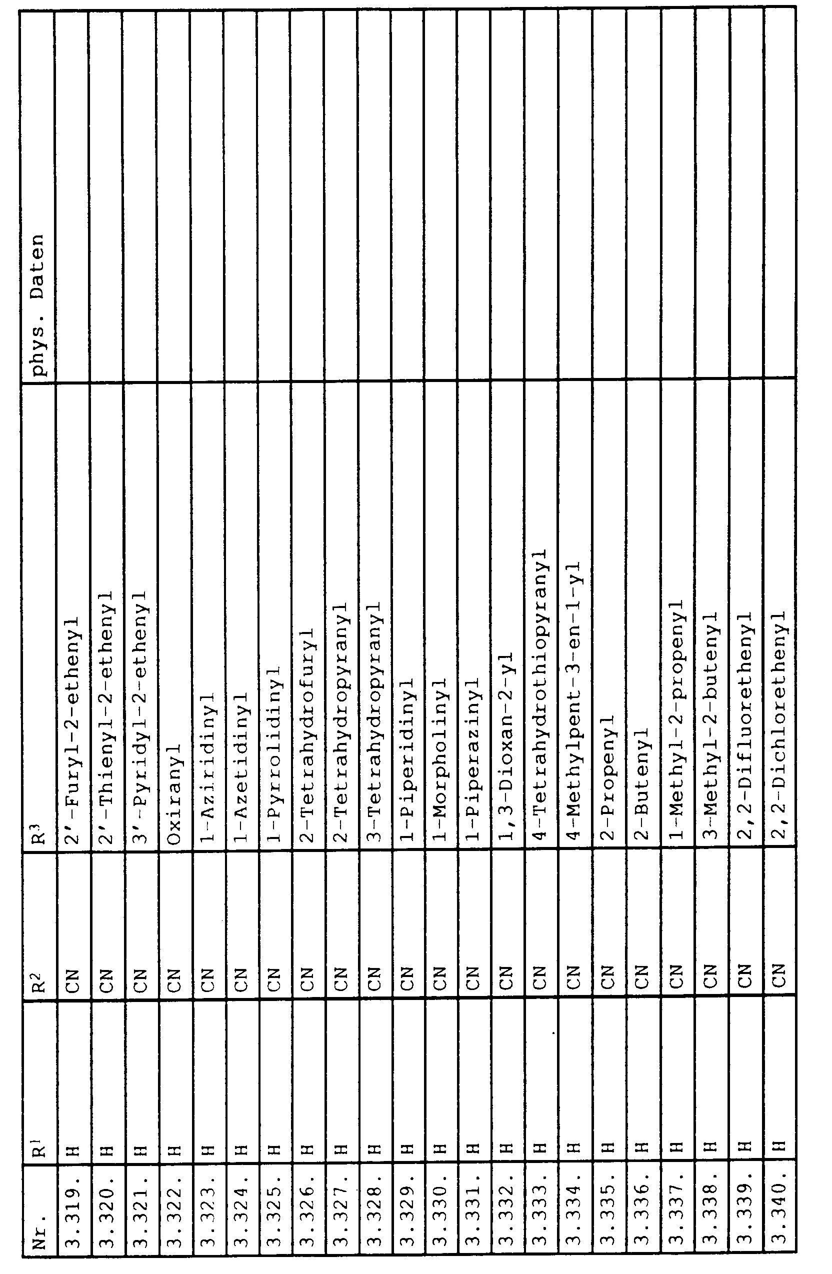

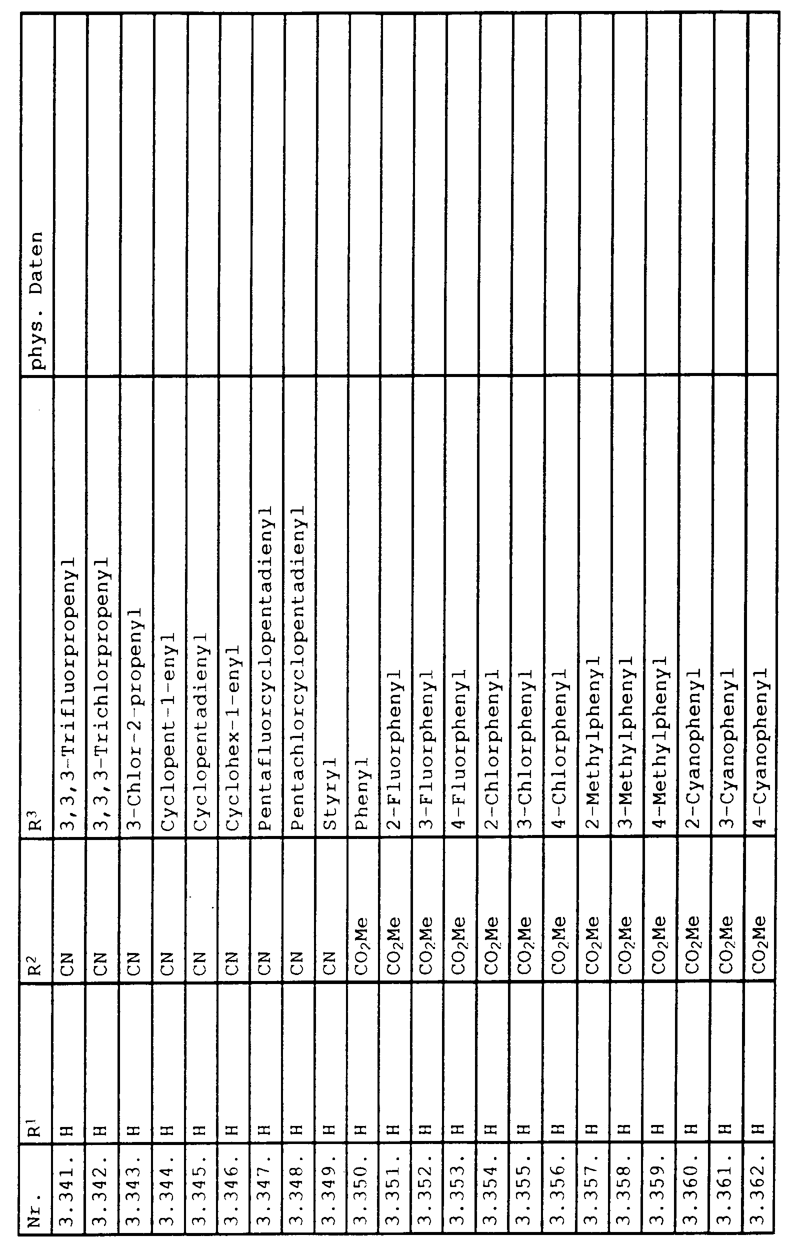

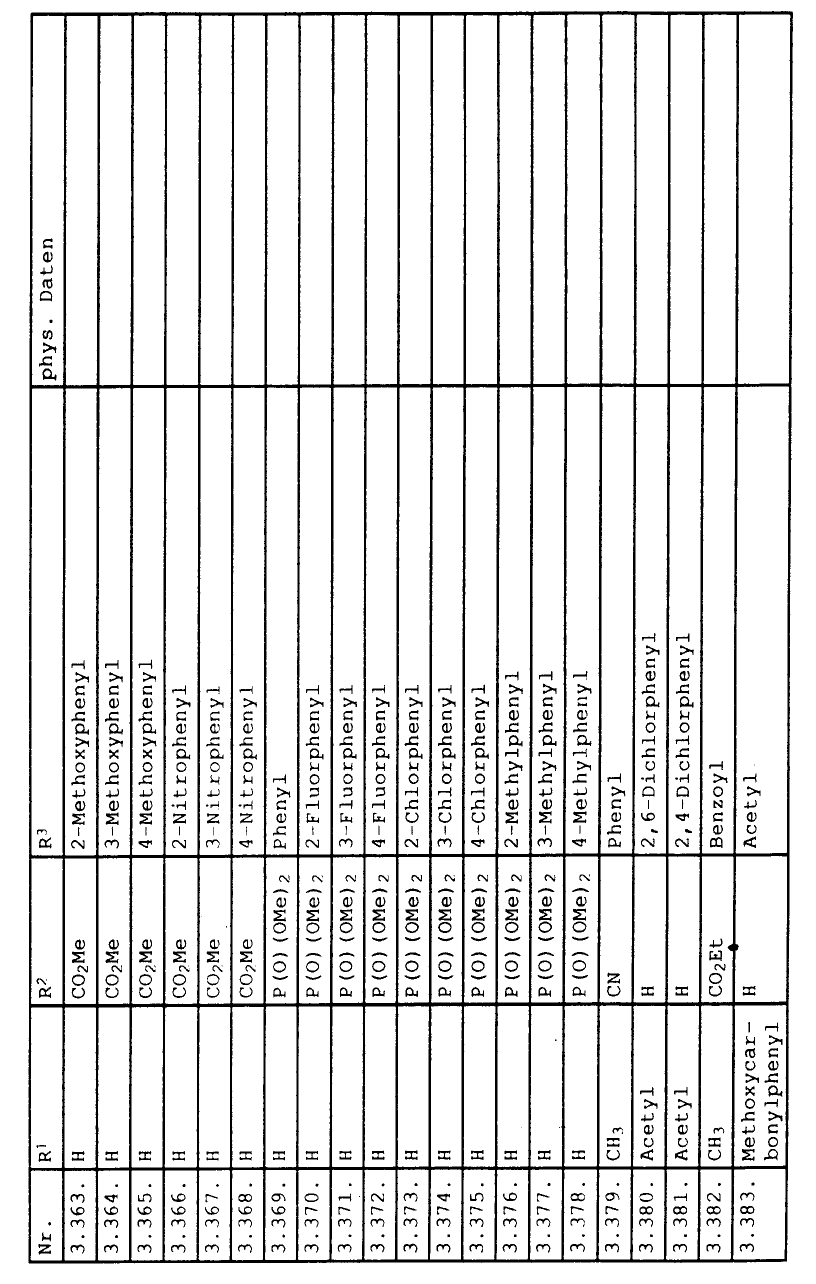

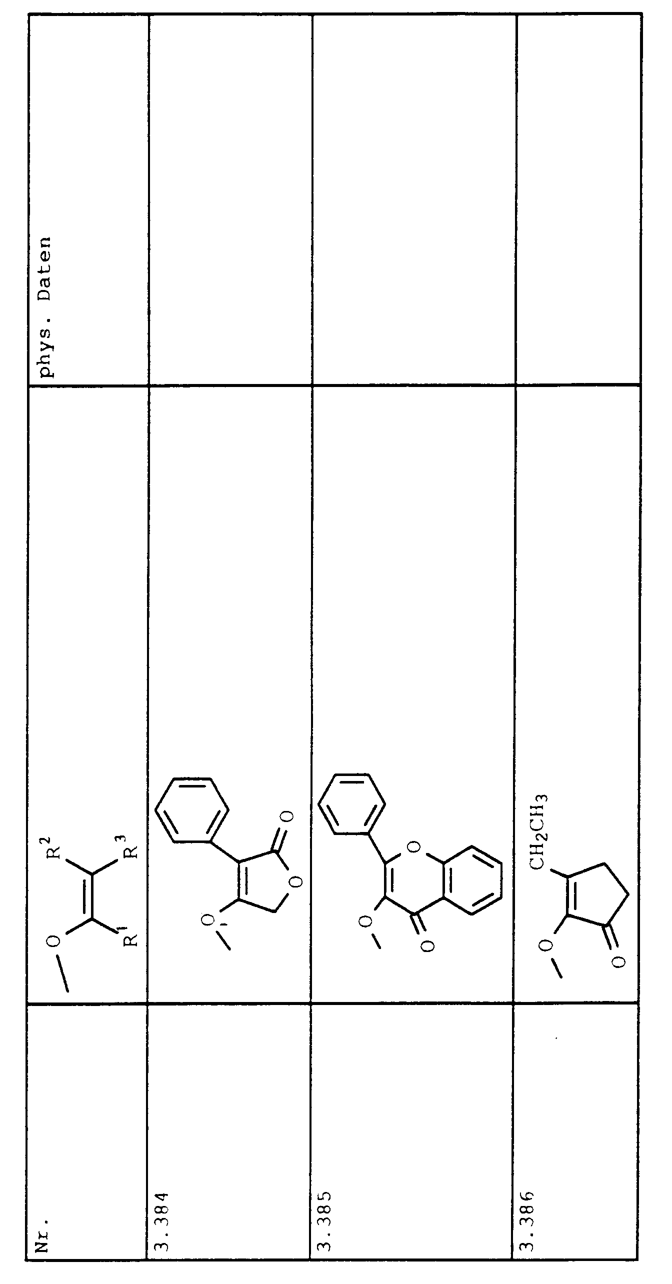

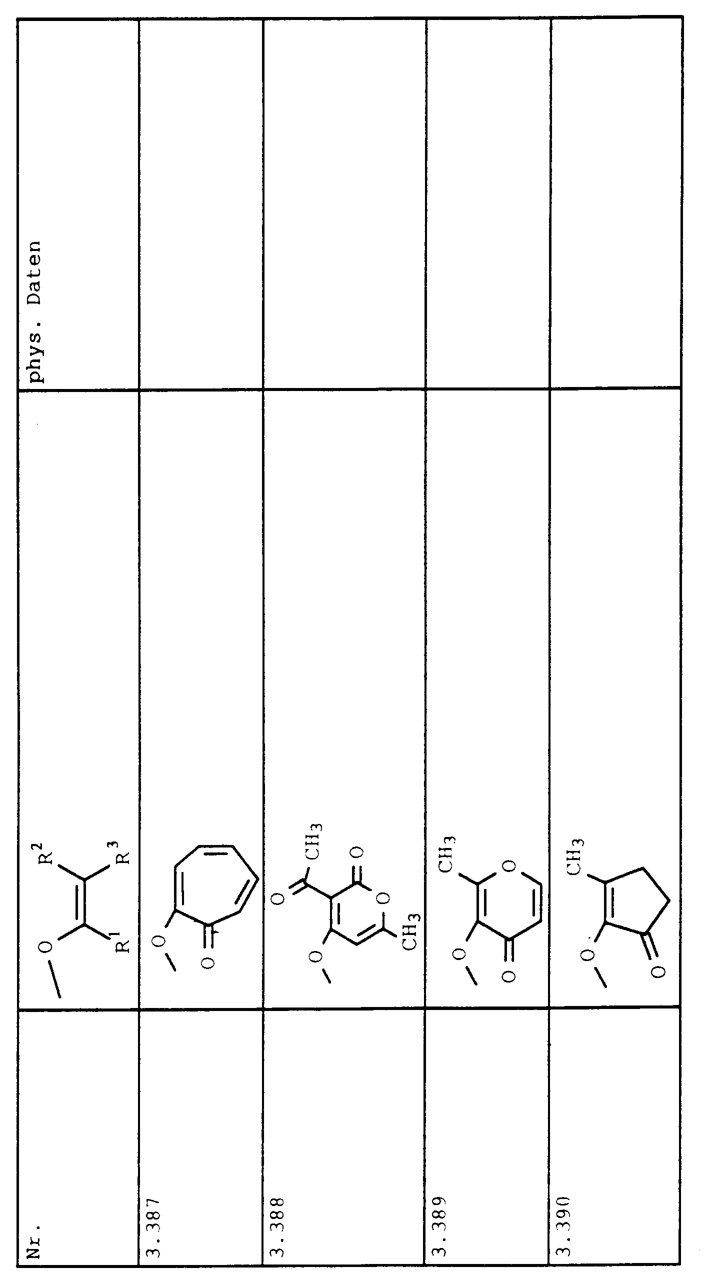

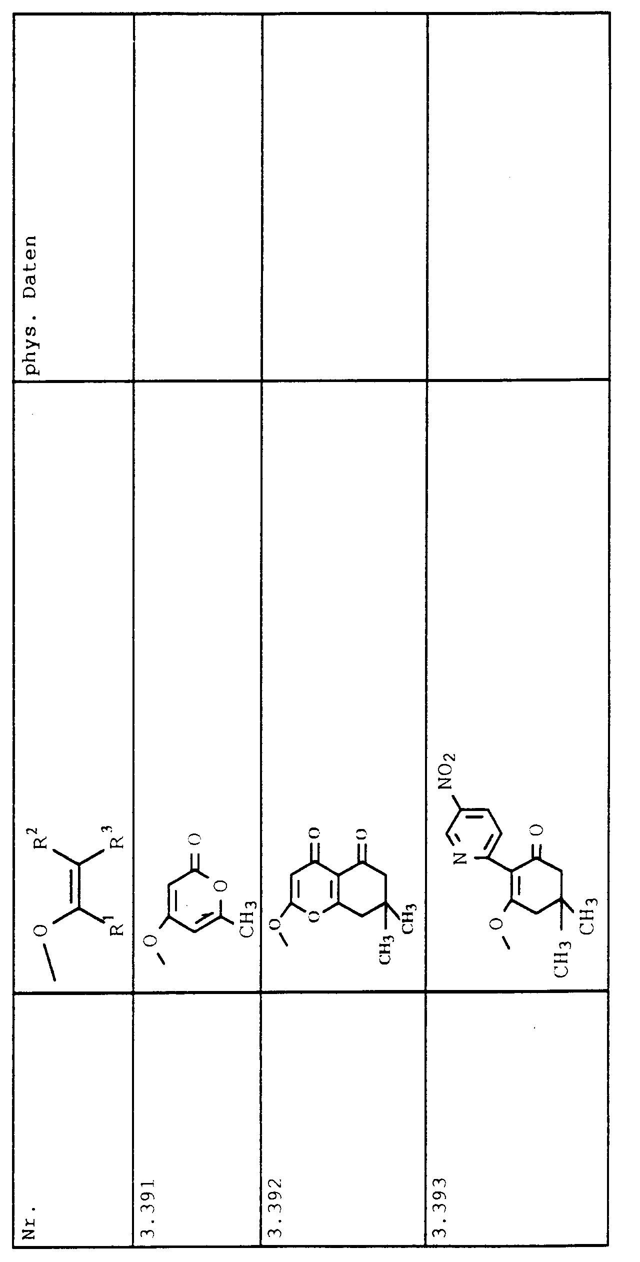

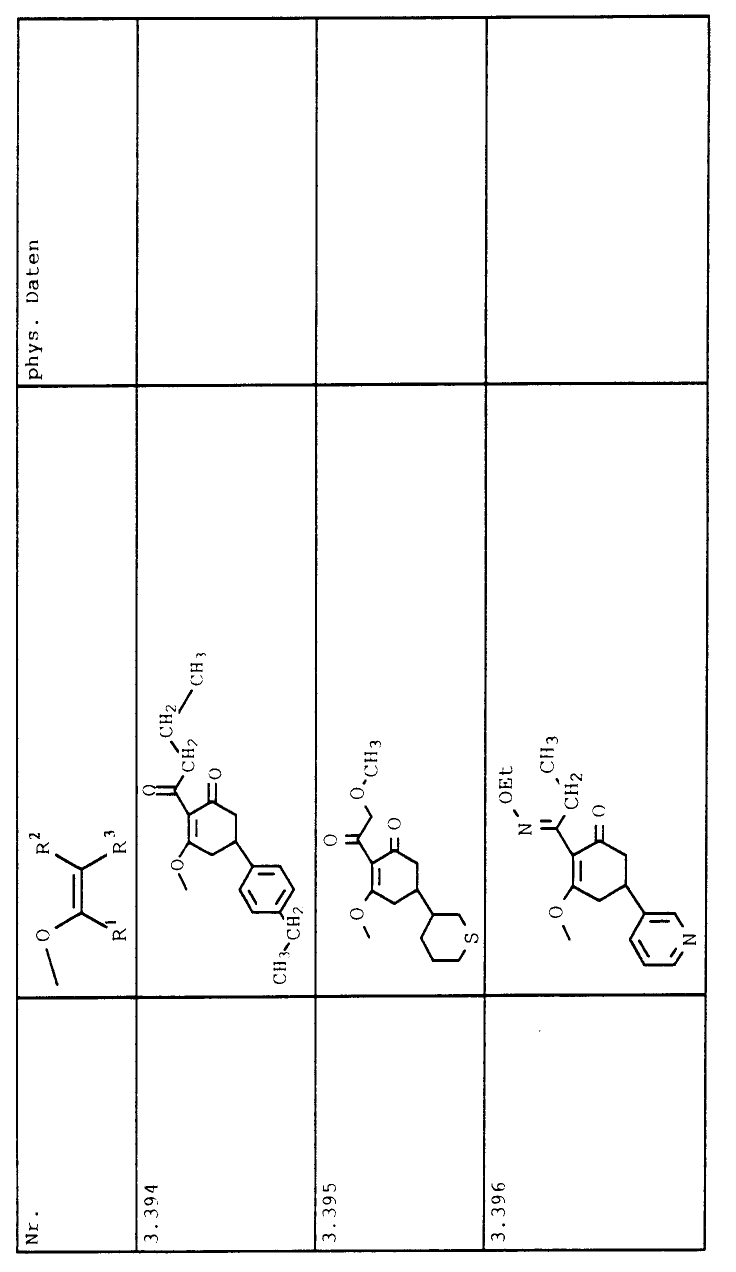

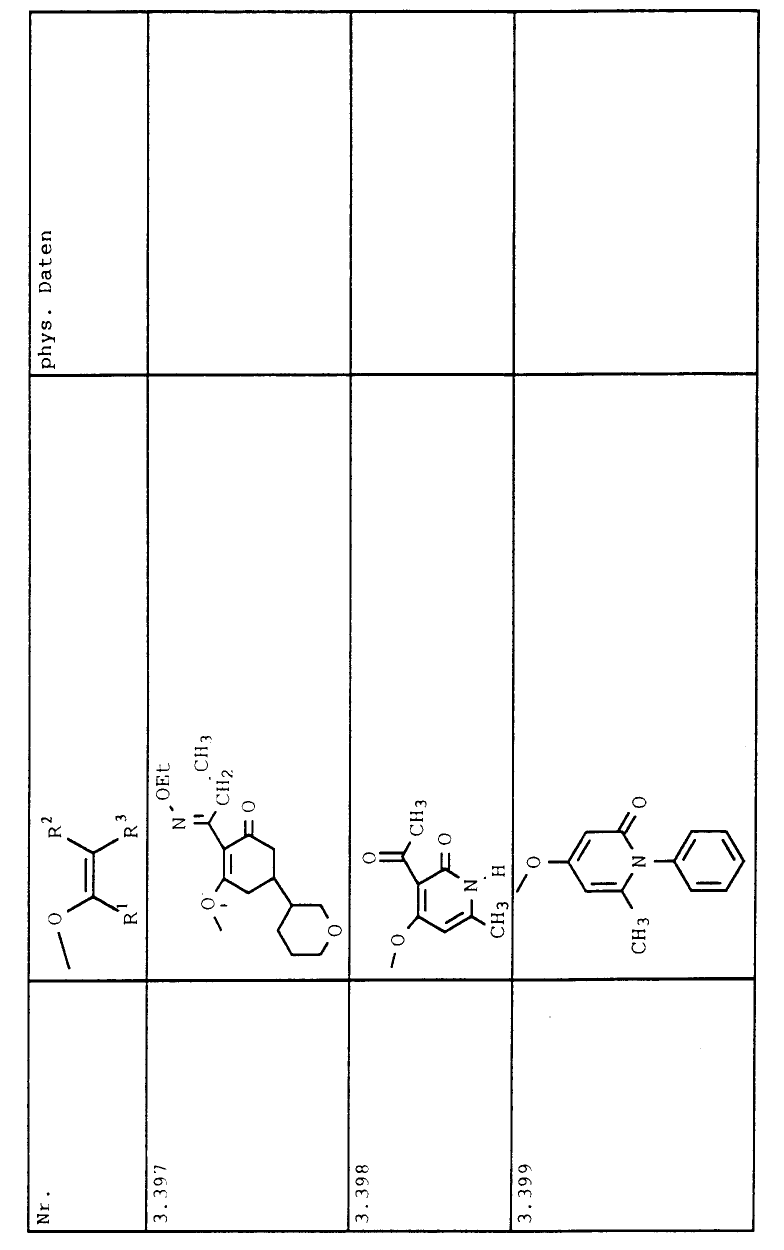

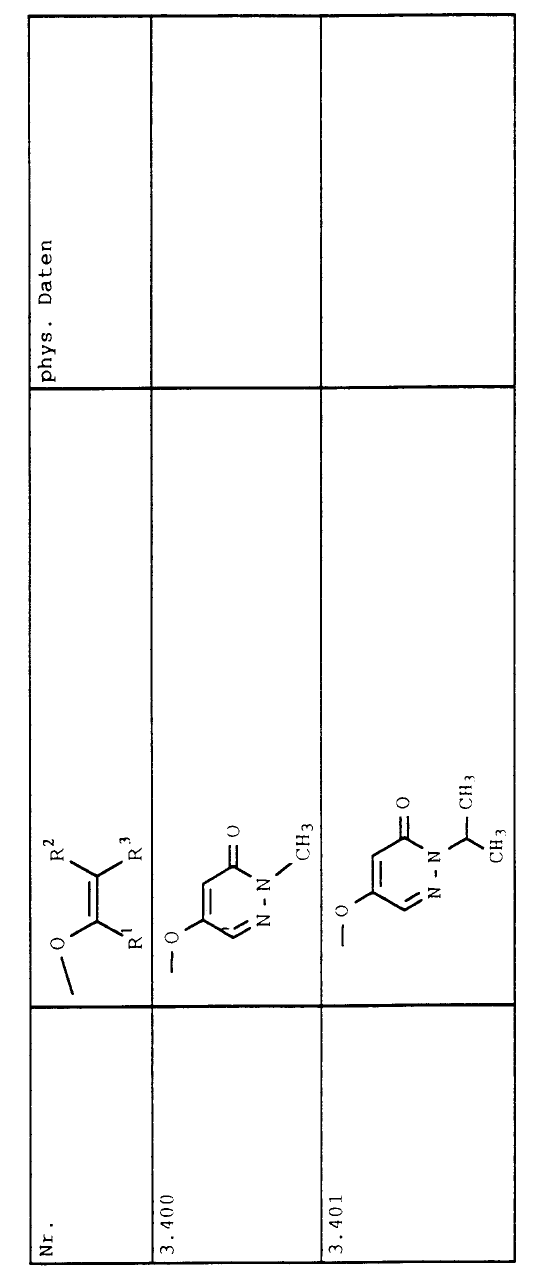

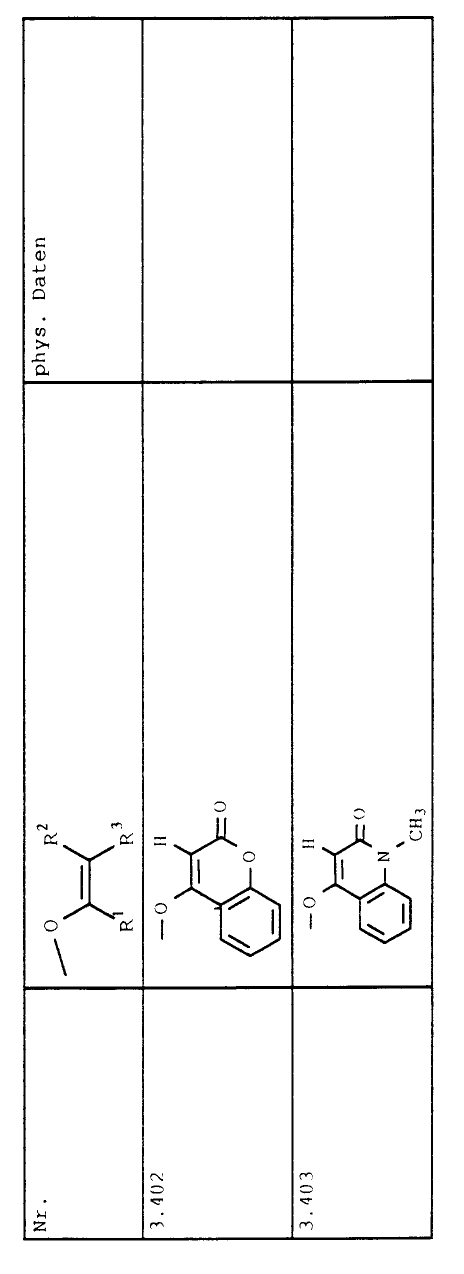

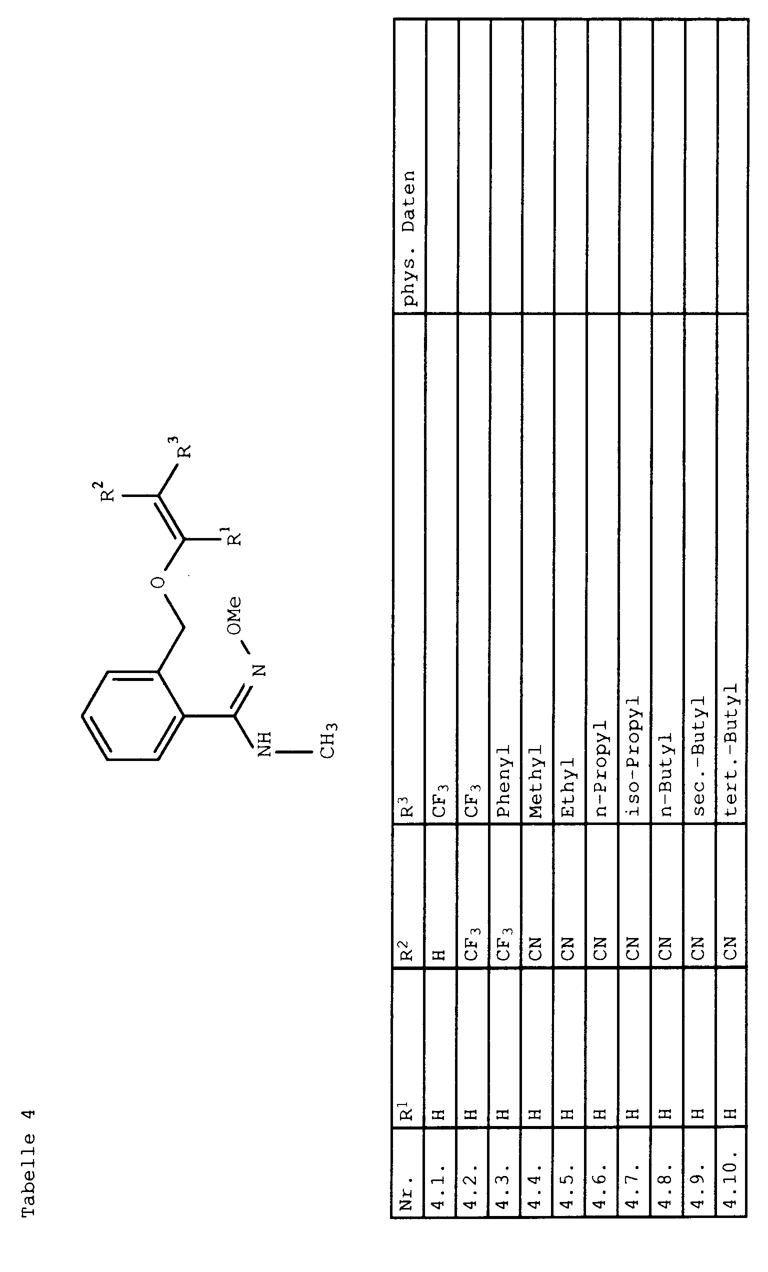

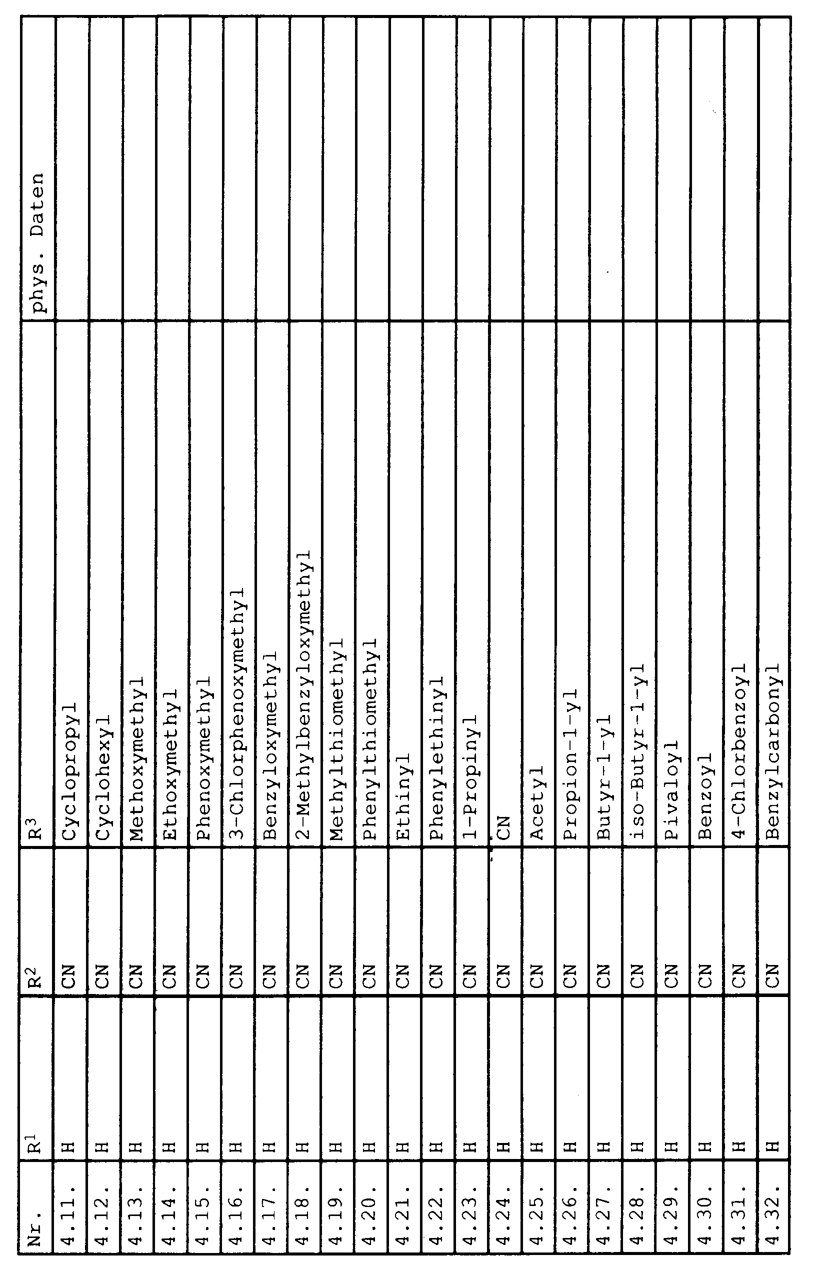

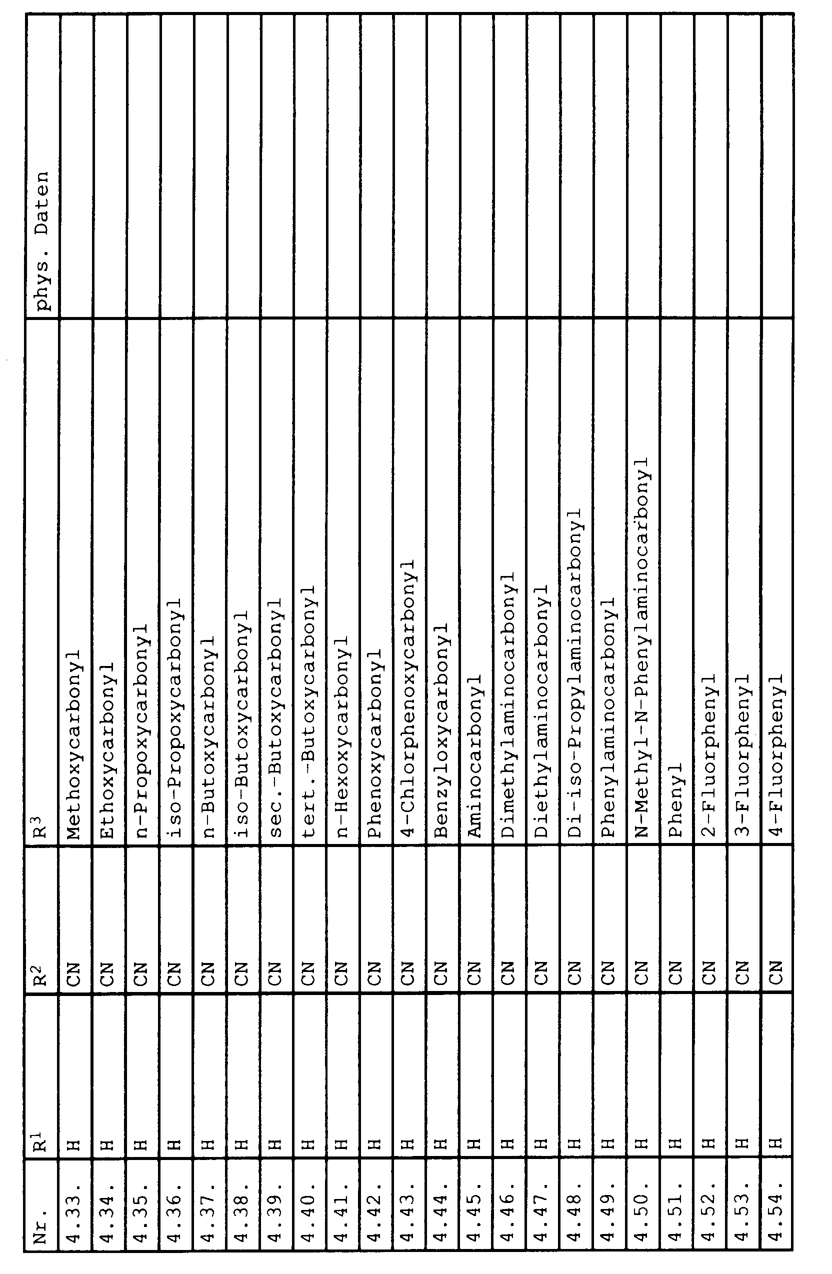

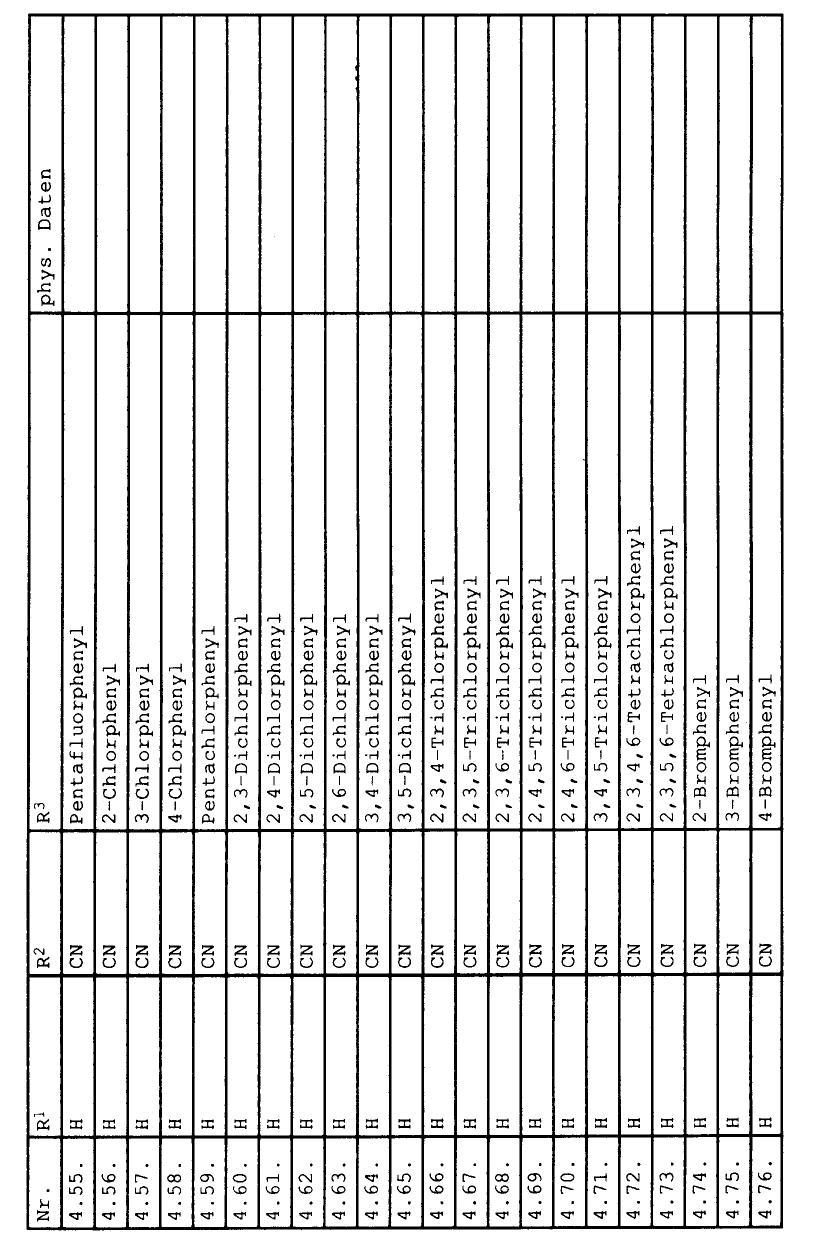

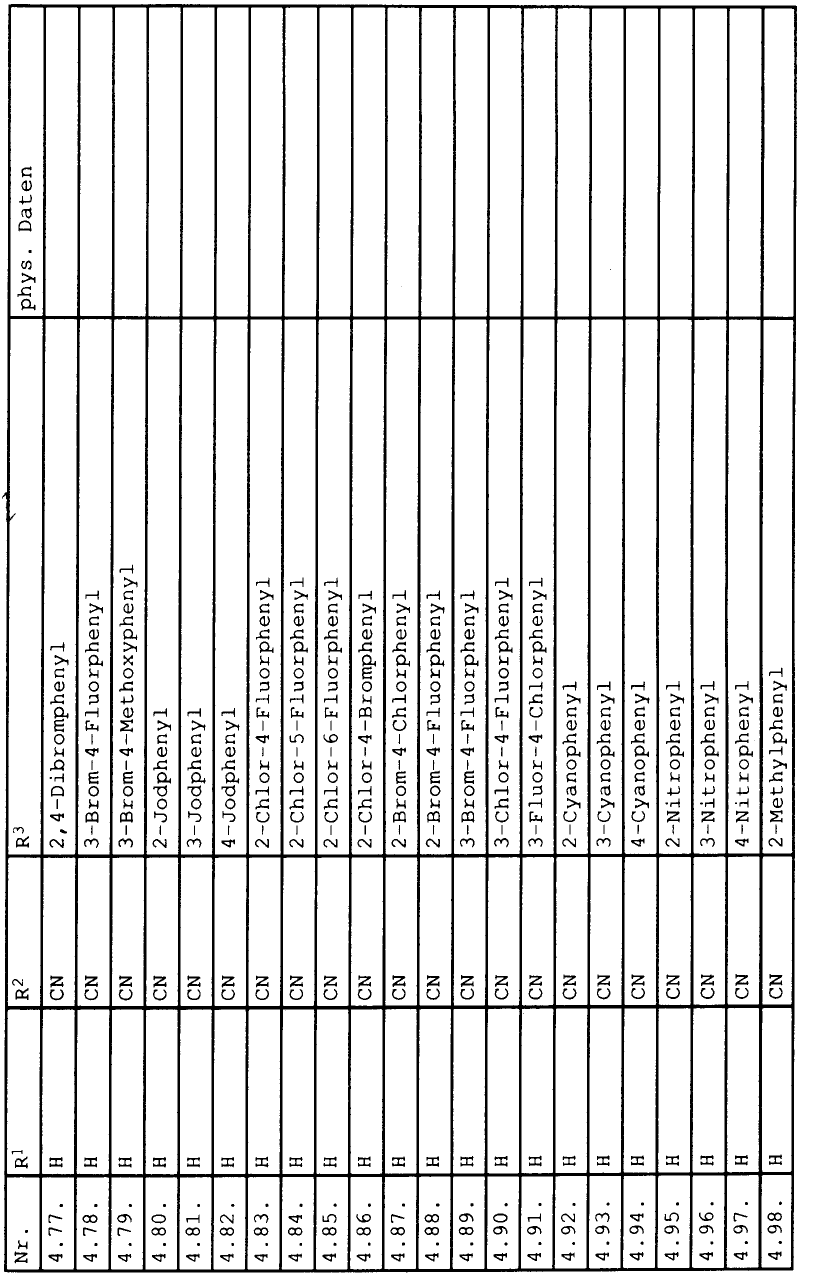

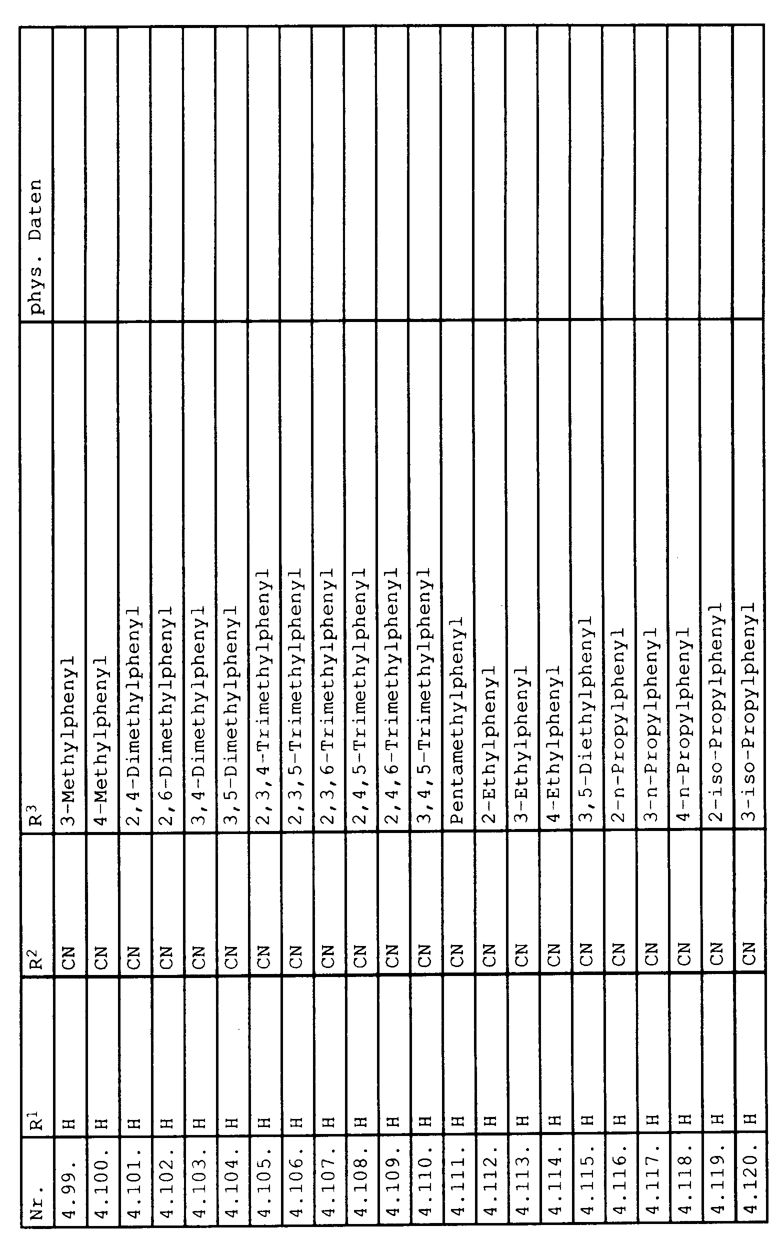

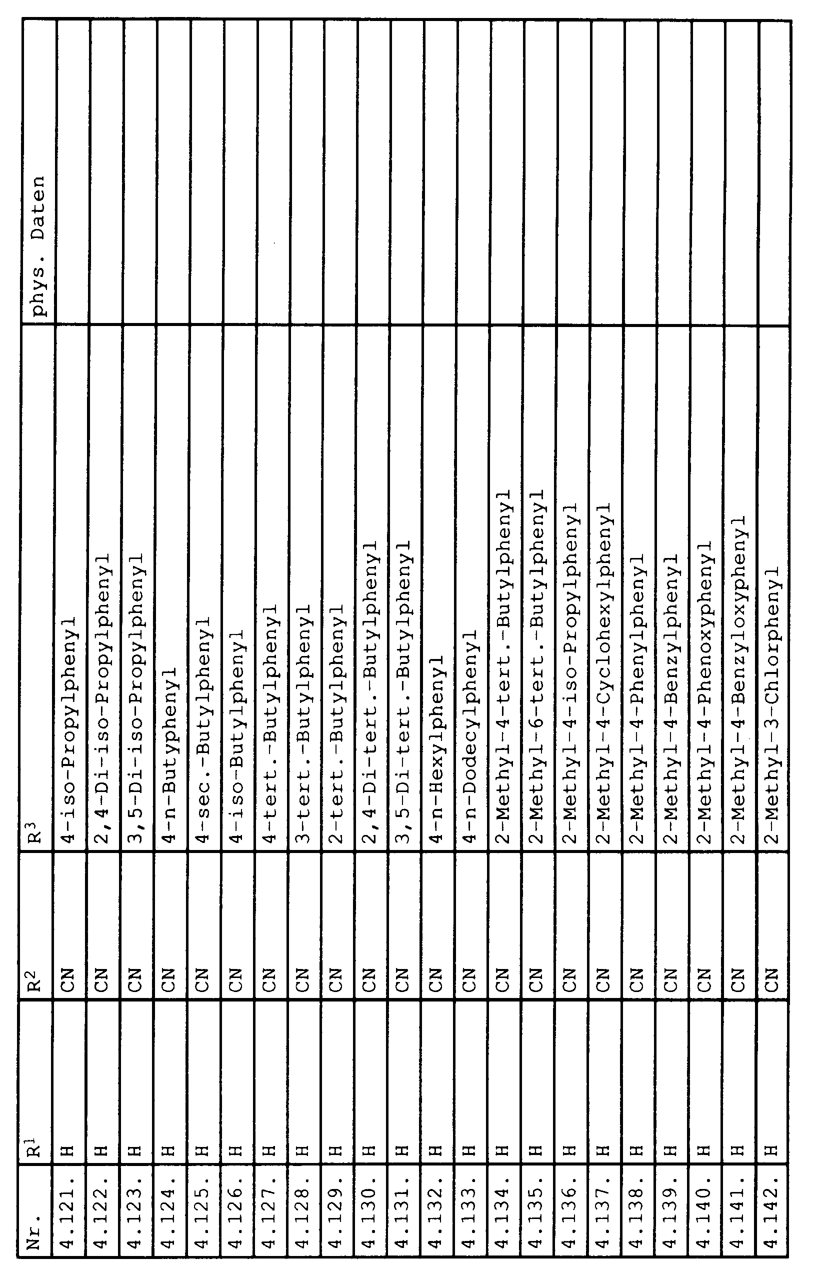

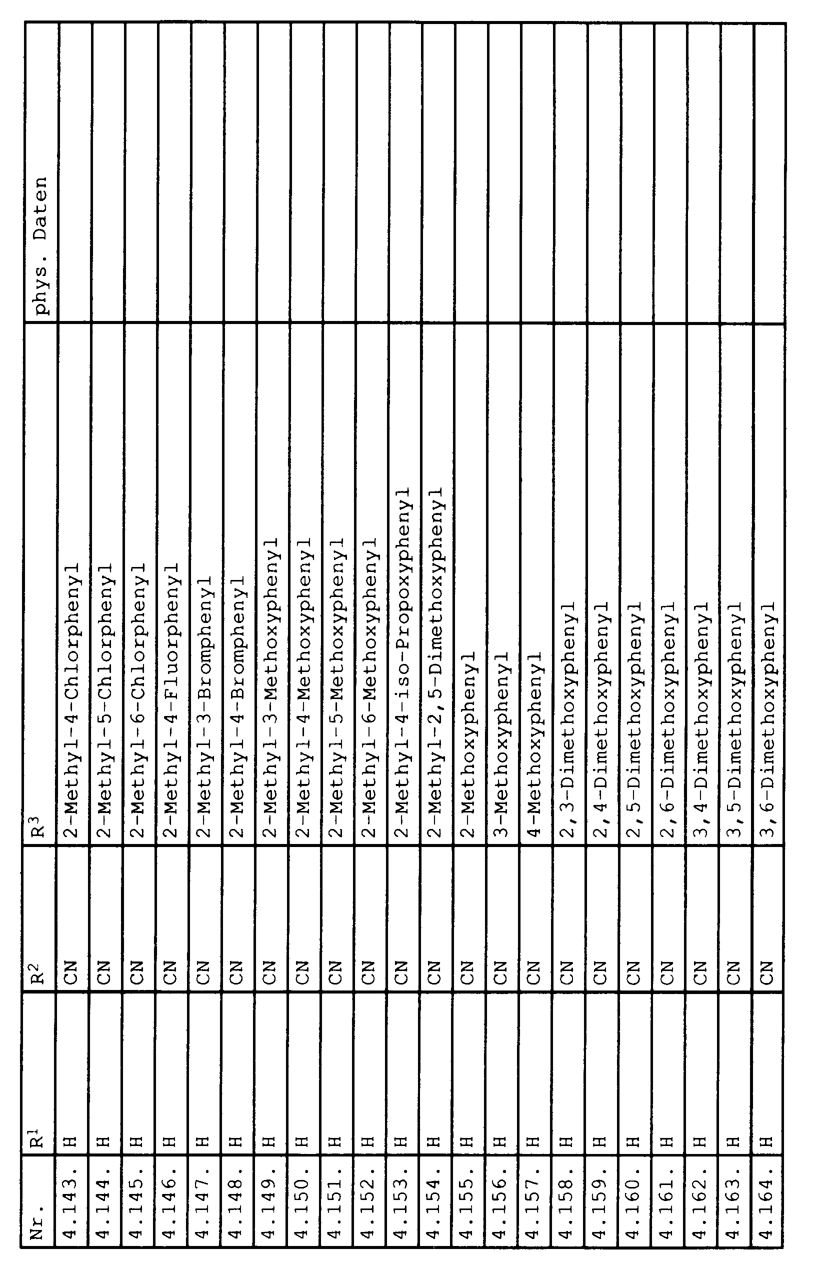

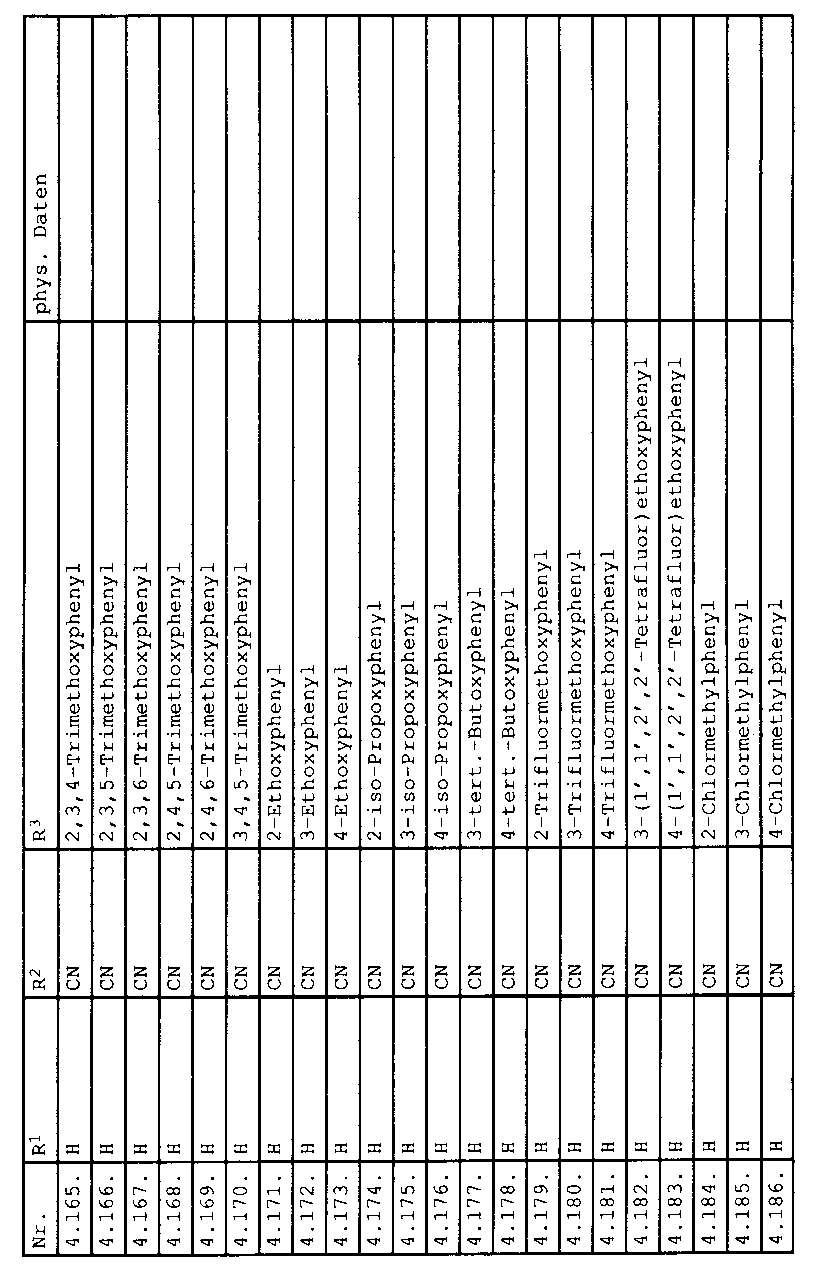

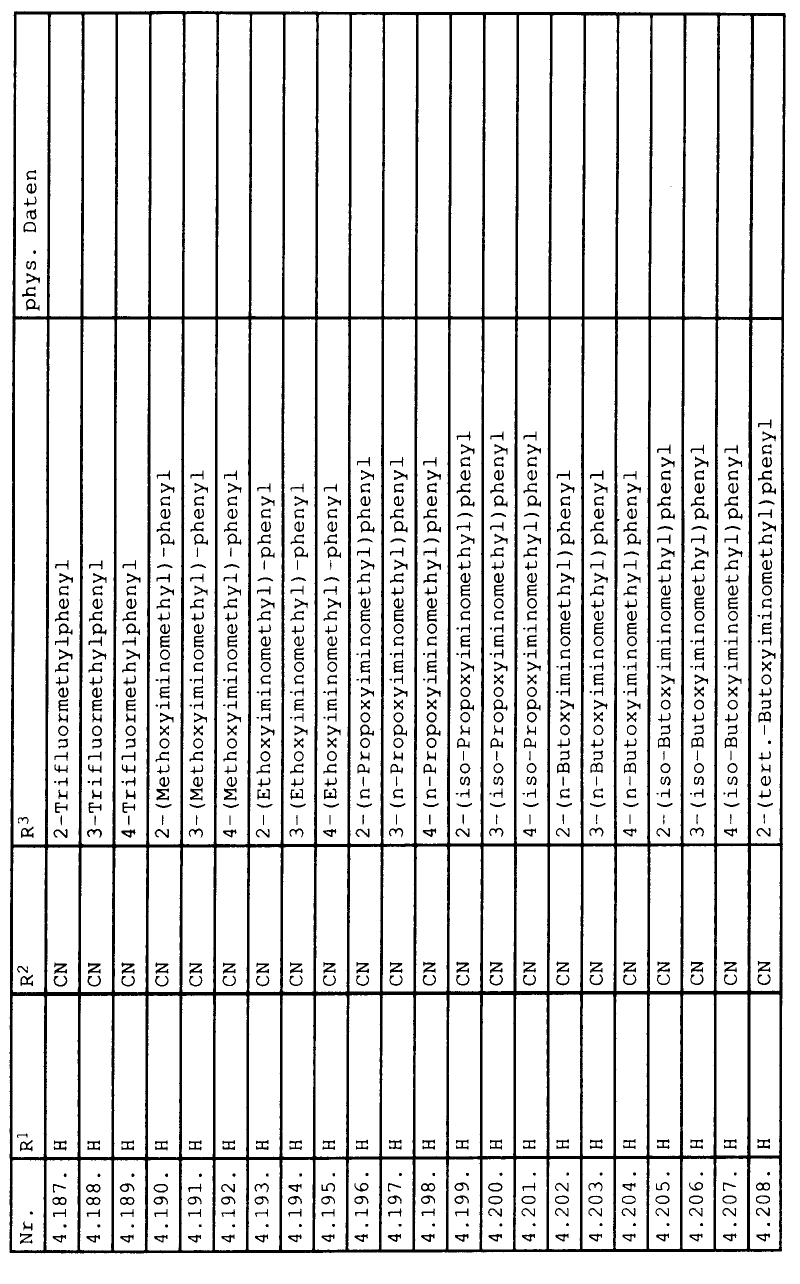

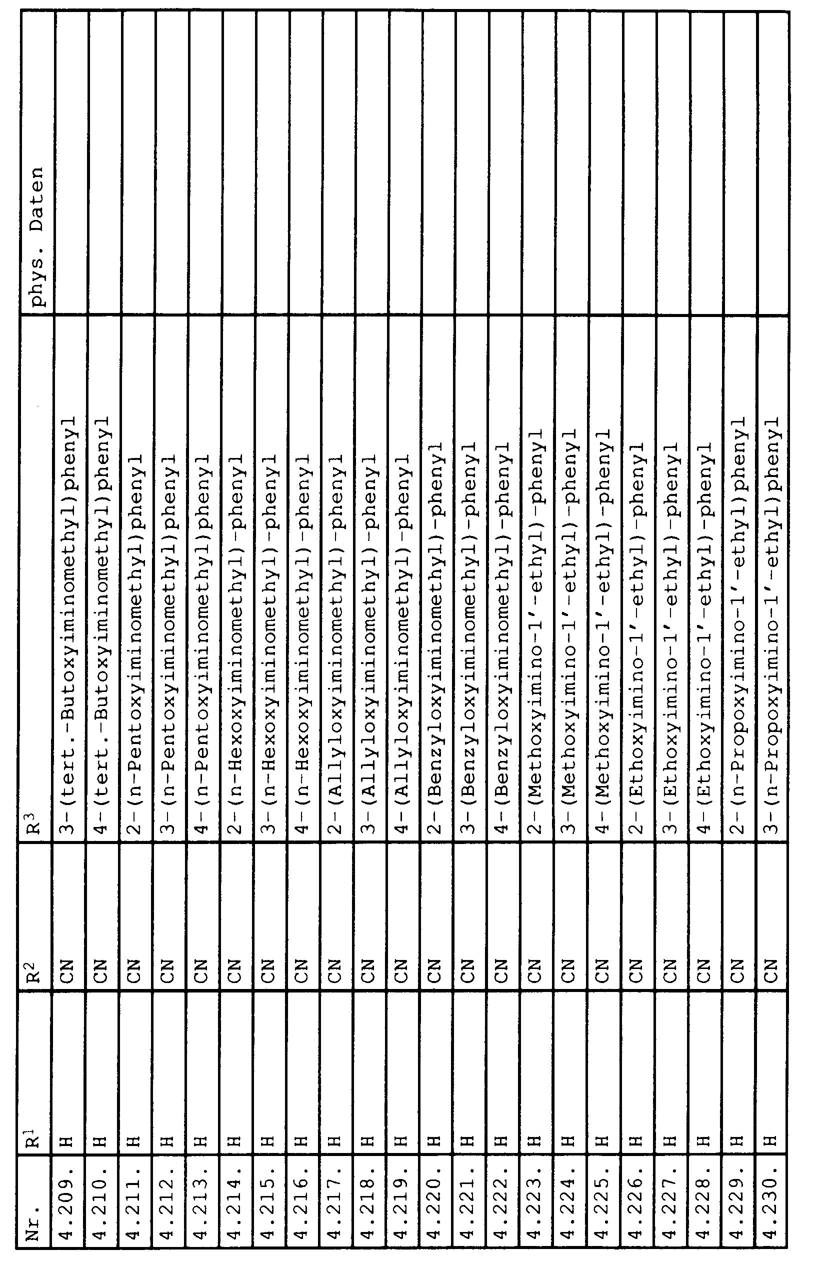

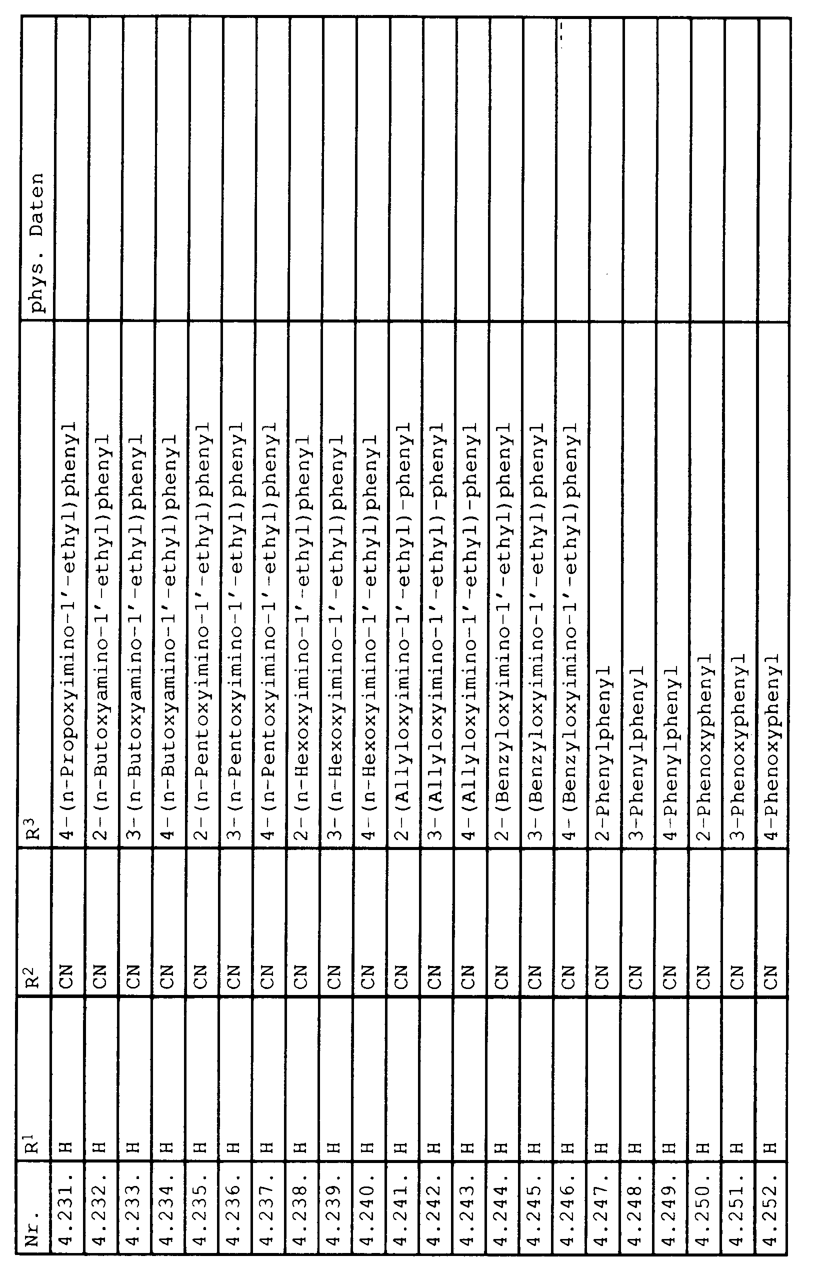

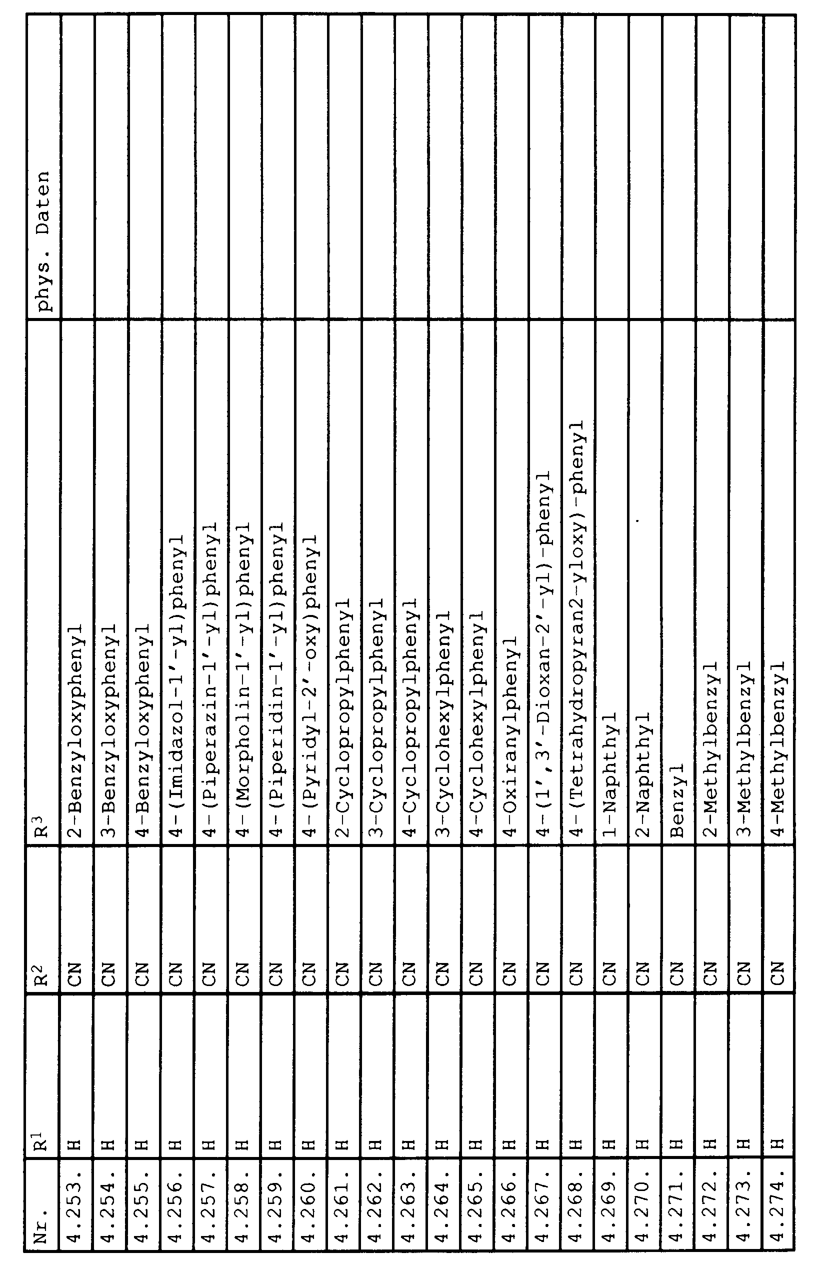

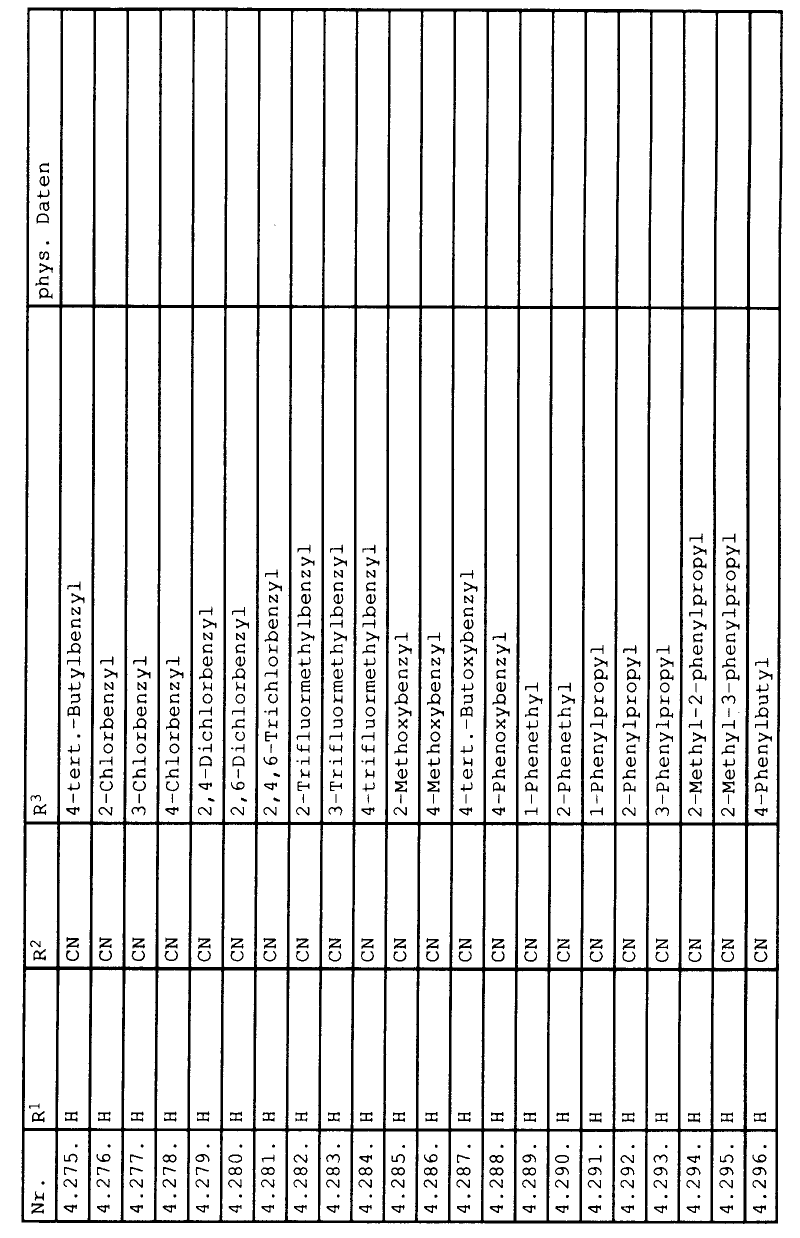

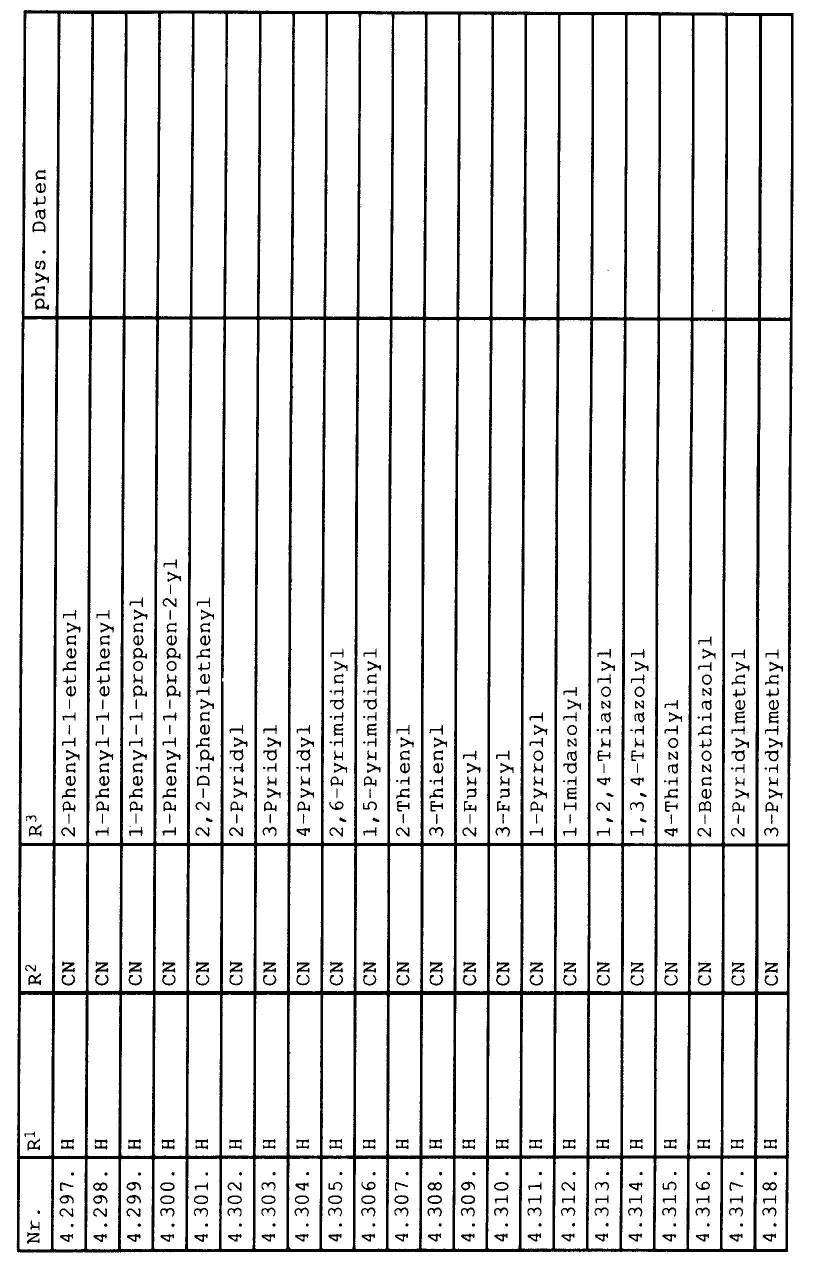

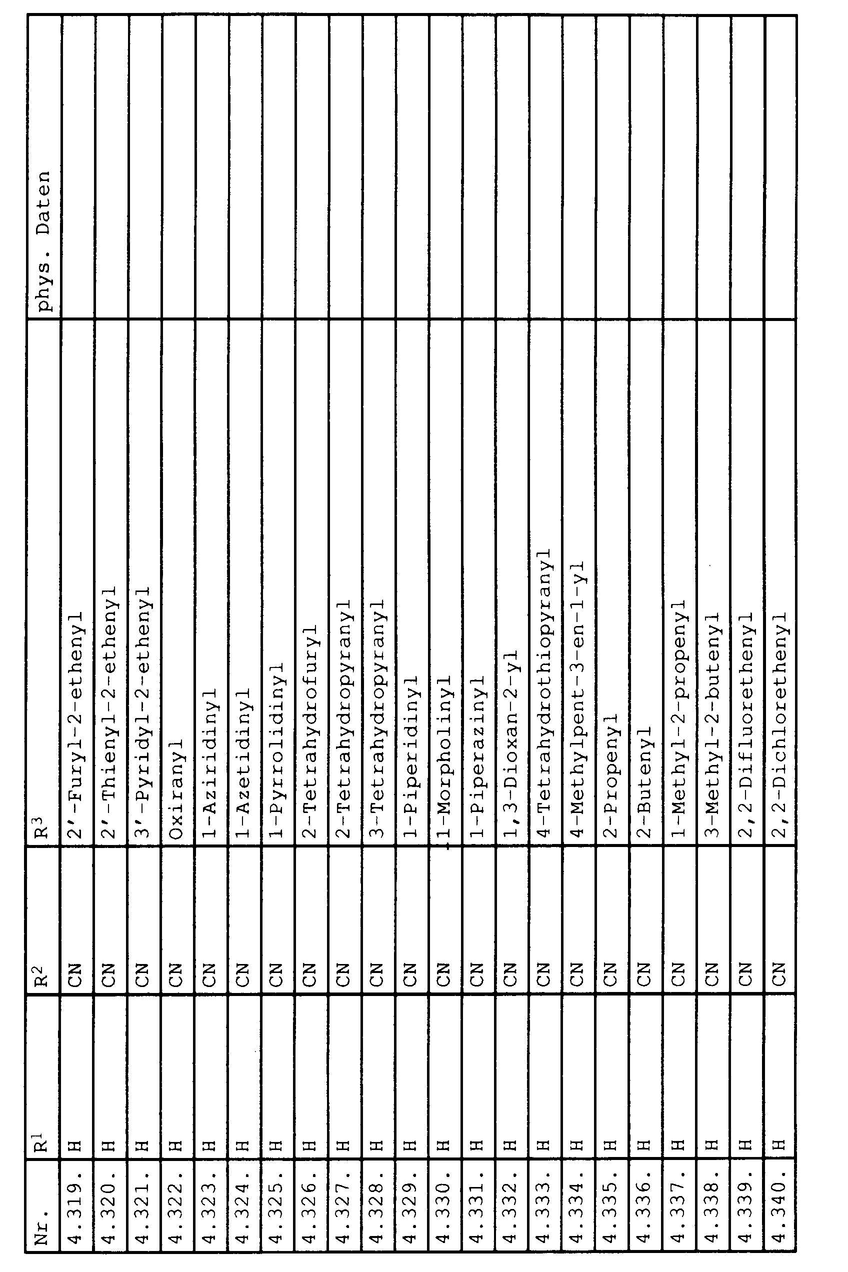

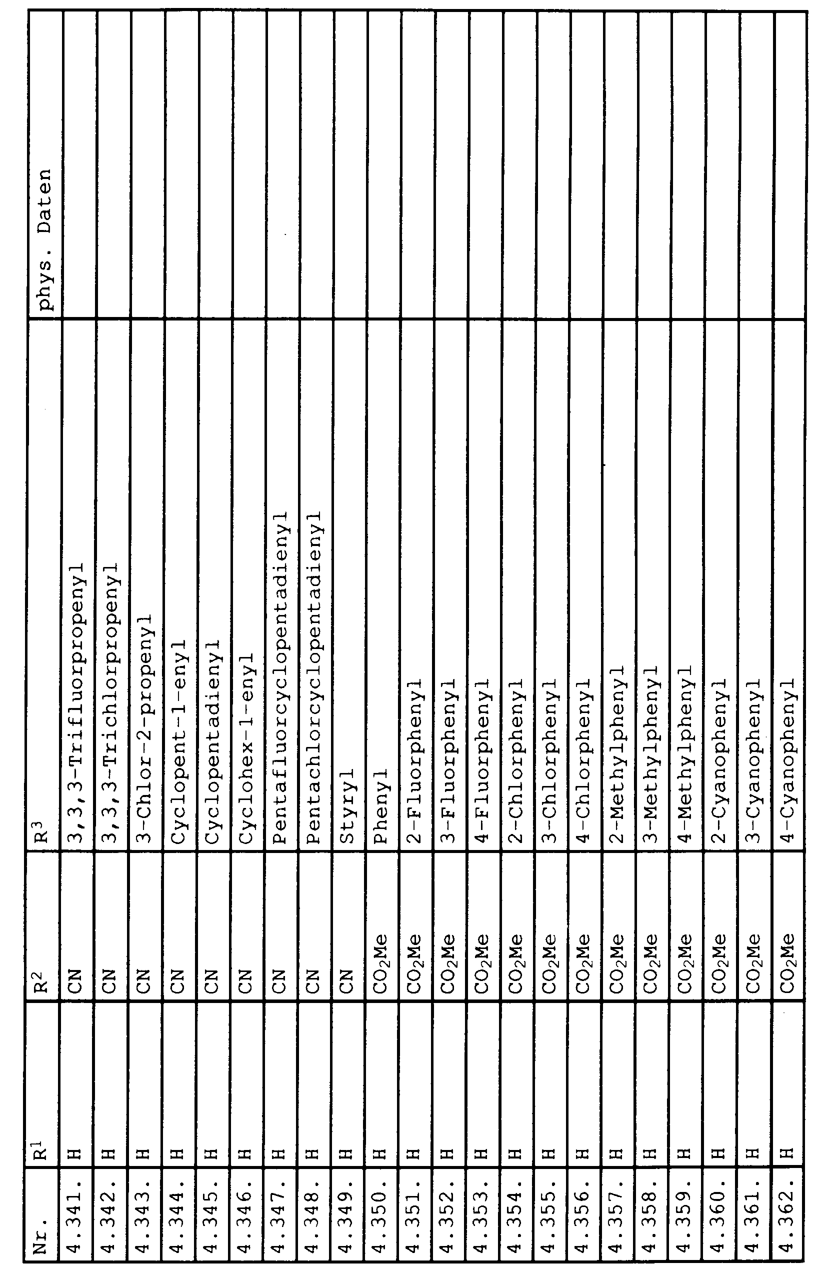

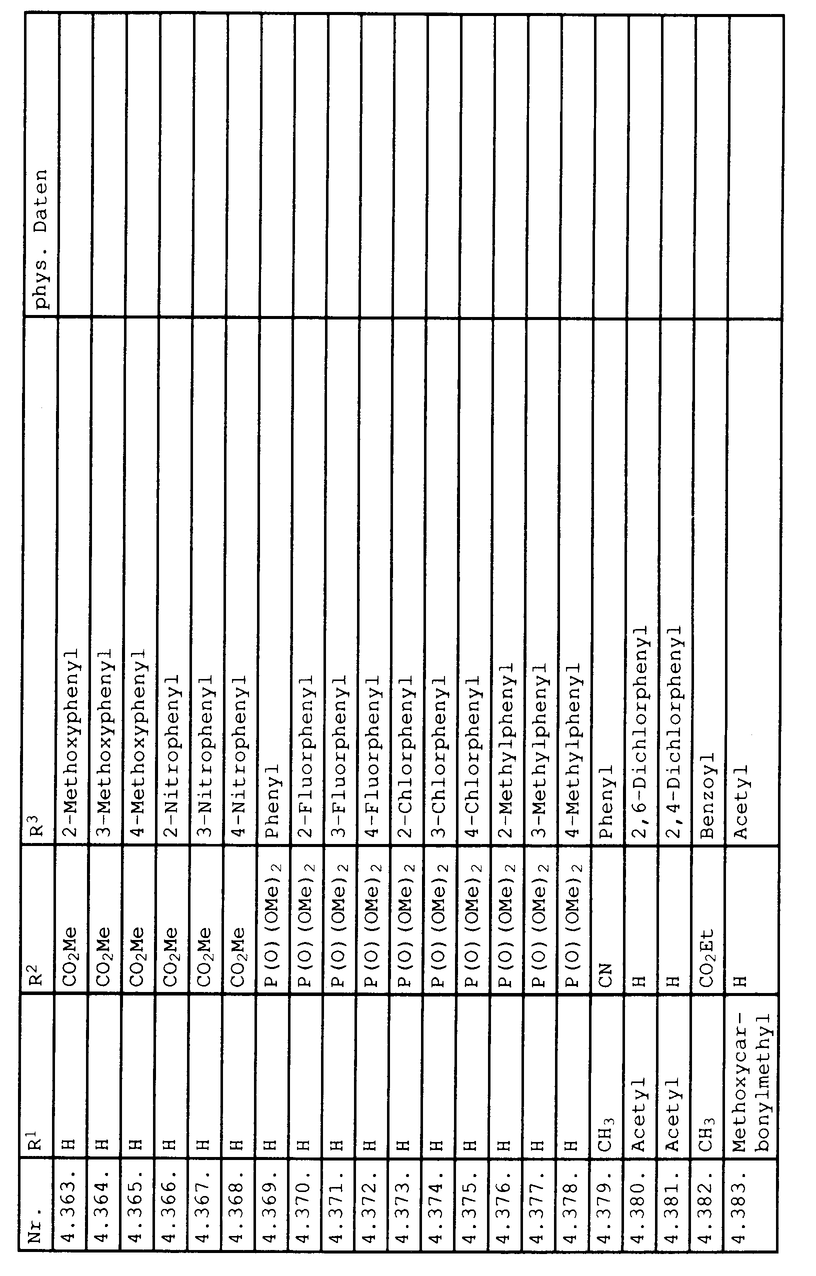

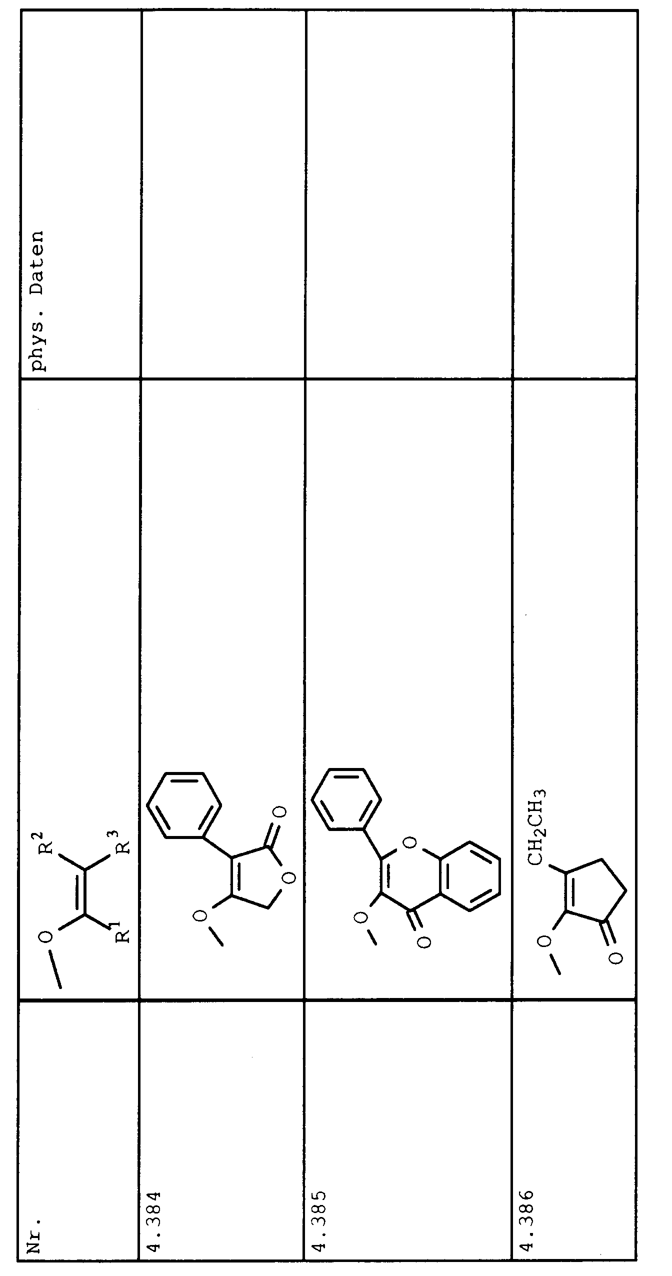

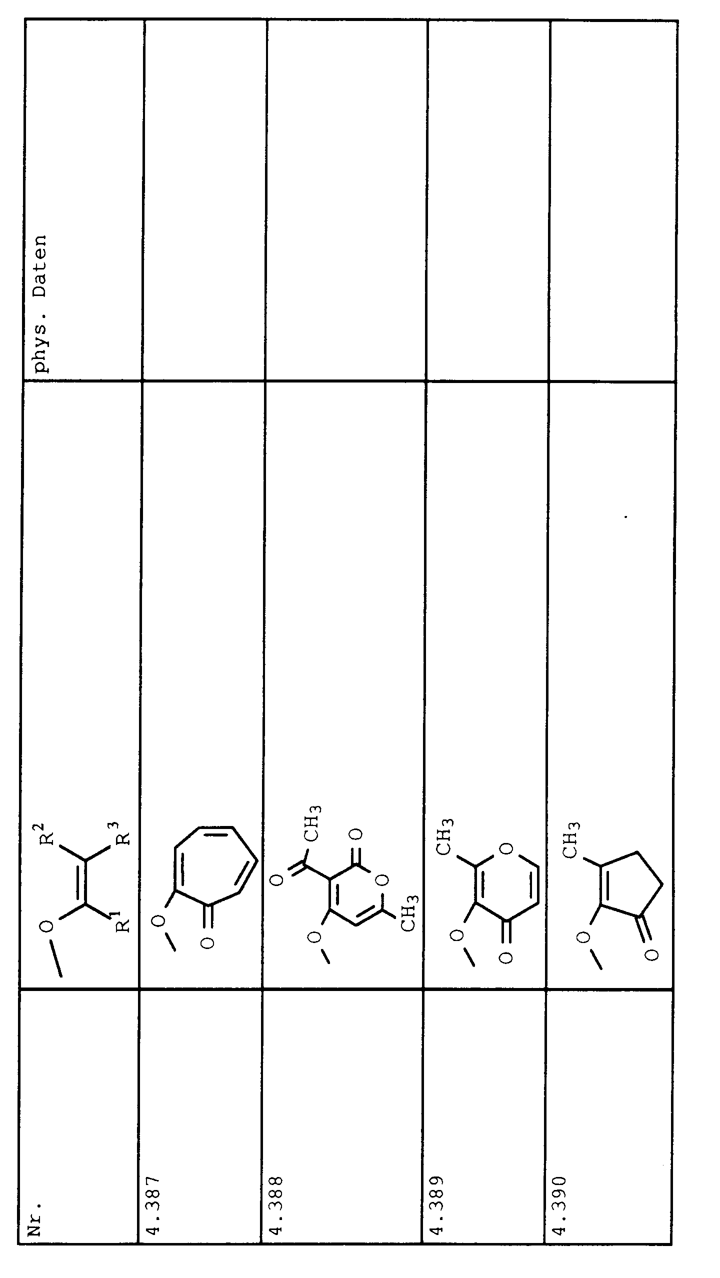

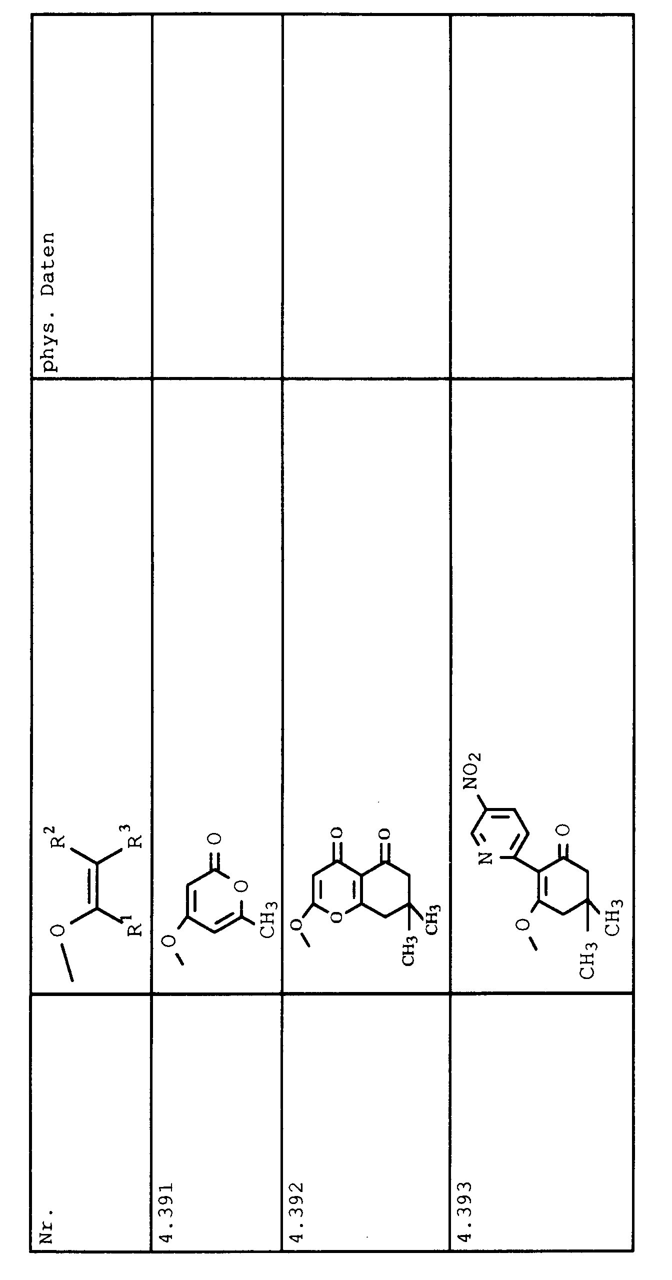

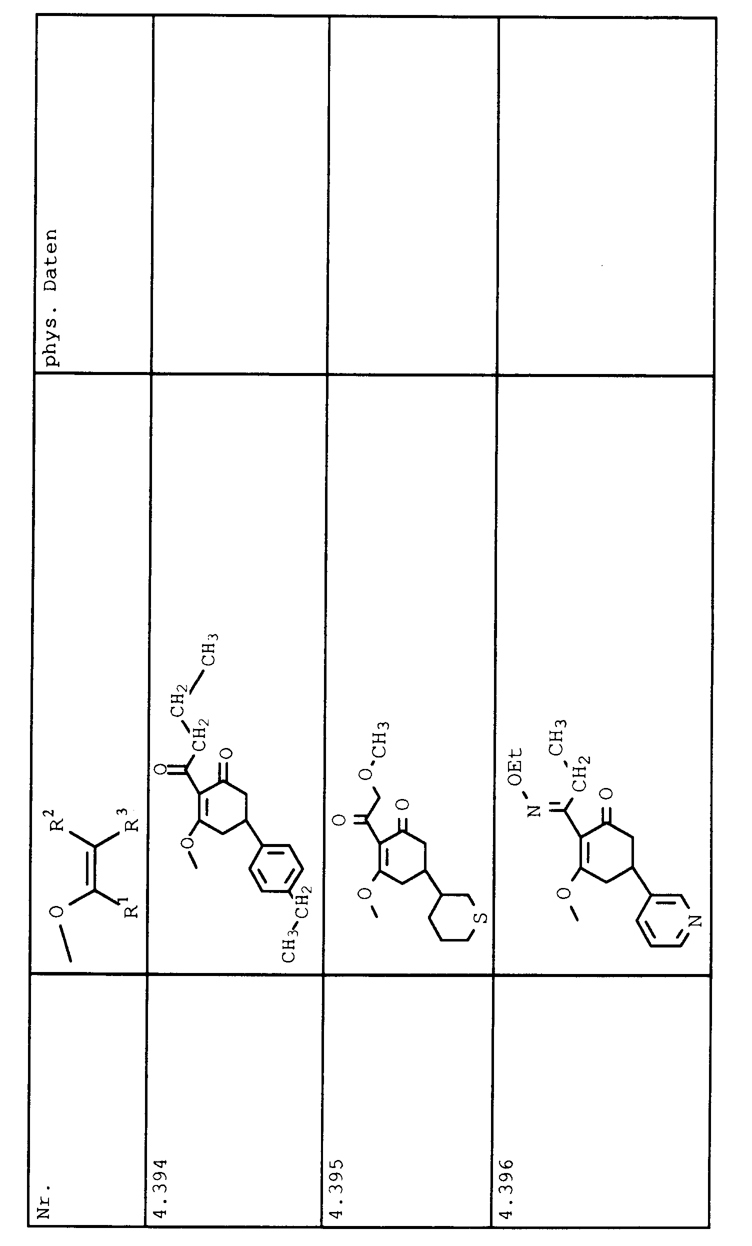

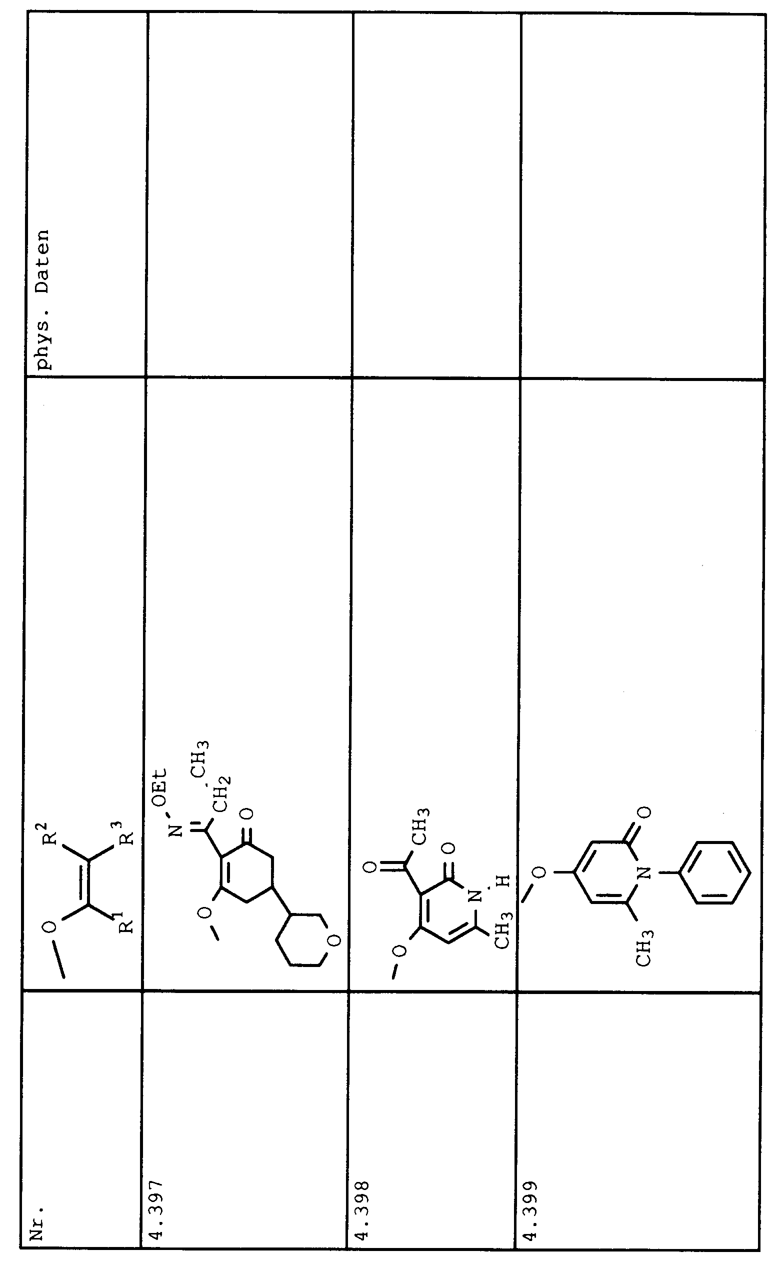

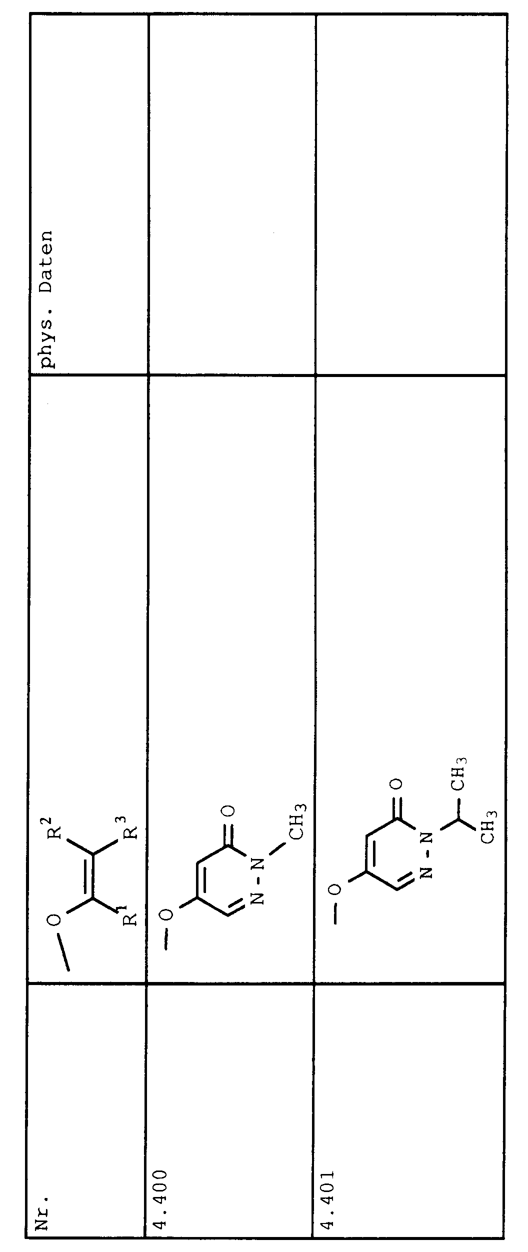

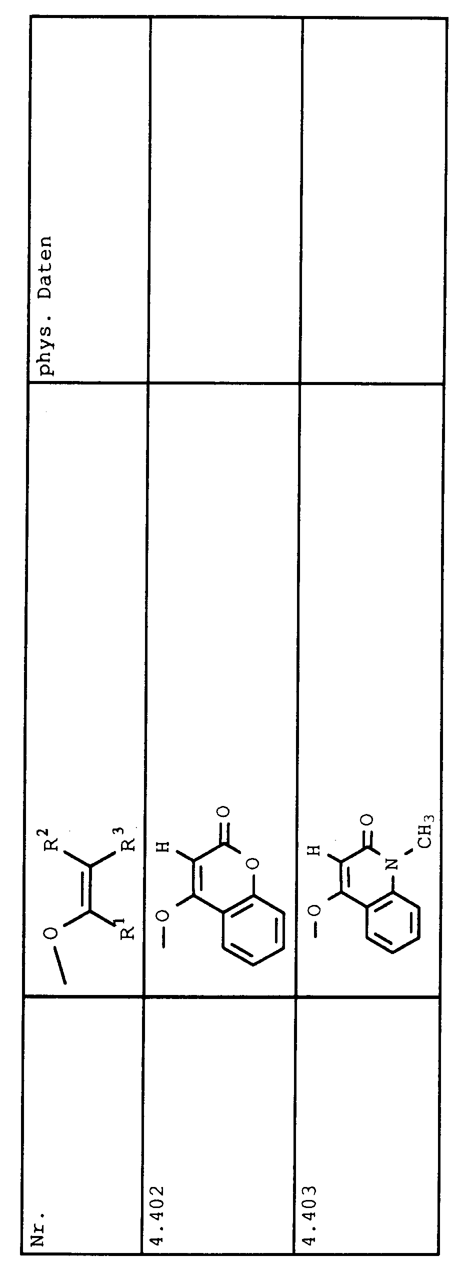

Die neuen Verbindungen eignen sich als Fungizide und Insektizide.

Die erfindungsgemäßen fungiziden und insektiziden Verbindungen bzw. die sie enthaltenden Mittel können beispielsweise in Form von direkt versprühbaren Lösungen, Pulvern, Suspensionen, auch hochprozentigen wäßrigen, öligen oder sonstigen Suspensionen oder Dispersionen, Emulsionen, Öldispersionen, Pasten, Stäubemitteln, Streumitteln oder Granulaten durch Versprühen, Vernebeln, Verstäuben, Verstreuen oder Gießen angewendet werden. Die Anwendungformen richten sich nach den Verwendungszwecken; sie sollten in jedem Fall möglichst die feinste Verteilung der erfindungsgemäßen Wirkstoffe gewährleisten.

Normalerweise werden die Pflanzen mit den Wirkstoffen besprüht oder bestäubt oder die Samen der Pflanzen mit den Wirkstoffen behandelt.

Die Formulierungen werden in bekannter Weise hergestellt, z.B. durch Verstrecken des Wirkstoffs mit Lösungsmitteln und/oder Trägerstoffen, gewünschtenfalls unter Verwendung von Emulgiermitteln und Dispergiermitteln, wobei im Falle von Wasser als Verdünnungsmittel auch andere organische Lösungsmittel als Hilfslösungsmittel verwendet werden können. Als Hilfsstoffe kommen dafür im wesentlichen in Betracht: Lösungsmittel wie Aromaten (z.B. Xylol), chlorierte Aromaten (z.B. Chlorbenzole), Paraffine (z.B. Erdölfraktionen), Alkohole (z.B. Methanol, Butanol), Ketone (z.B. Cyclohexanon), Amine (z.B. Ethanolamin, Dimethylformamid) und Wasser; Trägerstoffe wie natürliche Gesteinsmehle (z.B. Kaoline, Tonerden, Talkum, Kreide) und synthetische Gesteinsmehle (z.B. hochdisperse Kieselsäure, Silikate); Emulgiermittel wie nichtionogene und anionische Emulgatoren (z.B. Polyoxyethylen-Fettalkohol-Ether, Alkylsulfonate und Arylsulfonate) und Dispergiermittel wie Ligninsulfitablaugen und Methylcellulose.

Als oberflächenaktive Stoffe kommen die Alkali-, Erdalkali-, Ammoniumsalze von aromatischen Sulfonsäuren, z.B. Lignin-, Phenol-, Naphthalin- und Dibutylnaphthalinsulfonsäure, sowie von Fettsäuren, Alkyl- und Alkylarylsulfonaten, Alkyl-, Laurylether- und Fettalkoholsulfaten, sowie Salze sulfatierter Hexa-, Hepta- und Octadecanolen, sowie von Fettalkoholglykolether, Kondensationsprodukte von sulfoniertem Naphthalin und seiner Derivate mit Formaldehyd, Kondensationsprodukte des Naphthalins bzw. der Naphthalinsulfonsäuren mit Phenol und Formaldehyd, Polyoxyethylenoctylphenolether, ethoxyliertes Isooctyl-, Octyl- oder Nonylphenol, Alkylphenol-, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether oder Polyoxypropylen, Laurylalkoholpolyglykoletheracetat, Sorbitester, Lignin-Sulfitablaugen oder Methylcellulose in Betracht.

Pulver-, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate können durch Bindung der Wirkstoffe an feste Trägerstoffe hergestellt werden. Feste Trägerstoffe sind Mineralerden wie Silicagel, Kieselsäuren, Kieselgele, Silikate, Talkum, Kaolin, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie Ammoniumsulfat, Ammoniumphosphat, Ammoniumnitrat, Harnstoffe und pflanzliche Produkte, wie Getreidemehl, Baumrinden-, Holz- und Nußschalenmehl, Cellulosepulver oder andere feste Trägerstoffe.

Beispiele für solche Zubereitungen sind:
I. eine Lösung aus 90 Gew.-Teilen der Verbindung Nr. 1.52 und 10 Gew.-Teilen N-Methyl-α-pyrrolidon, die zur Anwendung in Form kleinster Tropfen geeignet ist;
II. eine Mischung aus 20 Gew.-Teilen der Verbindung Nr. 1.56, 80 Gew.Teilen Xylol, 10 Gew.-Teilen des Anlagerungsproduktes von 8 bis 10 Mol Ethylenoxid an 1 Mol Ölsäure-N-monoethanolamid, 5 Gew.Teilen Calciumsalz der Dodecylbenzolsulfonsäure, 5 Gew.-Teilen des Anlagerungsproduktes von 40 Mol Ethylenoxid an 1 Mol Ricinusöl; durch feines Verteilen der Lösung in Wasser erhält man eine Dispersion.
III. eine wäßrige Dispersion aus 20 Gew.-Teilen der Verbindung Nr. 1.98, 40 Gew.-Teilen Cyclohexanon, 30 Gew.-Teilen Isobutanol, 20 Gew.-Teilen des Anlagerungsproduktes von 40 mol Ethylenoxid an 1 mol Ricinusöl;
IV. eine wäßrige Dispersion aus 20 Gew.-Teilen der Verbindung Nr. 1.100, 25 Gew.-Teilen Cyclohexanol, 65 Gew.-Teilen einer Mineralölfraktion vom Siedepunkt 210 bis 280°C und 10 Gew. -Teilen des Anlagerungsproduktes von 40 mol Ethylenoxid an 1 mol Ricinusöl;
V. eine in einer Hammermühle vermahlene Mischung aus 80 Gew.-Teilen der Verbindung Nr. 2.52, 3 Gew.-Teilen des Natriumsalzes der Diisobutylnaphtalin-α-sulfonsäure, 10 Gew.-Teilen des Natriumsalzes einer Ligninsulfonsäure aus einer Sulfitablauge und 7 Gew.-Teilen pulverförmigem Kieselsäuregel; durch feines Verteilen der Mischung in Wasser erhält man eine Spritzbrühe;
VI. eine innige Mischung aus 3 Gew.-Teilen der Verbindung Nr. 2.100 und 97 Gew.-Teilen feinteiligem Kaolin; dieses Stäubemittel enthält 3 Gew.-% Wirkstoff;
VII. eine innige Mischung aus 30 Gew.-Teilen der Verbindung Nr. 1.52, 92 Gew.-Teilen pulverförmigem Kieselsäuregel und 8 Gew.-Teilen Paraffinöl, das auf die Oberfläche dieses Kieselsäuregels gesprüht wurde; diese Aufbereitung gibt dem Wirkstoff eine gute Haftfähigkeit;
VIII. eine stabile wäßrige Dispersion aus 40 Gew.-Teilen der Verbindung Nr. 1.56, 10 Gew.-Teilen des Natriumsalzes eines Phenolsulfonsäure-harnstoffformaldehyd-Kondensates, 2 Gew.-Teilen Kieselgel und 48 Gew.-Teilen Wasser, die weiter verdünnt werden kann;
IX. eine stabile ölige Dispersion aus 20 Gew.-Teilen der Verbindung Nr. 1.98, 2 Gew.-Teilen des Calciumsalzes der Dodecylbenzolsulfonsäure, 8 Gew.-Teilen Fettalkohol-polyglykolether, 20 Gew.-Teilen des Natriumsalzes eines Phenolsulfonsäure-harnstoff-formaldehydKondensates und 68 Gew.-Teilen eines paraffinischen Mineralöls.

Die Verbindungen zeichnen sich durch eine hervorragende Wirksamkeit gegen ein breites Spektrum von pflanzenpathogenen Pilzen, insbesondere aus der Klasse der Ascomyceten und Basidiomyceten, aus. Sie sind zum Teil systemisch wirksam und können als Blatt- und Bodenfungizide eingesetzt werden.

Besondere Bedeutung haben sie für die Bekämpfung einer Vielzahl von Pilzen an verschiedenen Kulturpflanzen wie Weizen, Roggen, Gerste, Hafer, Reis, Mais, Gras, Baumwolle, Soja, Kaffee, Zuckerrohr, Wein, Obst- und Zierpflanzen und Gemüsepflanzen wie Gurken, Bohnen und Kürbisgewächsen, sowie an den Samen dieser Pflanzen.

Die Verbindungen werden angewendet, indem man die Pilze oder die vor Pilzbefall zu schützenden Saatgüter, Pflanzen, Materialien oder den Erdboden mit einer fungizid wirksamen Menge der Wirkstoffe behandelt.

Die Anwendung erfolgt vor oder nach der Infektion der Materialien, Pflanzen oder Samen durch die Pilze.

Speziell eignen sich die Verbindungen I zur Bekämpfung folgender Pflanzenkrankheiten:
Erysiphe graminis (echter Mehltau) in Getreide,
Erysiphe cichoracearum und Sphaerotheca fuliginea an Kürbisgewächsen,
Podosphaera leucotricha an Äpfeln,
Uncinula necator an Reben,
Puccinia-Arten an Getreide,
Rhizoctonia-Arten an Baumwolle und Rasen,
Ustilago-Arten an Getreide und Zuckerrohr,
Venturia inaequalis (Schorf) an Äpfeln,
Helminthosporium-Arten an Getreide,
Septoria nodorum an Weizen,
Botrytis cinerea (Grauschimmel) an Erdbeeren, Reben,
Cercospora arachidicola an Erdnüssen,
Pseudocercosporella herpotrichoides an Weizen, Gerste,
Pyricularia oryzae an Reis,
Phytophthora infestans an Kartoffeln und Tomaten,
Fusarium- und Verticillium-Arten an verschiedenen Pflanzen,
Plasmopara viticola an Reben,
Alternaria-Arten an Gemüse und Obst.

Die neuen Verbindungen können auch im Materialschutz (Holzschutz) eingesetzt werden, z.B. gegen Paecilomyces variotii.

Die fungiziden Mittel enthalten im allgemeinen zwischen 0,1 und 95, vorzugsweise zwischen 0,5 und 90 Gew.% Wirkstoff.

Die Aufwandmengen liegen je nach Art des gewünschten Effektes zwischen 0,02 und 3 kg Wirkstoff pro ha.

Bei der Saatgutbehandlung werden im allgemeinen Wirkstoffmengen von 0,001 bis 50 g, vorzugsweise 0,01 bis 10 g je Kilogramm Saatgut benötigt.

Die erfindungsgemäßen Mittel können in der Anwendungsform als Fungizide auch zusammen mit anderen Wirkstoffen vorliegen, der z.B. mit Herbiziden, Insektiziden, Wachstumsregulatoren, Fungiziden oder auch mit Düngemitteln.

Beim Vermischen mit Fungiziden erhält man dabei in vielen Fällen eine Vergrößerung des fungiziden Wirkungsspektrums.

### Anwendungsbeispiele

Als Vergleichswirkstoffe wurden 2-[2-(Phenyloxymethylen)-phenyl]-3-methoxy-acrylsäuremethylester (A) - bekannt aus EP 178 826 - und 2-[2-(Phenyloxymethylen)-phenyl]-glyoxylsäuremethylester-O-methyloximether (B) - bekannt aus EP 253 213 - verwendet.

### Anwendungsbeispiel

### Wirksamkeit gegen Rebenperonospora

Blätter von Topfreben der Sorte "Müller Thurgau" wurden mit wäßriger Spritzbrühe, die 80 % Wirkstoff und 20 % Emulgiermittel in der Trockensubstanz enthielt, besprüht. Um die Wirkungsdauer der Wirkstoffe beurteilen zu können, wurden die Pflanzen nach dem Antrocknen des Spritzbelages 8 Tage im Gewächshaus aufgestellt. Erst dann wurden die Blätter mit einer Zoosporenaufschwemmung von Plasmopara viticola (Rebenperonospora) infiziert. Danach wurden die Reben zunächst für 48 Stunden in einer wasserdampfgesättigten Kammer bei 24°C und anschließend für 5 Tage in einem Gewächshaus bei Temperaturen zwischen 20 und 30°C aufgestellt. Nach dieser Zeit wurden die Pflanzen zur Beschleunigung des Sporangienträgerausbruchs abermals für 16 Stunden in der feuchten Kammer aufgestellt. Dann erfolgte die Beurteilung des Ausmaßes des Pilzausbruchs auf den Blattunterseiten.

Das Ergebnis zeigt, daß die Wirkstoffe 1.52, 1.56, 1.98, 1.100, 2.52 und 2.100 bei der Anwendung als 15 ppm Wirkstoff enthaltende Spritzbrühe eine bessere fungizide Wirkung zeigen (95 %) als die bekannten Vergleichswirkstoffe A (30 %) und B (65 %).

## Patentansprüche

1. Benzylenolether-Derivate der allgemeinen Formel I 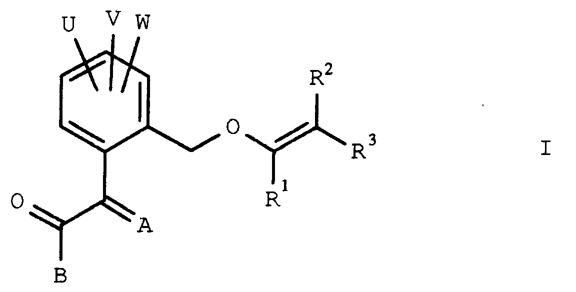 in der
B
OH, C₁-C₄-Alkoxy, C₁-C₄-Alkylthio und C₁-C₄-Alkylamino bedeutet,
U, V, W
gleich oder verschieden sind und Wasserstoff, Halogen, C₁-C₄-Alkyl und C₁-C₄-Alkoxy bedeuten, und
a) für den Fall, daß
A
CH₂, CHCl, CH-O-C₁-C₄-Alkyl, CH-S-C₁-C₄-Alkyl, N-O-C₁-C₄-Alkyl bedeutet, und
R¹, R², R³
gleich oder verschieden sind und Wasserstoff, Cyano, Halogen, NR⁴R⁵, CO₂R⁴, CONR⁴R⁵, COR⁴, S(O)ₙR⁴ mit n = 0, 1 und 2, PO(OR⁴)₂, ggf. verzweigtes C₁-C₁₀-Alkyl, C₁-C₄-Halogenalkyl, C₃-C₆-Cycloalkyl, C₃-C₆-Halogencycloalkyl, C₃-C₆-Cycloalkyl-C₁-C₄-Alkyl, C₁-C₄-Alkoxy-C₁-C₄-Alkyl, C₁-C₄-Alkylthio-C₁-C₄-Alkyl, Arylthio-C₁-C₄-Alkyl, C₂-C₆-Alkenyl, C₂-C₅-Halogenalkenyl, C₃-C₆-Cycloalkenyl, C₃-C₆-Halogencycloalkenyl, C₂-C₆-Alkinyl, C₁-C₆-Alkoxy, C₁-C₆-Halogenalkoxy, C₁-C₄-Alkylthio, Benzylthio, C₁-C₄-Alkylcarbonyl, ggf. subst. Phenylcarbonyl, ggf. subst. Benzylcarbonyl, C₁-C₄Alkoxycarbonyl, ggf. subst. Phenoxycarbonyl, ggf. subst. Benzyloxycarbonyl, ggf. subst. Aryl, ggf. subst. Aryloxy, ggf. subst. Arylthio, ggf. subst. Aryl-C₁-C₄-alkyl, ggf. subst. Aryl-C₂-C₄-alkenyl, ggf. subst. Aryloxy-C₁-C₄-alkyl, ggf. subst. Arylthio-C₁-C₄-alkyl, ggf. subst. Hetaryl, ggf. subst. Hetaryloxy, ggf. subst. Hetarylthio, ggf. subst. Heteroaryl-C₁-C₄-alkyl, ggf. subst. Hetaryl-C₂-C₄-alkenyl, ggf. subst. Hetaryloxy-C₁-C₄-alkyl, ggf. subst. Heterocyclyl, ggf. subst. Heterocyclyloxy bedeuten,
wobei "ggf. subst." neben Wasserstoff die Reste Halogen, Cyano, Nitro, C₁-C₄-Alkyl, C₁-C₄-Alkoxy, C₁-C₄-Halogenalkyl, C₁-C₄-Halogenalkoxy, C₁-C₁₀-Alkoximino-C₁-C₂-alkyl, Aryl, Aryloxy, Benzyloxy, Hetaryl, Hetaryloxy, C₃-C₆-Cycloalkyl, Heterocyclyl, Heterocyclyloxy bedeuten,
und die Reste
R⁴, R⁵
gleich oder verschieden sind und Wasserstoff und C₁-C₄-Alkyl bedeuten,
R¹ und R³ zusammen, sowie auch R² und R³ zusammen jeweils einen carbocyclischen oder einen heterocyclischen Ring bilden können, der seinerseits benzanneliert sein kann und durch die unter "ggf. subst." genannten Rest substituiert sein kann,
und für den Fall, daß
b) A CH-C₁-C₄-Alkyl bedeutet,
R¹, R², R³
gleich oder verschieden sind und Wasserstoff, Cyano, Halogen, NR⁴R⁵, CO₂R⁴, CONR⁴R⁵, COR⁴, S(O)ₙR⁴ mit n = 0, 1 und 2, PO(OR⁴)₂, ggf. verzweigtes C₁-C₁₀-Alkyl, C₁-C₄-Halogenalkyl, C₃-C₆-Cycloalkyl, C₃-C₆-Halogencycloalkyl, C₃-C₆-Cycloalkyl-C₁-C₄-Alkyl, C₁-C₄-Alkoxy-C₁-C₄-Alkyl, C₁-C₄-Alkylthio-C₁-C₄-Alkyl, Arylthio-C₁-C₄-Alkyl, C₂-C₆-Alkenyl, C₂-C₅-Halogenalkenyl, C₃-C₆-Cycloalkenyl, C₃-C₆-Halogencycloalkenyl, C₂-C₆-Alkinyl, C₁-C₆-Alkoxy, C₁-C₆-Halogenalkoxy, C₁-C₄-Alkylthio, Benzylthio, C₁-C₄-Alkylcarbonyl, ggf. subst. Phenylcarbonyl, ggf. subst. Benzylcarbonyl, C₁-C₄Alkoxycarbonyl, ggf. subst. Phenoxycarbonyl, ggf. subst. Benzyloxycarbonyl, ggf. subst. Aryl, ggf. subst. Aryloxy, ggf. subst. Arylthio, ggf. subst. Aryl-C₁-C₄-alkyl, ggf. subst. Aryl-C₂-C₄-alkenyl, ggf. subst. Aryloxy-C₁-C₄-alkyl, ggf. subst. Arylthio-C₁-C₄-alkyl, ggf. subst. Hetaryl, ggf. subst. Hetaryloxy, ggf. subst. Hetarylthio, ggf. subst. Heteroaryl-C₁-C₄-alkyl, ggf. subst. Hetaryl-C₂-C₄-alkenyl, ggf. subst. Hetaryloxy-C₁-C₄-alkyl, ggf. subst. Heterocyclyl, ggf. subst. Heterocyclyloxy bedeuten,
wobei "ggf. subst." neben Wasserstoff die Reste Halogen, Cyano, Nitro, C₁-C₄-Alkyl, C₁-C₄-Alkoxy, C₁-C₄-Halogenalkyl, C₁-C₄-Halogenalkoxy, C₁-C₁₀-Alkoximino-C₁-C₂-alkyl, Aryl, Aryloxy, Benzyloxy, Hetaryl, Hetaryloxy, C₃-C₆-Cycloalkyl, Heterocyclyl, Heterocyclyloxy bedeuten,
und die Reste
R⁴, R⁵
gleich oder verschieden sind und Wasserstoff und C₁-C₄-Alkyl bedeuten,
R¹ und R³ zusammen, sowie auch R² und R³ zusammen jeweils einen der folgenden Ringsysteme A - F bilden können
für die Gruppierung 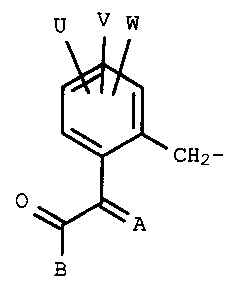 steht im folgenden das Symbol 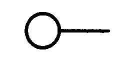 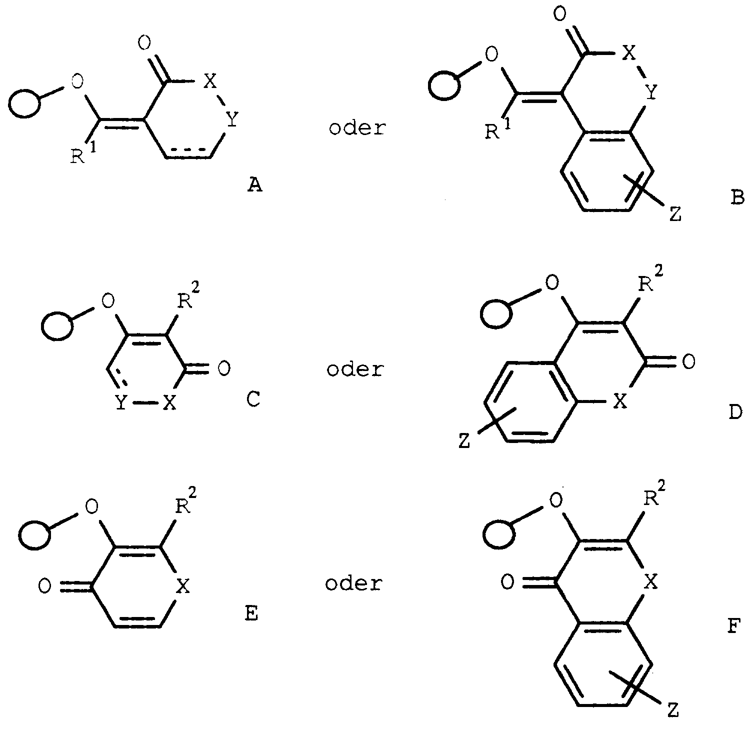 wobei
X
CH₂, O, NR⁴ bedeutet,
Y
CH, (CH₂)ₙ, mit n = 0, 1 und 2 bedeutet,
Z
Wasserstoff, Halogen, C₁-C₄-Alkyl und C₁-C₄-Alkoxy und
R⁴
Wasserstoff und C₁-C₄-Alkyl und ···· eine Einfachbindung oder eine Doppelbindung bedeutet.

2. Benzylenolether der Formel II 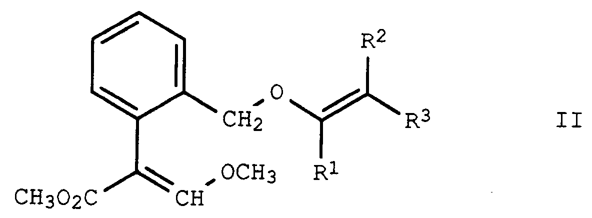 wobei R¹, R² und R³ die in Anspruch 1 genannten Reste bedeuten.

3. Benzylenolether der Formel III 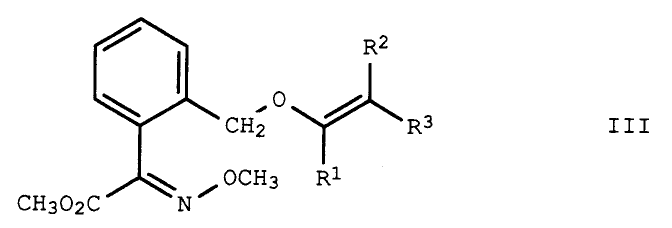 wobei R¹, R² und R³ die in Anspruch 1 genannten Reste bedeuten.

4. Benzylenolether der Formel IV 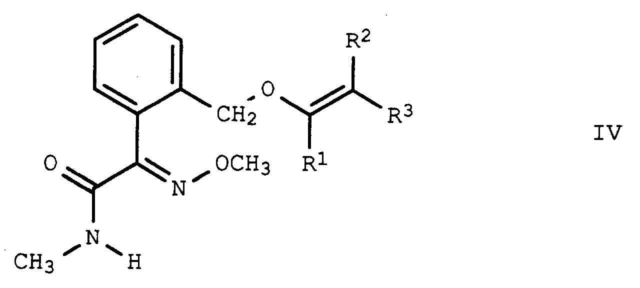 wobei R¹, R² und R³ die in Anspruch 1 genannten Reste bedeuten.

5. Verfahren zur Herstellung von Benzylenolethern der Formel I gemäß Anspruch 1, dadurch gekennzeichnet, daß man ein Benzyldervat der Formel 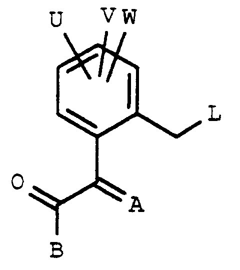 in der U, V, W, A und B die in Anspruch 1 genannte Bedeutung haben und L eine Abgangsgruppe bedeutet, mit einem Enol der Formel 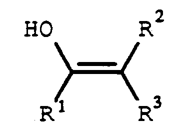 in der R¹, R² und R³ die in Anspruch 1 genannte Bedeutung haben, umsetzt.

6. Fungizid, enthaltend einen inerten Trägerstoff und eine fungizid wirksame Menge eines Benzylenolethers der Formel I gemäß Anspruch 1.

7. Verfahren zur Bekämpfung von Pilzen, dadurch gekennzeichnet, daß man die Pilze oder die von Pilzbefall bedrohten Materialien, Pflanzen, Saatgut oder den Erdboden mit einer Fungizid wirksamen Menge einer Verbindung der Formel I gemäß Anspruch 1 behandelt.

8. Verbindung der Formel I gemäß Anspruch 1, dadurch gekennzeichnet, daß A CHOCH₃, B OCH₃, U, V, W und R¹ Wasserstoff, R² Cyano und R³ 2-Fluorphenyl bedeuten.

9. Verbindung der Formel I gemäß Anspruch 1, dadurch gekennzeichnet, daß A CHOCH₃, B OCH₃, U, V, W und R¹ Wasserstoff, R² Cyano und R³ 4-Methylphenyl bedeuten.

10. Verbindung der Formel I gemäß Anspruch 1, dadurch gekennzeichnet, daß A NOCH₃, B OCH₃, U, V, W und R¹ Wasserstoff, R² Cyano und R³ 2-Fluorphenyl bedeuten.

## Claims

1. A benzyl enol ether derivative of the general formula I 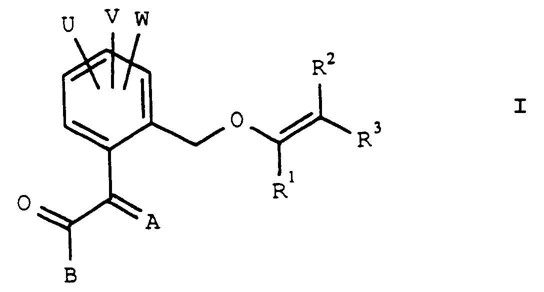 where
B is
OH, C₁-C₄-alkoxy, C₁-C₄-alkylthio and C₁-C₄-alkylamino,
U, V and W
are identical or different and are hydrogen, halogen, C₁-C₄-alkyl or C₁-C₄-alkoxy, and
for the case that
a) A is
CH₂, CHCl, CH-O-C₁-C₄-alkyl, CH-S-C₁-C₄-alkyl or N-O-C₁-C₄-alkyl,
R¹, R² and R³
are identical or different and are hydrogen, cyano, halogen, NR⁴R⁵, CO₂R⁴, CONR⁴R⁵, COR⁴, S(O)ₙR⁴ where n = 0, 1 or 2, PO(OR⁴)₂, unbranched or branched C₁-C₁₀-alkyl, C₁-C₄-haloalkyl, C₃-C₆-cycloalkyl, C₃-C₆-halocycloalkyl, C₃-C₆-cycloalkyl-C₁-C₄-alkyl, C₁-C₄-alkoxy-C₁-C₄-alkyl, C₁-C₄-alkylthio-C₁-C₄-alkyl, arylthio-C₁-C₄-alkyl, C₂-C₆-alkenyl, C₂-C₅-haloalkenyl, C₃-C₆-cycloalkenyl, C₃-C₆-halocycloalkenyl, C₂-C₆-alkynyl, C₁-C₆-alkoxy, C₁-C₆-haloalkoxy, C₁-C₄-alkylthio, benzylthio, C₁-C₄-alkylcarbonyl, unsubstituted or substituted phenylcarbonyl, unsubstituted or substituted benzylcarbonyl, C₁-C₄-alkoxycarbonyl, unsubstituted or substituted phenoxycarbonyl, unsubstituted or substituted benzyloxycarbonyl, unsubstituted or substituted aryl, unsubstituted or substituted aryloxy, unsubstituted or substituted arylthio, unsubstituted or substituted aryl-C₁-C₄-alkyl, unsubstituted or substituted aryl-C₂-C₄-alkenyl, unsubstituted or substituted aryloxy-C₁-C₄-alkyl, unsubstituted or substituted arylthio-C₁-C₄-alkyl, unsubstituted or substituted hetaryl, unsubstituted or substituted hetaryloxy, unsubstituted or substituted hetarylthio, unsubstituted or substituted heteroaryl-C₁-C₄-alkyl, unsubstituted or substituted hetaryl-C₂-C₄-alkenyl, unsubstituted or substituted hetaryloxy-C₁-C₄-alkyl, unsubstituted or substituted heterocyclyl, unsubstituted or substituted heterocyclyloxy,
where unsubstituted or substituted, in addition to hydrogen, includes the radicals halogen, cyano, nitro, C₁-C₄-alkyl, C₁-C₄-alkoxy, C₁-C₄-haloalkyl, C₁-C₄-haloalkoxy, C₁-C₁₀-alkoximino-C₁-C₂-alkyl, aryl, aryloxy, benzyloxy, hetaryl, hetaryloxy, C₃-C₆-cycloalkyl, heterocyclyl, heterocyclyloxy,
and the radicals
R⁴ and R⁵
are identical or different and are hydrogen or C₁-C₄-alkyl,
R¹ and R³ together, and also R² and R³ together, in each case can form a carbocyclic or a heterocyclic ring which can in turn be benzo-fused and can be substituted by the radical mentioned under unsubstituted or substituted,
and for the case that
b) A is CH-C₁-C₄-alkyl,
R¹, R² and R³
are identical or different and are hydrogen, cyano, halogen, NR⁴R⁵, CO₂R⁴, CONR⁴R⁵, COR⁴, S(O)ₙR⁴ where n = 0, 1 or 2, PO(OR⁴)₂, unbranched or branched C₁-C₁₀-alkyl, C₁-C₄-haloalkyl, C₃-C₆-cycloalkyl, C₃-C₆-halocycloalkyl, C₃-C₆-cycloalkyl-C₁-C₄-alkyl, C₁-C₄-alkoxy-C₁-C₄-alkyl, C₁-C₄-alkylthio-C₁-C₄-alkyl, arylthio-C₁-C₄-alkyl, C₂-C₆-alkenyl, C₂-C₅-haloalkenyl, C₃-C₆-cycloalkenyl, C₃-C₆-halocycloalkenyl, C₂-C₆-alkynyl, C₁-C₆-alkoxy, C₁-C₆-haloalkoxy, C₁-C₄-alkylthio, benzylthio, C₁-C₄-alkylcarbonyl, unsubstituted or substituted phenylcarbonyl, unsubstituted or substituted benzylcarbonyl, C₁-C₄-alkoxycarbonyl, unsubstituted or substituted phenoxycarbonyl, unsubstituted or substituted benzyloxycarbonyl, unsubstituted or substituted aryl, unsubstituted or substituted aryloxy, unsubstituted or substituted arylthio, unsubstituted or substituted aryl-C₁-C₄-alkyl, unsubstituted or substituted aryl-C₂-C₄-alkenyl, unsubstituted or substituted aryloxy-C₁-C₄-alkyl, unsubstituted or substituted arylthio-C₁-C₄-alkyl, unsubstituted or substituted hetaryl, unsubstituted or substituted hetaryloxy, unsubstituted or substituted hetarylthio, unsubstituted or substituted heteroaryl-C₁-C₄-alkyl, unsubstituted or substituted hetaryl-C₂-C₄-alkenyl, unsubstituted or substituted hetaryloxy-C₁-C₄-alkyl, unsubstituted or substituted heterocyclyl, unsubstituted or substituted heterocyclyloxy,
where unsubstituted or substituted, in addition to hydrogen, includes the radicals halogen, cyano, nitro, C₁-C₄-alkyl, C₁-C₄-alkoxy, C₁-C₄-haloalkyl, C₁-C₄-haloalkoxy, C₁-C₁₀-alkoximino-C₁-C₂-alkyl, aryl, aryloxy, benzyloxy, hetaryl, hetaryloxy, C₃-C₆-cycloalkyl, heterocyclyl, heterocyclyloxy,
and the radicals
R⁴ and R⁵
are identical or different and are hydrogen or C₁-C₄-alkyl,
R¹ and R³ together, and also R² and R³ together, in each case can form one of the following ring systems A - F
the symbol 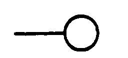 is the group 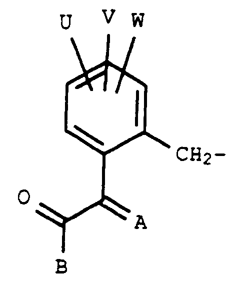 in the following 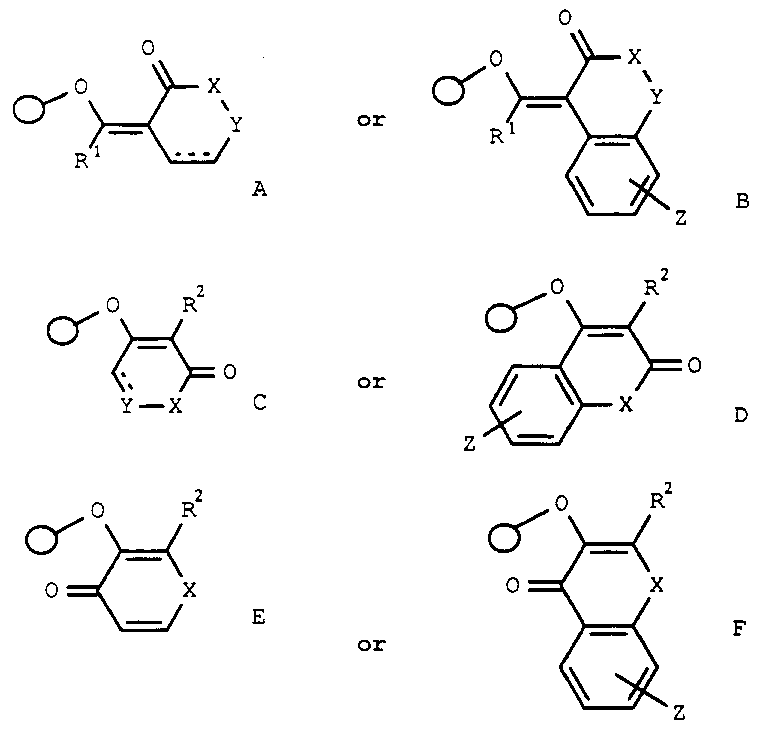 where
X is
CH₂, O, NR⁴,
Y is
CH, (CH₂)ₙ, where n = 0, 1 or 2,
Z is
hydrogen, halogen, C₁-C₄-alkyl or C₁-C₄-alkoxy and R⁴ is
hydrogen or C₁-C₄-alkyl and .... is a single bond or a double bond.

2. A benzyl enol ether of the formula II 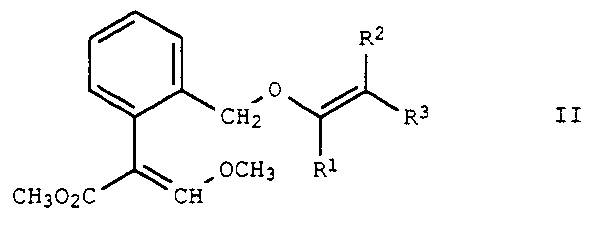 where R¹, R² and R³ are the radicals mentioned in claim 1.

3. A benzyl enol ether of the formula III 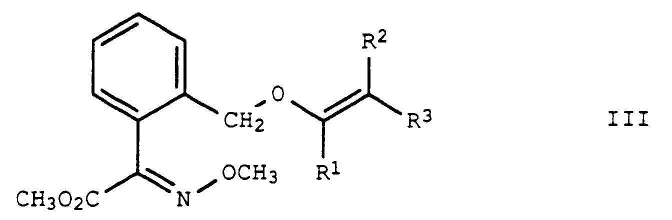 where R¹, R² and R³ are the radicals mentioned in claim 1.

4. A benzyl enol ether of the formula IV 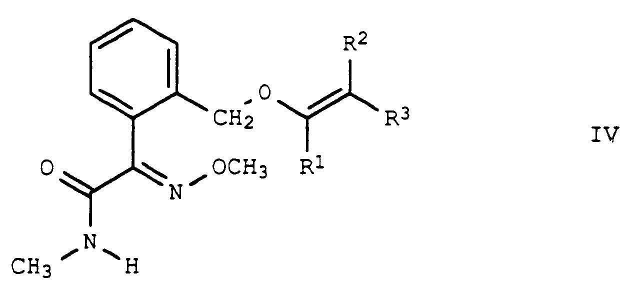 where R¹, R² and R³ are the radicals mentioned in claim 1.

5. A process for preparing a benzyl enol ether of the formula I as claimed in claim 1, which comprises reacting a benzyl derivative of the formula 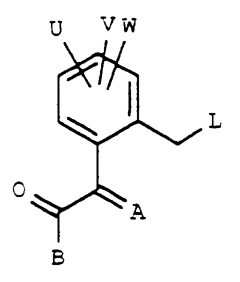 in which U, V, W, A and B have the meaning mentioned in claim 1 and L is a leaving group, with an enol of the formula 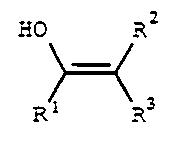 in which R¹, R² and R³ have the meaning mentioned in claim 1.

6. A fungicide containing an inert carrier and a fungicidally active amount of a benzyl enol ether of the formula I as claimed in claim 1.

7. A method for controlling fungi, which comprises treating the fungi or the materials, plants, seeds or the ground threatened by fungal attack with a fungicidally active amount of a compound of the formula I as claimed in claim 1.

8. A compound of the formula I as claimed in claim 1, wherein A is CHOCH₃, B is OCH₃, U, V, W and R¹ are hydrogen, R² is cyano and R³ is 2-fluorophenyl.

9. A compound of the formula I as claimed in claim 1, wherein A is CHOCH₃, B is OCH₃, U, V, W and R¹ are hydrogen, R² is cyano and R³ is 4-methylphenyl.

10. A compound of the formula I as claimed in claim 1, wherein A is NOCH₃, B is OCH₃, U, V, W and R¹ are hydrogen, R² is cyano and R³ is 2-fluorophenyl.

## Revendications

1. Dérivé de benzylénoléther de formule générale I 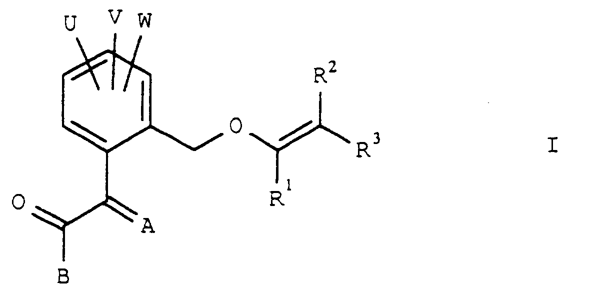 dans laquelle
B représente
un groupe OH, alcoxy en C1-C4, alkylthio en C1-C4 et alkylamino en C1-C4,
U, V, W sont
identiques ou différents et représentent un atome d'hydrogène, un atome d'halogène, un groupe alkyle en C1-C4 et alcoxy en C1-C4, et
a) dans le cas où
A représente
CH₂, CHCl, CH-O-(alkyle en C1-C4), CH-S-(alkyle en C1-C4), N-O-(alkyle en C1-C4) et
R¹, R², R³ sont
identiques ou différents et représentent un atome d'hydrogène, un groupe cyano, halogéno, NR⁴R⁵, CO₂R⁴, CONR⁴R⁵, COR⁴, S(O)ₙR⁴, avec n = 0, 1 et 2, PO(OR⁴)₂, éventuellement alkyle en C1-C10 ramifié, halogénoalkyle en C1-C4, cycloalkyle en C3-C6, halogénocycloalkyle en C3-C6, cycloalkyle(C3-C6)-alkyle(C1-C4), alcoxy(C1-C4)-alkyle(C1-C4), alkylthio(C1-C4)-alkyle(C1-C4), arylthio-alkyle(C1-C4), alcényle en C2-C6, halogénoalcényle en C2-C5, cycloalcényle en C3-C6, halogénocyclo-alcényle en C3-C6, alcinyle en C2-C6, alcoxy en C1-C6, halogénoalcoxy en C1-C6, alkylthio en C1-C4, benzylthio, alkylcarbonyle en C1-C4, phénylcarbonyle éventuellement substitué, benzylcarbonyle éventuellement substitué, alcoxycarbonyle en C1-C4, phénoxycarbonyle éventuellement substitué, benzyloxycarbonyle éventuellement substitué, aryle éventuellement substitué, aryloxy éventuellement substitué, arylthio éventuellement substitué, arylalkyle en C1-C4 éventuellement substitué, arylalcényle en C2-C4 éventuellement substitué, aryloxy-alkyle en C1-C4 éventuellement substitué, arylthio-alkyle en C1-C4 éventuellement substitué, hétaryle éventuellement substitué, hétaryloxy éventuellement substitué, hétarylthio éventuellement substitué, hétéroaryl-alkyle en C1-C4 éventuellement substitué, hétaryl-alcényle en C2-C4 éventuellement substitué, hétaryloxy-alkyle en C1-C4 éventuellement substitué, hétérocyclyle éventuellement substitué, hétérocyclyloxy éventuellement substitué,
où "éventuellement substitué" signifie, en plus d'un atome d'hydrogène, les restes halogéno, cyano, nitro, alkyle en C1-C4, alcoxy en C1-C4, halogénoalkyle en C1-C4, halogénoalcoxy en C1-C4, alcoximino(C1-C10)-alkyle(C1-C2), aryle, aryloxy, benzyloxy, hétaryle, hétaryloxy, cycloalkyle en C3-C6, hétérocyclyle, hétérocyclyloxy,
et les restes
R⁴, R⁵ sont
identiques ou différents et représentent un atome d'hydrogène ou un groupe alkyle en C1-C4,
R¹ et R³ peuvent former ensemble, ainsi que R² et R³, un composé carbocyclique ou hétérocyclique, qui de son côté peut être condensé à un cycle benzénique, et peut être substitué par les restes mentionnés dans "éventuellement substitué",
et dans le cas où,
b) A représente CH-alkyle en C1-C4,
R¹, R², R³ sont
identiques ou différents et représentent un atome d'hydrogène, un groupe cyano, halogéno, NR⁴R⁵, CO₂R⁴, CONR⁴R⁵, COR⁴, S(O)ₙR⁴, avec n = 0, 1 et 2, PO(OR⁴)₂, éventuellement alkyle en C1-C10 ramifié, halogénoalkyle en C1-C4, cycloalkyle en C3-C6, halogénocycloalkyle en C3-C6, cycloalkyle(C3-C6)-alkyle(C1-C4), alcoxy(C1-C4)-alkyle(C1-C4), alkylthio(C1-C4)-alkyle(C1-C4), arylthio-alkyle(C1-C4), alcényle en C2-C6, halogénoalcényle en C2-C5, cycloalcényle en C3-C6, halogénocyclo-alcényle en C3-C6, alcinyle en C2-C6, alcoxy en C1-C6, halogénoalcoxy en C1-C6, alkylthio en C1-C4, benzylthio, alkylcarbonyle en C1-C4, phénylcarbonyle éventuellement substitué, benzylcarbonyle éventuellement substitué, alcoxycarbonyle en C1-C4, phénoxycarbonyle éventuellement substitué, benzyloxycarbonyle éventuellement substitué, aryle éventuellement substitué, aryloxy éventuellement substitué, arylthio éventuellement substitué, arylalkyle en C1-C4 éventuellement substitué, arylalcényle en C2-C4 éventuellement substitué, aryloxy-alkyle en C1-C4 éventuellement substitué, arylthio-alkyle en C1-C4 éventuellement substitué, hétaryle éventuellement substitué, hétaryloxy éventuellement substitué, hétarylthio éventuellement substitué, hétéroaryl-alkyle en C1-C4 éventuellement substitué, hétaryl-alcényle en C2-C4 éventuellement substitué, hétaryloxy-alkyle en C1-C4 éventuellement substitué, hétérocyclyle éventuellement substitué, hétérocyclyloxy éventuellement substitué,
où "éventuellement substitué" signifie, en plus d'un atome d'hydrogène, les restes halogéno, cyano, nitro, alkyle en C1-C4, alcoxy en C1-C4, halogénoalkyle en C1-C4, halogénoalcoxy en C1-C4, alcoximino(C1-C10)-alkyle(C1-C2), aryle, aryloxy, benzyloxy, hétaryle, hétaryloxy, cycloalkyle en C3-C6, hétérocyclyle, hétérocyclyloxy,
et les restes
R⁴, R⁵ sont
identiques ou différents et représentent un atome d'hydrogène ou un groupe alkyle en C1-C4,
R¹ et R³ peuvent former ensemble, ainsi que R² et R³, un des systèmes cycliques A-F suivants
le groupement 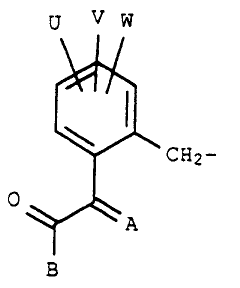 étant représenté dans ce qui suit par le symbole 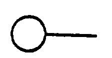 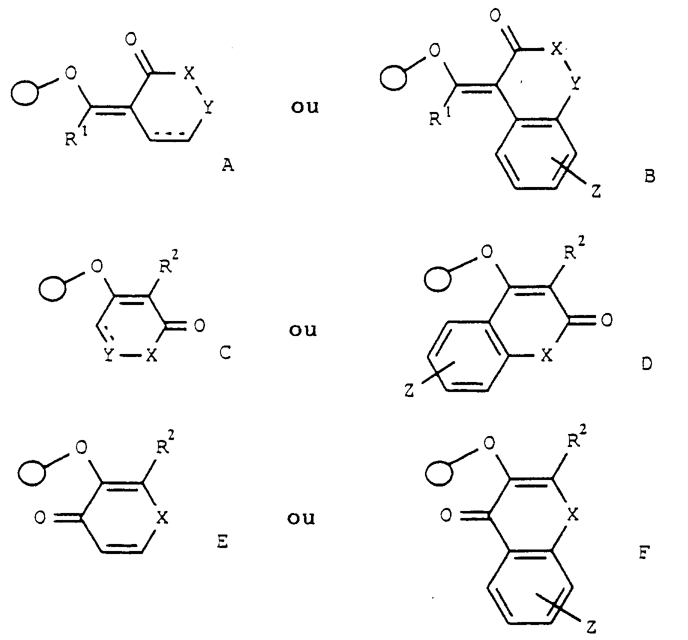 où
X représente
CH₂, O, NR⁴,
Y représente
CH, (CH₂)ₙ, avec n = 0, 1 ou 2,
Z représente
un atome d'hydrogène, un atome d'halogène, un groupe alkyle en C1-C4 et alcoxy en C1-C4, et
R⁴ représente
un atome d'hydrogène et un groupe alkyle en C1-C4 et ···· représente une liaison simple ou une liaison double.

2. Benzylénoléther de formule II 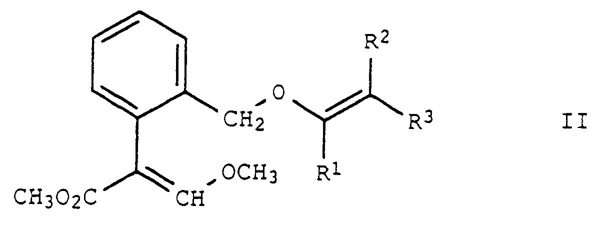 dans laquelle R¹, R² et R³ ont les significations mentionnées dans la revendication 1.

3. Benzylénoléther de formule III 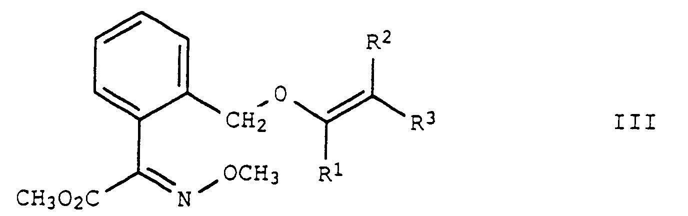 dans laquelle R¹, R² et R³ ont les significations mentionnées dans la revendication 1.

4. Benzylénoléther de formule IV 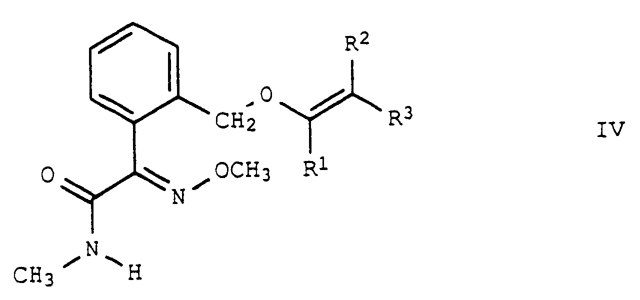 dans laquelle R¹, R² et R³ ont les significations mentionnées dans la revendication 1.

5. Procédé de préparation de benzylénoléthers de formule I selon la revendication 1, caractérisé par le fait que l'on fait réagir un dérivé de benzyle de formule 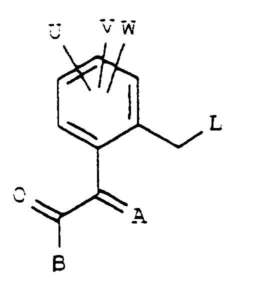 dans laquelle U, V, W, A et B ont les significations mentionnées dans la revendication 1 et L représente un groupe de départ, avec un énol de formule 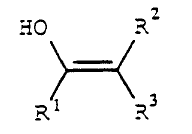 dans la quelle R¹, R² et R³ ont les significations mentionnées dans la revendication 1.

6. Fongicide, contenant un support inerte et une quantité ayant une activité fongicide d'un benzylénoléther de formule I selon la revendication 1.

7. Procédé de lutte contre les champignons, caractérisé par le fait que l'on traite les matières, végétaux, récoltes ou sols, qui sont infestés par les champignons, avec une quantité ayant une activité fongicide d'un composé de formule I selon la revendication 1.

8. Composé de formule I selon la revendication 1, caractérisé par le fait que A représente CHOCH₃, B représente OCH₃, U, V, W et R¹ représentent un atome d'hydrogène, R² représente un groupe cyano et R³ représente un groupe 2-fluorophényle.

9. Composé de formule I selon la revendication 1, caractérisé par le fait que A représente CHOCH₃, B représente OCH₃, U, V, W et R¹ représentent un atome d'hydrogène, R² représente un groupe cyano et R³ représente un groupe 4-méthylphényle.

10. Composé de formule I selon la revendication 1, caractérisé par le fait que A représente NOCH₃, B représente OCH₃, U, V, W et R¹ représentent un atome d'hydrogène, R² représente un groupe cyano et R³ représente un groupe 2-fluorophényle.
